# EUROPEAN PATENT APPLICATION

(11) **EP 4 733 413 A1**
(43) Date of publication of application: **29.04.2026**
(21) Application number: 24306787.3
(22) Date of filing: 23.10.2024
(51) Int. Cl.: C12Q 1/6883

(54) **BIOMARKERS FOR HYPOXIC-ISCHEMIC ENCEPHALOPATHY, AND DIAGNOSTIC METHODS USING THEREOF**

(71) Applicant: Commissariat à l'Energie Atomique et aux Energies Alternatives, 75015 Paris (FR); University of Sharjah, Sharjah, 27272 (AE); Université Paris-Saclay, 91190 Gif-sur-Yvette (FR); Institut National de la Recherche pour l'Agriculture, l'Alimentation et l'Environnement, 75007 Paris (FR)
(72) Inventor: MABONDZO, Aloïse, 91190 GIF-SUR-YVETTE (FR); COSTA, Narciso, 91190 GIF-SUR-YVETTE (FR); DISDIER, Clémence, 91190 GIF-SUR-YVETTE (FR); BOUZID, Amal, 27272 SHARJAH (AE); HAMOUDI, Rifat, 27272 SHARJAH (AE)
(74) Representative: Ipsilon

(57) **Abstract**

The invention relates to the proteins Agrin, Zyxin, Synaptotagmin-5, and combinations thereof, as well as the gene transcripts form the corresponding genes, as biomarkers of a hypoxic-ischemic encephalopathy (HIE).

## Description

### [TECHNICAL FIELD]

The present disclosure relates to biomarkers for hypoxic-ischemic encephalopathy (HIE), and their use for diagnosis methods, for assessing the efficacy of therapeutic agents for the treatment of hypoxic-ischemic encephalopathy, and for screening therapeutic agents for the prevention and/or the treatment of hypoxic-ischemic encephalopathy.

### [TECHNICAL BACKGROUND]

Hypoxic-ischemic encephalopathy (HIE) is the leading cause of neurodevelopmental morbidities in full-term infants. HIE can result from a variety of pregnancy-related disorders before or during birth, including maternal vascular disorders, uterine rupture, umbilical cord entrapment, placental insufficiency, and preeclampsia. HIE occurs in 1 to 8 per 1000 live births in high-income countries and 26 per 1000 live births in low-resource countries. The prevalence of HIE in 2019 was estimated to range from 11,000 to 88,000 cases in high-resource countries and approximately 2,444,000 cases in low-resource countries. Hypoxia-ischemia (HI) induces brain injury via reduced blood flow to the brain combined with lower-than-normal concentrations of oxygen in the arterial blood. These events can result in significant mortality and long-term neurological disabilities, including cerebral palsy, epilepsy, severe learning and mental disabilities, developmental cognitive impairment, motor and behavioral deficits and later psychiatric disorders.

HIE can result from a variety of pregnancy-related disorders before or during birth, including maternal vascular disorders, uterine rupture, umbilical cord entrapment, placental insufficiency, and pre-eclampsia, etc.

Hypothermia is the only therapeutic intervention currently approved to treat HIE in newborn infants. However, this therapeutic modality is partially protective and can be used to treat only full-term infants with HIE. Notably, the death rates after HI remain high, and neurological sequelae remain significant even after treatment with therapeutic hypothermia. Despite treatment with therapeutic hypothermia, sixty percent of infants exposed to moderate or severe HIE expire or have severe residual disabilities, including intellectual disabilities, epilepsy, and cerebral palsy. Furthermore, the long-term beneficial effects of hypothermia in these infants remain controversial. In spite of multiple studies on the pathophysiology of neonatal brain injury, there are currently no pharmacological agents that can protect against brain injury in neonates. A major limitation of unsuccessful approaches is their focus on either the neuronal compartment or the blood-brain barrier (BBB) without accounting for the dynamic interactions between these two biological systems. Therefore, there is an urgent need to develop alternative and/or adjunctive novel therapeutic agents to attenuate the development of HIE.

Pharmacological agents are not yet available to treat or attenuate the development of HIE.

Therefore, there is an urgent need to develop alternative and/or adjunctive novel therapeutic agents to treat HIE.

There is a significant unmet necessity to develop efficacious therapeutics with the potential for neuroprotective capabilities to protect and/or treat neonates, who are exposed to HIE.

For developing new therapeutic tools for preventing and/or treating HIE, there is a need to have biomarkers allowing measuring the efficacy of drug candidates on HIE.

Therefore, there is a need for biomarkers of hypoxic-ischemic encephalopathy (HIE) suitable for systemic or *in vitro* measures.

There is a need for biomarkers which can be easily dosed in biological samples.

There is a need for biomarkers which can allow discriminating a hypoxic-ischemic encephalopathy, from other cerebral disorders.

There is a need for biomarkers which can allow the monitoring of a treatment in an individual suffering from a hypoxic-ischemic encephalopathy.

There is a need for biomarkers which can be used as diagnostic tools as well as tools for monitoring the efficacy of a treatment of a hypoxic-ischemic encephalopathy or monitoring the evolution of a hypoxic-ischemic encephalopathy, or else for screening drug candidates.

The present disclosure has for purpose to satisfy all or part of those needs.

### [SUMMARY]

According to one of its objects, the present disclosure relates to an isolated protein selected from Agrin, Zyxin, Synaptotagmin-5, and a combination thereof, or an isolated gene transcript of a gene or a combination of genes selected from a group of genes comprising or consisting of AGRN (Agrin), ZYX (Zyxin), VWF (Von Willebrand factor), VEGFA (Vascular endothelial growth factor A), ANGT1 (Angiopoietin 1), ANGT2 (Angiopoietin 2), APCDD1 (APC down-regulated 1), ADGRL1 (Adhesion G Protein-Coupled Receptor L1), ATP2A2 (ATPase sarcoplasmic/endoplasmic reticulum Ca²⁺ transporting 2), ERC2 (ELKS/RAB6-Interacting/CAST family member 2), LETM1 (Leucine Zipper and EF-hand containing transmembrane protein 1), NCALD (Neurocalcin delta), NCS1 (Neuronal calcium sensor 1), SESTD1 (SEC14 and Spectrin domain containing 1), SYT2 (Synaptotagmin-2), SEC14 (SEC14-like protein), PACS1 (Phosphofurin acidic cluster sorting protein 1), RAB1A (RAB1A, member RAS oncogene family), MAP2 (Microtubule associated protein 2), NEUN (Neuronal nuclei), SLC17A7 (Solute carrier family 17 member 7), GABRR1 (Gamma-aminobutyric acid type A receptor rho1 subunit), SERPINA3N (Serine (or cysteine) peptidase inhibitor, clade A, member 3N), TXN2 (thioredoxin 2), GFAP (Glial fibrillary acidic protein), IBA-1 (Ionized calcium-binding adapter molecule), SYT5 (Synaptotagmin-5), CDK5 (Cyclin dependent kinase 5), HINT1 (Histidine triad nucleotide binding protein 1), MARCKSL1 (Myristoylated alanine-rich C-kinase substrate Like 1), NIPSNAP2 (Nipsnap Homolog 2), PACS2 (Phosphofurin acidic cluster sorting protein 2), and TRPV2 (Transient receptor potential cation channel subfamily V member 2), for use as a biomarker.

According to one of its objects, the present disclosure relates to an isolated protein selected from Agrin, Zyxin, Synaptotagmin-5, and a combination thereof, or an isolated gene transcript from a gene or a combination of genes selected from a group of genes comprising or consisting of AGRN (Agrin), ZYX (Zyxin), VWF (Von Willebrand factor), VEGFA (Vascular endothelial growth factor A), ANGT1 (Angiopoietin 1), ANGT2 (Angiopoietin 2), APCDD1 (APC down-regulated 1), ADGRL1 (Adhesion G Protein-Coupled Receptor L1), ATP2A2 (ATPase sarcoplasmic/endoplasmic reticulum Ca²⁺ transporting 2), ERC2 (ELKS/RAB6-Interacting/CAST family member 2), LETM1 (Leucine Zipper and EF-hand containing transmembrane protein 1), NCALD (Neurocalcin delta), NCS1 (Neuronal calcium sensor 1), SESTD1 (SEC14 and Spectrin domain containing 1), SYT2 (Synaptotagmin-2), SEC14 (SEC14-like protein), PACS1 (Phosphofurin acidic cluster sorting protein 1), RAB1A (RAB1A, member RAS oncogene family), MAP2 (Microtubule associated protein 2), NEUN (Neuronal nuclei), SLC17A7 (Solute carrier family 17 member 7), GABRR1 (Gamma-aminobutyric acid type A receptor rho1 subunit), SERPINA3N (Serine (or cysteine) peptidase inhibitor, clade A, member 3N), TXN2 (thioredoxin 2), GFAP (Glial fibrillary acidic protein), and IBA-1 (Ionized calcium-binding adapter molecule)AGRIN, ZYXIN, SYNAPTOTAGMIN-5, and a combination thereof, for use as a biomarker in a method for diagnosing a hypoxic-ischemic encephalopathy, or in a method for monitoring a therapeutic efficacy of a therapeutic treatment proposed for preventing and/or treating a hypoxic-ischemic encephalopathy, or in a method for selecting a candidate therapeutic agent for preventing and/or treating a hypoxic-ischemic encephalopathy, or in a method for monitoring an evolution of a hypoxic-ischemic encephalopathy.

According to one of its objects, the present disclosure relates to isolated protein or isolated gene transcript from a gene or a combination of genes selected from a group of genes comprising or consisting of AGRN (Agrin), ZYX (Zyxin), VWF (Von Willebrand factor), VEGFA (Vascular endothelial growth factor A), ANGT1 (Angiopoietin 1), ANGT2 (Angiopoietin 2), APCDD1 (APC down-regulated 1), ADGRL1 (Adhesion G Protein-Coupled Receptor L1), ATP2A2 (ATPase sarcoplasmic/endoplasmic reticulum Ca²⁺ transporting 2), ERC2 (ELKS/RAB6-Interacting/CAST family member 2), LETM1 (Leucine Zipper and EF-hand containing transmembrane protein 1), NCALD (Neurocalcin delta), NCS1 (Neuronal calcium sensor 1), SESTD1 (SEC14 and Spectrin domain containing 1), SYT2 (Synaptotagmin-2), SEC14 (SEC14-like protein), PACS1 (Phosphofurin acidic cluster sorting protein 1), RAB1A (RAB1A, member RAS oncogene family), MAP2 (Microtubule associated protein 2), NEUN (Neuronal nuclei), SLC17A7 (Solute carrier family 17 member 7), GABRR1 (Gamma-aminobutyric acid type A receptor rho1 subunit), SERPINA3N (Serine (or cysteine) peptidase inhibitor, clade A, member 3N), TXN2 (thioredoxin 2), GFAP (Glial fibrillary acidic protein), IBA-1 (Ionized calcium-binding adapter molecule), SYT5 (Synaptotagmin-5), CDK5 (Cyclin dependent kinase 5), HINT1 (Histidine triad nucleotide binding protein 1), MARCKSL1 (Myristoylated alanine-rich C-kinase substrate Like 1), NIPSNAP2 (Nipsnap Homolog 2), PACS2 (Phosphofurin acidic cluster sorting protein 2), and TRPV2 (Transient receptor potential cation channel subfamily V member 2), for use as a biomarker in a method for monitoring a therapeutic efficacy of a therapeutic treatment proposed for preventing and/or treating a hypoxic-ischemic encephalopathy, or in a method for selecting a candidate therapeutic agent for preventing and/or treating a hypoxic-ischemic encephalopathy.

According to one of its objects, the present disclosure relates to a method for diagnosing a hypoxic-ischemic encephalopathy in an individual in need thereof, said method comprising at least the steps of:
a) measuring an amount of protein or of gene transcript from a gene or a combination of genes, the gene or combination of genes being selected from a group of genes comprising or consisting of AGRN (Agrin), ZYX (Zyxin), VWF (Von Willebrand factor), VEGFA (Vascular endothelial growth factor A), ANGT1 (Angiopoietin 1), ANGT2 (Angiopoietin 2), APCDD1 (APC down-regulated 1), ADGRL1 (Adhesion G Protein-Coupled Receptor L1), ATP2A2 (ATPase sarcoplasmic/endoplasmic reticulum Ca²⁺ transporting 2), ERC2 (ELKS/RAB6-lnteracting/CAST family member 2), LETM1 (Leucine Zipper and EF-hand containing transmembrane protein 1), NCALD (Neurocalcin delta), NCS1 (Neuronal calcium sensor 1), SESTD1 (SEC14 and Spectrin domain containing 1), SYT2 (Synaptotagmin-2), SEC14 (SEC14-like protein), PACS1 (Phosphofurin acidic cluster sorting protein 1), RAB1A (RAB1A, member RAS oncogene family), MAP2 (Microtubule associated protein 2), NEUN (Neuronal nuclei), SLC17A7 (Solute carrier family 17 member 7), GABRR1 (Gamma-aminobutyric acid type A receptor rho1 subunit), SERPINA3N (Serine (or cysteine) peptidase inhibitor, clade A, member 3N), TXN2 (thioredoxin 2), GFAP (Glial fibrillary acidic protein), and IBA-1 (Ionized calcium-binding adapter molecule), in an isolated biological sample obtained from said individual, and
b) comparing the amount measured at step a) with a reference value,
wherein a deviation observed between the measured amount and the reference value is indicative of a hypoxic-ischemic encephalopathy in said individual.

According to one of its objects, the present disclosure relates to a method for monitoring an evolution of a hypoxic-ischemic encephalopathy in an individual in need thereof, said method comprising the steps of:
a) measuring an amount of protein or of gene transcript from a gene or a combination of genes, the gene or the combination of genes being selected from the group of genes as defined above, in an isolated biological sample obtained from said individual at a first point of time,
b) measuring an amount of protein or of gene transcript from a gene or a combination of genes, the gene or the combination of genes being selected from the group of genes as defined in step a), in an isolated biological sample obtained from said individual at a second point of time, the gene or combination of genes of step a) and b) being the same, and
c) comparing the amount measured at step a) with the amount measured at step b),
wherein a deviation observed between the amounts measured at step a) and at step b) is indicative of an improvement or an aggravation of the hypoxic-ischemic encephalopathy in said individual.

In some embodiments, the combination of genes comprising at least two genes.

In some embodiments, the combination of genes comprising at least AGRN and ZYX.
the gene or combination of genes may consist of:
- AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N, or
- TXN2, GFAP and IBA-1, or
- AGRN, ZYX, VWF, VEGFA, ANGT1, ANGT2, APCDD1, LETM1, PACS1, TXN2, GFAP, and IBA-1, or
- AGRN, ZYX, VWF, VEGFA, ANGT1, ANGT2, and APCDD1, or
- AGRN and ZYX.

According to another of its objects, the present disclosure relates to a method for monitoring a therapeutic efficacy of a therapeutic treatment (therapeutic agent) proposed for preventing and/or treating a hypoxic-ischemic encephalopathy in an individual in need thereof, said method comprising the steps of:
a) measuring an amount of protein or of gene transcript from a gene or a combination of genes, the gene or the combination of genes being selected from the group of genes comprising or consisting of AGRN (Agrin), ZYX (Zyxin), VWF (Von Willebrand factor), VEGFA (Vascular endothelial growth factor A), ANGT1 (Angiopoietin 1), ANGT2 (Angiopoietin 2), APCDD1 (APC down-regulated 1), ADGRL1 (Adhesion G Protein-Coupled Receptor L1), ATP2A2 (ATPase sarcoplasmic/endoplasmic reticulum Ca²⁺ transporting 2), ERC2 (ELKS/RAB6-Interacting/CAST family member 2), LETM1 (Leucine Zipper and EF-hand containing transmembrane protein 1), NCALD (Neurocalcin delta), NCS1 (Neuronal calcium sensor 1), SESTD1 (SEC14 and Spectrin domain containing 1), SYT2 (Synaptotagmin-2), SEC14 (SEC14-like protein), PACS1 (Phosphofurin acidic cluster sorting protein 1), RAB1A (RAB1A, member RAS oncogene family), MAP2 (Microtubule associated protein 2), NEUN (Neuronal nuclei), SLC17A7 (Solute carrier family 17 member 7), GABRR1 (Gamma-aminobutyric acid type A receptor rho1 subunit), SERPINA3N (Serine (or cysteine) peptidase inhibitor, clade A, member 3N), TXN2 (thioredoxin 2), GFAP (Glial fibrillary acidic protein), IBA-1 (Ionized calcium-binding adapter molecule), SYT5 (Synaptotagmin-5), CDK5 (Cyclin dependent kinase 5), HINT1 (Histidine triad nucleotide binding protein 1), MARCKSL1 (Myristoylated alanine-rich C-kinase substrate Like 1), NIPSNAP2 (Nipsnap Homolog 2), PACS2 (Phosphofurin acidic cluster sorting protein 2), and TRPV2 (Transient receptor potential cation channel subfamily V member 2), in an isolated biological sample obtained from said individual before administration of said therapeutic treatment,
b) measuring an amount protein or of gene transcript from a gene or a combination of genes, the gene or the combination of genes being selected from the group of genes as defined in step a), in an isolated biological sample obtained from said individual after administration of said therapeutic treatment, the gene or combination of genes of step a) and b) being the same, and
c) comparing the amount measured at step a) with the amount measured at step b),
wherein a deviation observed between the amounts measured at step a) and at step b) is indicative of a therapeutic efficacy of said therapeutic treatment on said hypoxic-ischemic encephalopathy.

According to one of its objects, the disclosure relates to a method for selecting a candidate therapeutic agent for preventing and/or treating a hypoxic-ischemic encephalopathy in an individual in need thereof, said method comprising the steps of:
a) measuring an amount of protein or of gene transcript from a gene or a combination of genes, the gene or the combinations of genes being selected from the group of genes as defined above, in an isolated biological sample obtained from a biological model of a hypoxic-ischemic encephalopathy before contacting said biological model with said candidate therapeutic agent,
b) measuring an amount of protein or of gene transcript from a gene or a combination of genes, the gene or the combination of genes being selected from the group of genes as defined in step a), in an isolated biological sample obtained from said biological model of step a) after contacting said biological model with said candidate therapeutic agent, the gene or combination of genes of step a) and b) being the same,
c) comparing the amount measured at step a) with the amount measured at step b), and
d) selecting a candidate therapeutic agent for which a deviation observed between the amounts measured at step a) and at step b) is indicative of a therapeutic efficacy of said candidate therapeutic agent on said hypoxic-ischemic encephalopathy.

In some embodiments, the combination of genes comprising at least two genes.

In some embodiments, the combination of genes comprising at least AGRN and ZYX, and optionally SYT5.

In some embodiments, the gene or combination of genes may consist of:
- AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, SERPINA3N, SYT5, CDK5, HINT1, MARCKSL1, NIPSNAP2, PACS2, and TRPV2,
   or
- AGRN, ZYX, VWF, VEGFA, ANGT1, ANGT2, APCDD1, SEC14, PACS1, RAB1A, MAP2, NEUN, SLC17A7, GABRR1, SERPINA3N, SYT5, CDK5, HINT1, MARCKSL1, NIPSNAP2, PACS2, and TRPV2,
   or
- AGRN, ZYX, VWF, VEGFA, ANGT1, ANGT2, APCDD1, SEC14, PACS1, RAB1A, MAP2, NEUN, SLC17A7, GABRR1, SERPINA3N, and SYT5,
   or
- AGRN, ZYX, VWF, VEGFA, ANGT1, ANGT2, APCDD1, SEC14, PACS1, RAB1A, and SYT5,
   or
- AGRN, ZYX, VWF, VEGFA, ANGT1, ANGT2, APCDD1, and SYT5,
   or
- AGRN, ZYX and SYT5
   or
- AGRN and ZYX.

In some embodiments, step a) may comprise measuring an amount of the proteins from the genes AGRN, ZYX and SYT5

In some embodiments, step a) may comprise measuring an amount of the gene transcripts from the genes AGRN, ZYX and SYT5.

In some embodiments, steps a) and b) may comprise measuring an amount of the gene transcripts from the genes AGRN, ZYX and SYT5.

As shown in the Example section, the protein(s) and/or the gene transcript(s) from a gene or a combination of genes selected in the group of genes defined herein were differentially expressed in rats of a Rice-Vannuci model of neonatal hypoxia-ischemia.

The proteins or gene transcripts from the genes AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N were decreased following induction of hypoxia-ischemia, compared to sham-treated rats, and increased (or normalized compared to sham-treated rats) in hypoxic-ischemic rats treated with L-valyl ester of (2R)-2-[[6-[benzyl(ethyl)amino]-9-isopropyl-purin-2-yl] amino] butan-1-ol hydrochloride (BRT_002), a novel compound shown to improve neuronal plasticity and restore brain injuries following an hypoxia-ischemia.

The amounts of proteins or gene transcripts from the genes SYT5, CDK5, HINT1, MARCKSL1, NIPSNAP2, PACS2, and TRPV2 were non-affected following induction of hypoxia-ischemia, compared to sham-treated rats, but increased in hypoxic-ischemic rats treated with BRT_002.

The amounts of proteins or gene transcripts from the genes TXN2, GFAP and IBA-1 were increased following induction of hypoxia-ischemia, compared to sham-treated rats, and decreased (or normalized compared to sham-treated rats) in hypoxic-ischemic rats treated with BRT_002.

In particular, as shown in the Example section, the protein amounts of Agrin and Zyxin, as well as the gene transcript amounts of AGRIN and ZYXIN were downregulated in rats of a Rice-Vannuci model of neonatal hypoxia-ischemia, and were upregulated upon treatment with BRT_002.

Further, the Examples show that Synaptotagmin-5 is significantly overexpressed in brains of rats subjected to hypoxia-ischemia and treated with BRT-002 by comparison with the placebo-treated group.

Therefore, the Examples show that proteins or gene transcripts from the genes AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, SERPINA3N, TXN2, GFAP and IBA-1 can be used as biomarker for diagnosing a hypoxic-ischemic encephalopathy or monitoring the evolution of a hypoxic-ischemic encephalopathy.

Further, the Examples shows that proteins or gene transcripts from the genes AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, SERPINA3N, TXN2, GFAP, IBA-1, SYT5, CDK5, HINT1, MARCKSL1, NIPSNAP2, PACS2, and TRPV2 can be used as biomarker for monitoring a therapeutic efficacy of a therapeutic treatment proposed for preventing and/or treating a hypoxic-ischemic encephalopathy or for selecting a candidate therapeutic agent for preventing and/or treating a hypoxic-ischemic encephalopathy.

In some embodiments, the biological sample may be selected from the group consisting of blood sample, plasma sample, serum sample, brain tissue sample, and cerebrospinal fluid sample.

According to another of its objects, the disclosure relates to an antibody for use in a method as disclosed herein. The method may comprise at least a step of measuring an amount of protein from a gene or a combination of genes selected in a group of genes as above defined.

According to another of its objects, the disclosure relates to a set of primers for use in a method as disclosed herein. The method may comprise at least a step of measuring an amount of a gene transcript from a gene or a combination of genes selected in a group of genes as above defined.

According to another of its objects, the disclosure relates to a kit-of-parts comprising:
a) ) means for measuring an amount of at least one protein or of at least one gene transcript from a gene or a combination of genes selected from the group of genes as defined, and
b) a set of instructions for carrying out said measure.

In some embodiments, the means for determining the amount of protein may be configured for performing an immunoassay and/or a mass-spectrometric-based assay.

In some embodiments, the means for determining the amount of gene transcript may be configured for performing a quantitative polymerase chain reaction (qPCR) or a high-throughput sequencing process.

In some embodiments, the kit may comprise a set of instructions to compare the measured amount with a reference value.

According to another of its objects, the disclosure relates to a kit as disclosed herein for use in a method as disclosed herein.

### [DESCRIPTION OF THE FIGURES]

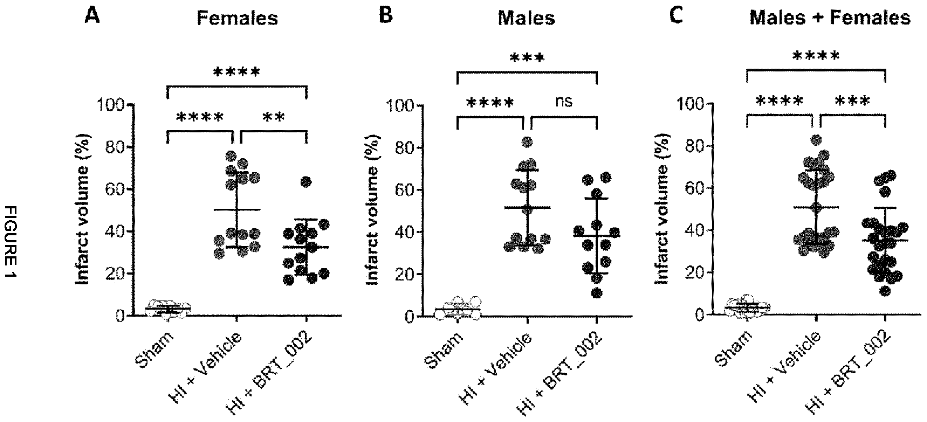

**FIGURE** 1: BRT_002 alleviates brain injury. Pups were randomly distributed into 3 groups: 1) sham control, 2) moderate HI + placebo (HI+Veh), and 3) moderate HI+BRT_002 (30 mg/kg). We administered BRT_002 immediately after carotid ligation and 90 min of hypoxia (HI) and administered two additional doses at 24 h and 48 h after HI. The animals were sacrificed 72 h after HI at P10, and the brains were harvested for analysis. Percent infarct volume plotted on the y-axis for the sham, HI+ Vehicle and HI-BRT_002 treated groups: females (A), males (B) and males + females (C). Sham designated by open circles, HI Vehicle designated by closed red circles, and HI+BRT_002 (30 mg/kg) designated by closed blue circles. Sham: females, n=10; males, n=7; HI + Vehicle: females, n=13; males, n=13; HI + BRT_002: females, n= 13, males, n=12. Statistical analysis by one-way ANOVA and Bonferroni post hoc correction; *P < 0.05; **P < 0.01; ***P < 0.001; **** P < 0.0001.

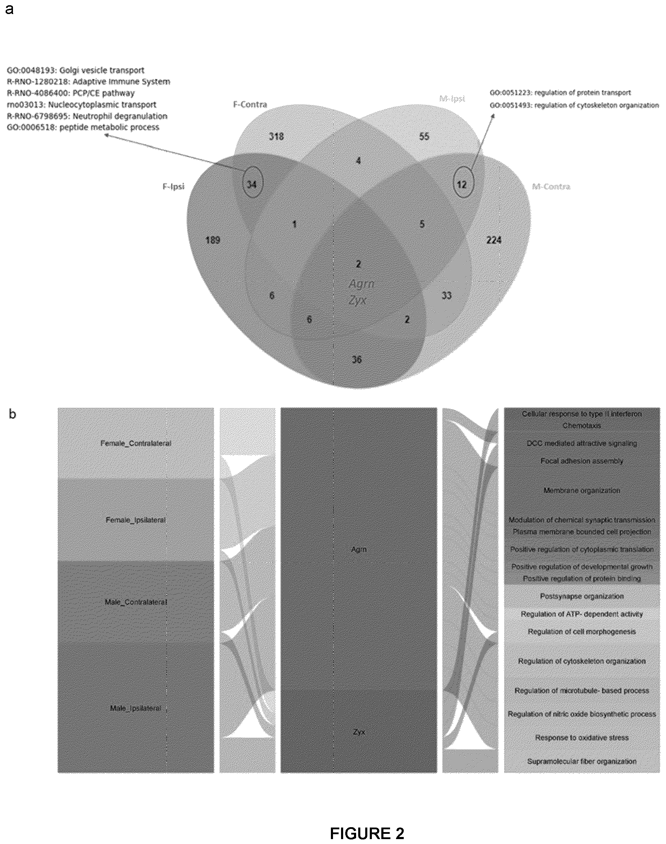

**FIGURE 2****:** Proteomic analysis revealed that treatment with BRT_002 results in unique protein expressions in the ipsilateral and contralateral sides of the brain in the male and female neonatal rats after exposure to HI. a) Comparisons of the differentially expressed proteins (DEPs) in the HI+BRT_002 vs. HI+Veh groups of males and females and separately for the ipsilateral and contralateral brain hemispheres. F: female; M: male; Ipsi: ipsilateral; Contra: contralateral. b) Alluvial plot of agrin- and zyxin-related pathways among the different comparisons between the HI+BRT_002 and HI+Veh groups of neonatal rats. The left column shows the different comparisons between the HI+BRT_002 and HI+Veh groups of neonatal rats, the right column shows the related pathways, and the edges represent their relationship with agrin and zyxin. A larger edge width represents pathway-related systems.

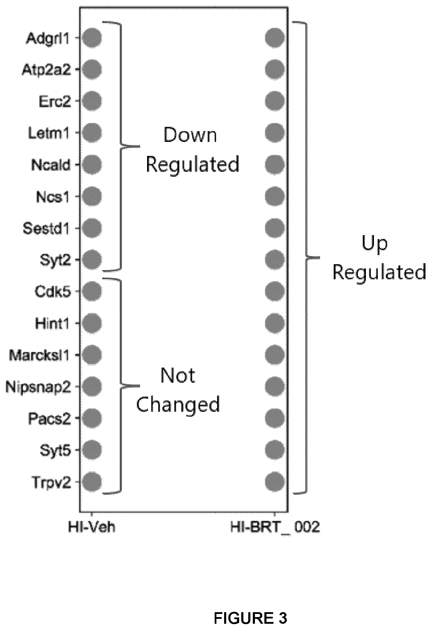

**FIGURE 3****:** Neurotransmitters and calcium sensors, which were selected from the comparison of the HI+BRT_ 002 and HI+Veh groups of neonatal rats after exposure to moderate HI independent of sex. a) Dots plot showing the protein signatures of corrected protein expression that were downregulated (green dots) or not changed (blue dots) compared to the sham group. Red dots indicate upregulated proteinsafter BRT_002 treatment compared with the HI+Veh group. b) Pathways related to BRT_002-induced neurotransmitters and calcium sensors after exposure to HI. Functional enrichment analysis of BRT_002-induced neurotransmitters and calcium sensors resulted from the comparison of the HI+BRT_ 002 and HI+Veh groups of neonatal rats after exposure to HI.

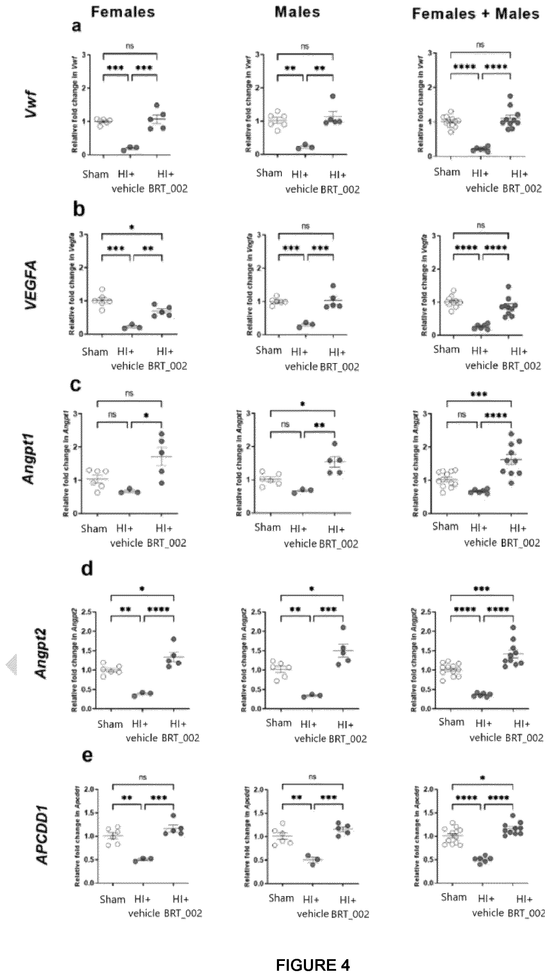

**FIGURE 4****:** BRT_002 facilitates transcriptional upregulation expression of key markers of brain capillaries related to Agrin and Zyxin in the ipsilateral hemisphere of female and male neonatal rats after exposure to moderate HI. a) mRNA expression of *Von Willebrand Factor (Vwf);* **b**) *VEGFA,* **c**) Angiopoietin 1 (*Angpt1)*, **d**) Angiopoietin 2 *(Angpt2),* and e) *APCDD1.* Sham designated by open circles, HI Vehicle designated by closed red circles, and HI+BRT_002 (30 mg/kg) designated by closed blue circles. Data for the ipsilateral hemisphere of females (sham, n=6; HI + Vehicle, n= 3; and HI + BRT_002, n=5), males (sham, n=6; HI + Vehicle, n= 3; and HI + BRT_002, n=5) and males + females (sham, n=12; HI + Vehicle, n= 6; and HI + BRT_002, n=10) were compared to those of the control and vehicle groups. *P < 0.05; **P < 0.01; ***P < 0.001; **** P < 0.0001 one-way ANOVA, Tukey's multiple comparison test.

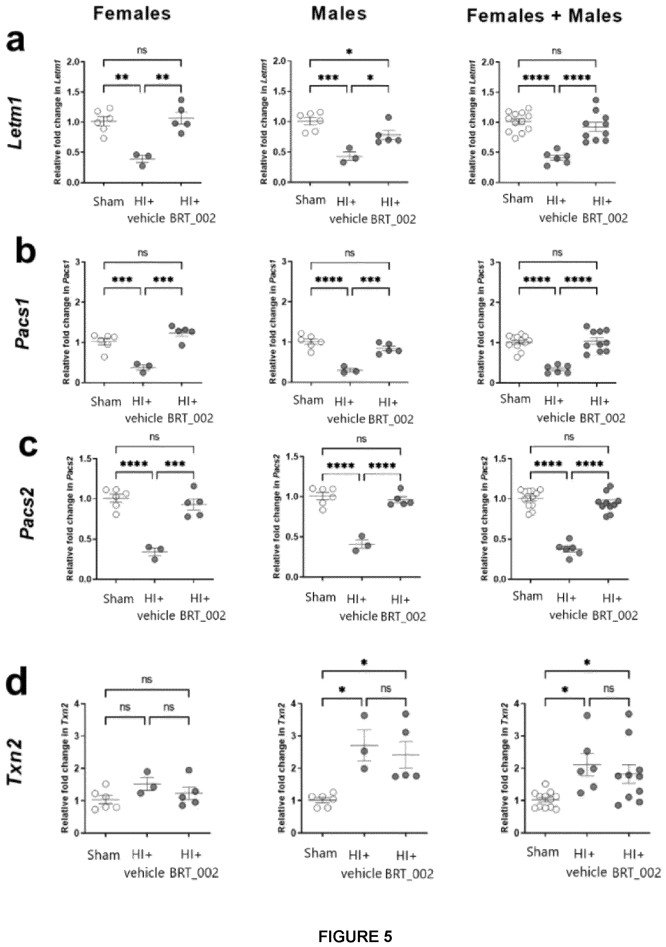

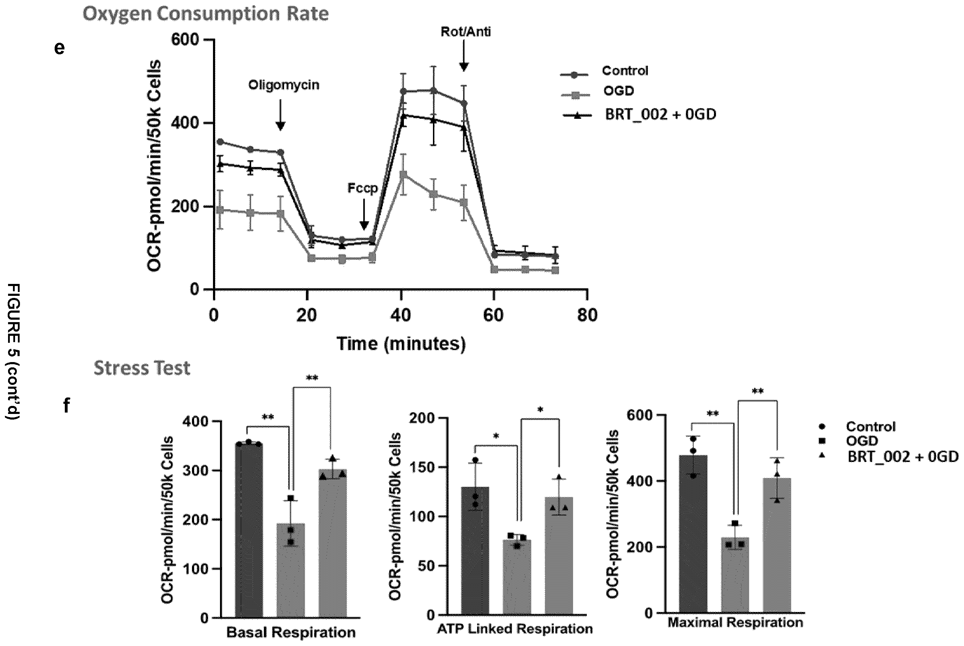

**FIGURE 5****:** BRT_002 modulates the transcriptional expression of key mitochondrial components in the ipsilateral brain hemisphere of female and male neonatal rats exposed to moderate HI and improves mitochondria function in, HI+Veh group 3 days after exposure to HI related brain injury. (**a**), *Letm1,* **(b)** *Pacs1,* **(c)** *Pacs2,* and **(d)** *Txn2.* Data for the ipsilateral hemisphere in females (sham, n=6; HI + Vehicle, n= 3; and HI + BRT_002, n=5), males (sham, n=6; HI + Vehicle, n = 3; and HI + BRT_002, n = 5) and males + females (sham, n = 12; HI + Vehicle, n = 6; and HI + BRT_002, n=10) compared to those in the Sham and HI + Vehicle groups. Sham designated by open circles, HI Vehicle designated by closed red circles, and HI+BRT_002 (30 mg/kg) designated by closed blue circles. *P < 0.05; **P < 0.01; ***P < 0.001; **** P < 0.0001, one-way ANOVA, Tukey's multiple comparison test. **(e** and **f)** *Regulation of mitochondrial functions.* Primary neuronal cells were cultured in seahorse plates (1 plate with control) and (1 plate with OGD exposure/and BRT_002+OGD exposure). In the plate with OGD exposure (for 60 mins), another set of the cells were pretreated with 10µM of BRT_002 for 24 h. Furthermore, after OGD exposure, plates were incubated for 24 hrs at 37°C/CO2 to facilitate reoxygenation followed by the seahorse measurements. Data represent three independent experiments expressed as mean ± SD (n=3). Statistical analysis by one-way ANOVA with *p<0.05, **p<0.01. Bar graphs represent as an individual data points.

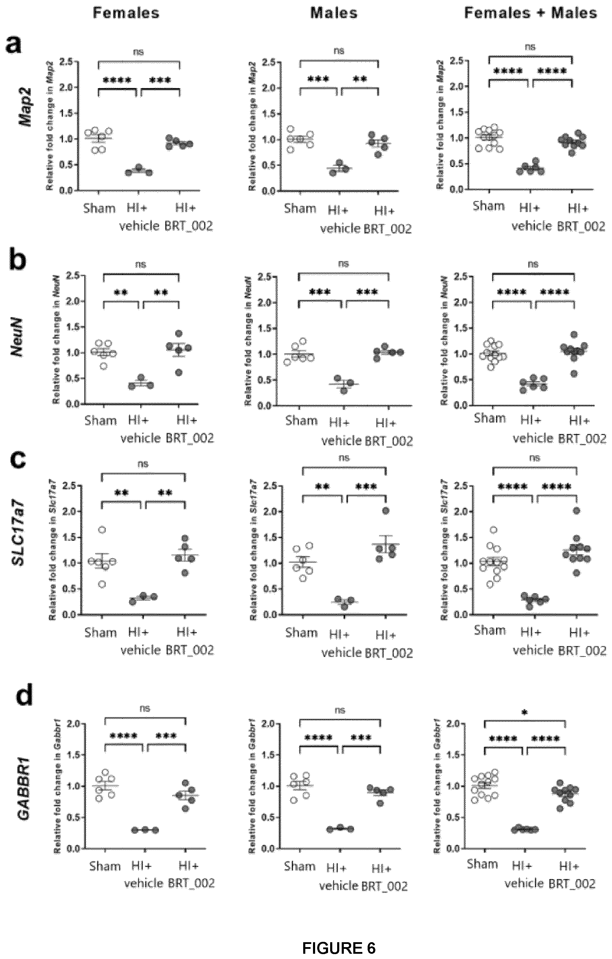

**FIGURE 6****:** Transcriptional expression of key synaptic markers in the ipsilateral brain hemisphere of female and male neonatal rats in the sham, HI+Veh and HI+ BRT_002 groups 3 days after exposure to HI related brain injury. **(a)** *MAP2,* **(b)** *NeuN,* **(c)** *SLC17a7,* **(d)** and *GABBR1* in the ipsilateral hemisphere. Data for females (sham, n=6; HI + Vehicle, n= 3; and HI + BRT_002, n=5), males (sham, n=6; HI + Vehicle, n= 3; and HI + BRT_002, n=5) and males + females (sham, n=12; HI + Vehicle, n= 6; and HI + BRT_002, n=10) were compared to those for the control and vehicle groups. *P < 0.05; **P < 0.01; ***P < 0.001; **** P < 0.0001, ordinary one-way ANOVA, Tukey's multiple comparison test.

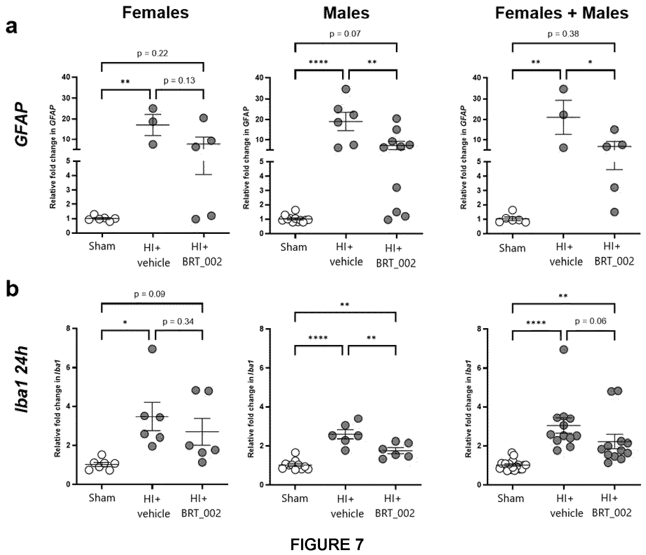

**FIGURE 7****:** BRT_002 attenuates neuroinflammation in neonatal rats after exposure to moderate HI in the ipsilateral brain hemisphere of female and male neonatal rats in the sham, HI+Veh and HI+ BRT_002 groups. **a)** mRNA expression of *GFAP* 3 days after HI related brain injury. **b)** mRNA expression of *Iba1* 24h after HI injury. Sham designated by open circles, HI Vehicle designated by closed red circles, and HI+BRT_002 (30 mg/kg) designated by closed blue circles. Data for females (sham, n=6; HI + Vehicle, n= 3 and HI + BRT_002, n=5), males (sham, n=6; HI + Vehicle, n= 3 and HI + BRT_002, n=5) and males + females (sham, n=12; HI + Vehicle, n= 6 and HI + BRT_002, n=10) were compared to those in the control and vehicle groups. *P < 0.05; **P < 0.01; ***P < 0.001; **** P < 0.0001, one-way ANOVA, Tukey's multiple comparison test.

### [DETAILED DESCRIPTION]

### Definitions

Unless otherwise defined herein, scientific and technical terms used in connection with the present disclosure shall have the meanings that are commonly understood by those of ordinary skill in the art. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, may provide one of skill in the art with a general dictionary of many of the terms used in this disclosure. Exemplary methods and materials are described below, although methods and materials similar or equivalent to those described herein can also be used in the practice or testing of the present disclosure. In case of conflict, the present specification, including definitions, will control. Generally, nomenclature used in connection with, and techniques of, cell and tissue culture, molecular biology, virology, immunology, microbiology, genetics, analytical chemistry, synthetic organic chemistry, medicinal and pharmaceutical chemistry, and protein and nucleic acid chemistry and hybridization described herein are those well-known and commonly used in the art. Enzymatic reactions and purification techniques are performed according to manufacturer's specifications, as commonly accomplished in the art or as described herein. Further, unless otherwise required by context, singular terms shall include pluralities and plural terms shall include the singular.

Units, prefixes, and symbols are denoted in their International System Units (Système International des Unités (SI)) accepted form. Numeric ranges are inclusive of the numbers defining the range. Unless otherwise indicated, amino acid sequences are written left to right in amino to carboxy orientation. The headings provided herein are not limitations of the various aspects of the disclosure. Accordingly, the terms defined immediately below are more fully defined by reference to the specification in its entirety.

All publications and other references mentioned herein are incorporated by reference in their entirety. Although a number of documents are cited herein, this citation does not constitute an admission that any of these documents forms part of the common general knowledge in the art.

Throughout this specification and embodiments, the words **"have"** and **"comprise,"** or **variations** such as **"has," "having," "comprises,"** or **"comprising,"** will be understood to imply the inclusion of a stated integer or group of integers but not the exclusion of any other integer or group of integers. It is understood that wherever aspects are described herein with the language "comprising," otherwise analogous aspects described in terms of "consisting of" and/or "consisting essentially of" are also provided.

It is to be noted that the term **"a"** or **"an"** entity refers to one or more of that entity; for example, "a nucleotide sequence," is understood to represent one or more nucleotide sequences. As such, the terms "a" (or "an"), "one or more," and "at least one" can be used interchangeably herein.

**"And/or"** where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

The term **"approximately"** or **"about"** is used herein to mean approximately, roughly, around, or in the regions of. When the term "about" is used in conjunction with a numerical range, it modifies that range by extending the boundaries above and below the numerical values set forth. In general, the term "about" can modify a numerical value above and below the stated value by a variance of, *e.g.,* 10 percent, up or down (higher or lower). In some embodiments, the term indicates deviation from the indicated numerical value by ±10%, ±5%, ±4%, ±3%, ±2%, ±1%, ±0.9%, ±0.8%, ±0.7%, ±0.6%, ±0.5%, ±0.4%, ±0.3%, ±0.2%, ±0.1%, ±0.05%, or ±0.01%. In some embodiments, "about" indicates deviation from the indicated numerical value by ±10%. In some embodiments, "about" indicates deviation from the indicated numerical value by ±5%. In some embodiments, "about" indicates deviation from the indicated numerical value by ±4%. In some embodiments, "about" indicates deviation from the indicated numerical value by ±3%. In some embodiments, "about" indicates deviation from the indicated numerical value by ±2%. In some embodiments, "about" indicates deviation from the indicated numerical value by ±1%. In some embodiments, "about" indicates deviation from the indicated numerical value by ±0.9%. In some embodiments, "about" indicates deviation from the indicated numerical value by ±0.8%. In some embodiments, "about" indicates deviation from the indicated numerical value by ±0.7%. In some embodiments, "about" indicates deviation from the indicated numerical value by ±0.6%. In some embodiments, "about" indicates deviation from the indicated numerical value by ±0.5%. In some embodiments, "about" indicates deviation from the indicated numerical value by ±0.4%. In some embodiments, "about" indicates deviation from the indicated numerical value by ±0.3%. In some embodiments, "about" indicates deviation from the indicated numerical value by ±0.1%. In some embodiments, "about" indicates deviation from the indicated numerical value by ±0.05%. In some embodiments, "about" indicates deviation from the indicated numerical value by ±0.01%.

Within the disclosure, the terms "amount" or "measured amount" intends to refer to an absolute or relative amount or concentration of a compound, e.g. a protein or a gene transcript, the presence or absence of a compound, a range of amounts or concentrations of a compound, a minimum and/or maximum amount or concentration of a compound, a mean amount or concentration of a compound, and/or a median amount or concentration of a compound; and, in addition, when analyzing a combination of compounds also the ratios of absolute or relative amounts or concentrations of two or more compounds with respect to each other may be measured. In some embodiments, measured amounts may be transformed by variance-stabilizing transformation, according to known statistical methods, before being compared to predetermined reference values.

**Gene transcript.** As used herein, this expression or equivalents thereof intend to refer to a molecule of RNA that is synthesized from the DNA sequence of a gene through the process of transcription. This RNA molecule carries the genetic information encoded in the DNA.

**Protein.** As used herein, this expression or equivalents thereof intend to refer to a chain of amino acids joined by peptide bonds.

**Deviation.** As used herein, this term or equivalent thereof intends to refer to a difference or departure from an expected, standard, or normal value or refer to a result or a measurement that falls outside of normal or expected ranges. A deviation between a measured results and a reference value may be used to detect a disease.

By **"purified"** and **"isolated"** it is meant, when referring to a biological sample, a gene transcript, or a protein, that the indicated element has been separated from other substances or components that were originally present in a mixture or from its natural environment. The term "purified" as used herein in particular means at least 75%, 85%, 95%, or 98% by weight, of elements of the same type are present.

**"Sample"** or **"biological sample"** intends to refer to a biological material obtained (or isolated) from a subject. The biological sample may contain any biological material suitable for detecting and measuring a biomarker, i.e., a protein or a gene transcript, and may comprise cellular and/or non-cellular material from the subject. Examples for biological samples are any suitable biological tissue or fluid such as of blood, plasma, serum, or cerebrospinal fluid. Also, a biological sample may be a brain organoid prepared by dedifferentiation and reprogramming of fibroblast cells obtained from said individual.

Within the disclosure, the term **"biomarker"** intends to mean a compound, such as a protein or a gene transcript, or a set of compounds, taking part in a particular biological process in an individual and which can be measured in the body, its products, or isolated biological sample, and influence or predict the incidence of outcome or disease. The biomarker might be an intermediate or a product of a biological process. A biomarker may be a compound, a measured amount of the compound or a result of comparison between a measured amount of the compound and a predetermined value of reference.

As used herein, the terms **"subject", "individual"** or **"patient"** are used interchangeably and denote a mammal, such as a rodent, a feline, a canine, and a primate. In some embodiments, a patient according to the disclosure is a human being. In particular, a patient according to the disclosure is an infant. In some embodiments, a patient according to the disclosure is a full term infant. In some embodiments, a patient according to the disclosure is a preterm infant. A patient in need thereof may be a subject known or presumed to have a hypoxic-ischemic encephalopathy.

"Healthy reference individual" means a subject which is on a normal, healthy state and from which a value of reference for a given biomarker may be obtained and use as "reference value".

Within the disclosure, the expression **"reference value"** or **"predetermined reference value"** are used interchangeably and intend to refer to a threshold value or amount, a reference range or a cut-off value or amount of a biomarker or an index defined by set of biomarkers that can be determined experimentally, empirically, or theoretically and by comparing with which, a diagnosis of a hypoxic-ischemic encephalopathy can be made. A threshold value or amount can also be arbitrarily selected based upon the existing experimental and/or clinical conditions, as would be recognized by a person of ordinary skilled in the art. A predetermined reference value or amount may be selected to maximize sensitivity while keeping the specificity above a user-defined threshold. A predetermined reference value or amount can also be selected to maximize specificity while keeping the sensitivity above a user-defined threshold, for example, 80% sensitivity. A predetermined reference value or amount can be a threshold amount or value of a protein or a biomarker or a threshold of an index defined by a set of biomarkers obtained from a population of healthy individuals.

A reference value can be an average value obtained from a set of healthy individuals and can be used to establish a benchmark, against which a deviation may be indicative of disease.

Alternatively, a reference value can be an average value obtained from a set of individuals suffering from a given disease and can be used to establish a benchmark, against which a deviation may be indicative of a therapeutic efficiency of a therapeutic treatment.

As used herein, the expression **"statistically different"** or **"significantly different"** refers to that an observed alteration is greater than what would be expected to occur by chance alone (e.g., a "false positive"). Statistical significance can be determined by any of various methods well-known in the art. An example of a commonly used measure of statistical significance is the p-value. The p-value represents the probability of obtaining a given result equivalent to a particular datapoint, where the datapoint is the result of random chance alone. A result is often considered significant (not random chance) at a p-value less than or equal to 0.05.

Within the disclosure, the term **"substantially"** used in conjunction with a feature, e.g., "an amount equal to or substantially equal to", of the disclosure intends to define a set of embodiments related to this feature which are largely but not wholly similar to this feature.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable sub-combination.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure.

All publications mentioned herein are incorporated herein by reference to disclose and describe the methods and/or materials in connection with which the publications are cited.

The list of sources, ingredients, and components as described hereinafter are listed such that combinations and mixtures thereof are also contemplated and within the scope herein.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

All lists of items, such as, for example, lists of ingredients, are intended to and should be interpreted as Markush groups. Thus, all lists can be read and interpreted as items "selected from the group consisting of' the list of items "and combinations and mixtures thereof."

Referenced herein may be trade names for components including various ingredients utilized in the present disclosure. The inventors herein do not intend to be limited by materials under any particular trade name. Equivalent materials (e.g., those obtained from a different source under a different name or reference number) to those referenced by trade name may be substituted and utilized in the descriptions herein.

### Detailed description

### Biomarkers

In some embodiments, the disclosure relates to protein from a gene or from a combination of genes, the gene or the combination of genes being selected from a group of genes comprising or consisting of AGRN (Agrin), ZYX (Zyxin), VWF (Von Willebrand factor), VEGFA (Vascular endothelial growth factor A), ANGT1 (Angiopoietin 1), ANGT2 (Angiopoietin 2), APCDD1 (APC down-regulated 1), ADGRL1 (Adhesion G Protein-Coupled Receptor L1), ATP2A2 (ATPase sarcoplasmic/endoplasmic reticulum Ca²⁺ transporting 2), ERC2 (ELKS/RAB6-Interacting/CAST family member 2), LETM1 (Leucine Zipper and EF-hand containing transmembrane protein 1), NCALD (Neurocalcin delta), NCS1 (Neuronal calcium sensor 1), SESTD1 (SEC14 and Spectrin domain containing 1), SYT2 (Synaptotagmin-2), SEC14 (SEC14-like protein), PACS1 (Phosphofurin acidic cluster sorting protein 1), RAB1A (RAB1A, member RAS oncogene family), MAP2 (Microtubule associated protein 2), NEUN (Neuronal nuclei), SLC17A7 (Solute carrier family 17 member 7), GABRR1 (Gamma-aminobutyric acid type A receptor rho1 subunit), SERPINA3N (Serine (or cysteine) peptidase inhibitor, clade A, member 3N), TXN2 (thioredoxin 2), GFAP (Glial fibrillary acidic protein), and IBA-1 (Ionized calcium-binding adapter molecule), as a biomarker of a hypoxic-ischemic encephalopathy.

In some embodiments, the biomarker may be used for diagnosing a hypoxic-ischemic encephalopathy in an individual in need thereof.

In some embodiments, the biomarker may be used for monitoring an evolution of a hypoxic-ischemic encephalopathy in an individual in need thereof.

In some embodiments, the biomarker may be used for monitoring a therapeutic efficacy of a therapeutic treatment proposed for preventing and/or treating a hypoxic-ischemic encephalopathy in an individual in need thereof.

In some embodiments, the biomarker may be used for selecting a candidate therapeutic agent for preventing and/or treating a hypoxic-ischemic encephalopathy in an individual in need thereof.

In some embodiments, the combination comprises at least or consists of two genes.

In some embodiments, the combination comprises at least or consists of the genes GRN and ZYX, and optionally SYT5.

In some embodiments, a protein or a gene transcript is a human protein or gene transcript.

The proteins are isolated proteins.

The gene transcripts are isolated gene transcripts.

A biomarker may be an amount of protein or of gene transcript. In some embodiments, a biomarker may be an observed deviation between an amount of protein or gene transcript measured in an individual suspected to suffer from a hypoxic-ischemic encephalopathy and a reference value of amount of said protein or of said gene transcript.

AGRN/Agrin, also known as Cms8 or Cmsppd, is an heparan sulfate proteoglycan widely expressed at the interneuron synapses and involved in the establishment of the tight blood-brain barrier, suggesting its role in the brain repair lesion in moderate HI. The protein contains several laminin G, Kazal type serine protease inhibitor, and epidermal growth factor domains. Additional post-translational modifications occur to add glycosaminoglycans and disulfide bonds. Further to the gene transcription, alternative splicing results in multiple transcript variants encoding different isoforms.

ZXN/Zyxin, also known as Esp-2; Hed-2, is a zinc-binding phosphoprotein that concentrates at focal adhesions and along the actin cytoskeleton. Zyxin has an N-terminal proline-rich domain and three LIM domains in its C-terminal half. The proline-rich domain may interact with SH3 domains of proteins involved in signal transduction pathways while the LIM domains are likely involved in protein-protein binding. Zyxin may function as a messenger in the signal transduction pathway that mediates adhesion-stimulated changes in gene expression and may modulate the cytoskeletal organization of actin bundles. Further to the gene transcription, alternative splicing results in multiple transcript variants that encode the same isoform. Zyxin is involved in the regulation regulates endothelial von Willebrand factor secretion by reorganizing actin filaments around exocytic granules which participates in the brain lesion repair after HI insult.

SYT5/Synaptotagmin-5 (Syt5) is part of the synaptotagmins family, a multifunctional integral membrane protein found predominantly on vesicles in neural and endocrine tissues. 17 isoforms of the synaptotagmin family in mammals have been identified, out of them 7 isoforms are known to be able to bind Ca²⁺ via their C2 domains. Recent work has highlighted how brain function relies on additional isoforms, with roles in postsynaptic receptor endocytosis, vesicle trafficking, membrane repair, synaptic plasticity, and protection against neurodegeneration.

VWF/Von Willebrand factor is a large multimeric glycoprotein that plays a role in hemostasis. It mediates platelet adhesion and aggregation at sites of vascular injury and It serves as a carrier protein for coagulation factor VIII in plasma.

VEGFA/Vascular endothelial growth factor A is a dimeric glycoprotein that plays a role in angiogenesis.

ANGT1/Angiopoietin 1 is a secreted glycoprotein that plays a role in vascular development and angiogenesis. It functions as a ligand for the TEK/TIE2 receptor tyrosine kinase, which is primarily expressed on endothelial cells.

ANGT2/Angiopoietin 2 is a secreted glycoprotein that plays a role in angiogenesis and vascular remodeling. It is an antagonist of Angiopoietin 1 and acts as a ligand for the TEK/TIE2 receptor tyrosine kinase.

APCDD1/APC down-regulated 1 is a membrane-bound glycoprotein that functions as a negative regulator of the Wnt signaling pathway

ADGRL1/Adhesion G Protein-Coupled Receptor L1 is a member of the latrophilin subfamily of G-protein coupled receptors (GPCRs).

ATP2A2/ATPase sarcoplasmic/endoplasmic reticulum Ca²⁺ transporting 2

ERC2/ELKS/RAB6-Interacting/CAST family member 2 is an intracellular calcium pump located in the sarcoplasmic or endoplasmic reticula of muscle cells.

LETM1/Leucine Zipper and EF-hand containing transmembrane protein 1 s an inner mitochondrial membrane protein that plays a crucial role in mitochondrial calcium (Ca2+) and proton (H+) homeostasis. The protein contains a leucine zipper domain, an EF-hand motif (EF1) in its C-terminal domain, and multiple transmembrane domains. The EF1 motif is highly conserved and required for LETM1's Ca²⁺ transport function.

NCALD/Neurocalcin delta is a protein that belongs to the neuronal calcium sensor (NCS) family. The protein contains an N-terminal myristoylation signal and four EF-hand calcium-binding loops. It is cytosolic at resting calcium levels but associates with membranes and partially co-localizes with the perinuclear trans-Golgi network when intracellular calcium levels are elevated.

NCS1/Neuronal calcium sensor 1 is a protein that belongs to the neuronal calcium sensor (NCS) family. NCS1 is a globular protein consisting of ten alpha-helices. It contains four EF-hand calcium-binding motifs, a myristoylation motif at the N-terminus, and multiple protein binding domains.

SESTD1/SEC14 and Spectrin domain containing 1 is a protein that contains both SEC14 and spectrin domains. SESTD1 contains a SEC14 domain, which is a lipid-binding domain found in proteins involved in lipid metabolism and transport. It also contains a spectrin domain, which is a structural motif found in proteins involved in cytoskeletal organization and membrane interactions.

SYT2/Synaptotagmin-2 is a calcium-binding protein that plays a crucial role in synaptic vesicle exocytosis and neurotransmitter release at the presynaptic terminal. SYT2 contains two C2 domains (C2A and C2B) that bind calcium ions and phospholipids. These domains are responsible for the calcium-dependent interactions of SYT2 with the SNARE complex and the presynaptic membrane.

SEC14/SEC14-like protein is essential for Golgi secretory function and cell viability. It catalyzes the transport of phosphatidylinositol (Ptdlns) and phosphatidylcholine (PtdCho) between lipid membranes.

PACS1/Phosphofurin acidic cluster sorting protein 1 is a coat protein that is involved in the localization of trans-Golgi network (TGN) membrane proteins containing acidic cluster sorting motifs.

RAB1A/RAB1A member RAS oncogene family play roles in regulating intracellular membrane trafficking and vesicle transport.

MAP2/Microtubule associated protein 2 is a protein that binds to and stabilizes microtubules in neurons. AP2 contains multiple microtubule-binding domains that allow it to bind along the length of microtubules. It also has projections that extend from the microtubule surface, potentially interacting with other cellular components.

### Neuronal Nuclear Antigen and Neuron Differentiation Marker

The Solute carrier family 17 member 7 (SLC17A7) gene encodes a vesicle-bound, sodium-dependent phosphate transporter specifically expressed in neuron-rich regions of the brain. This transporter is preferentially associated with the membranes of synaptic vesicles and plays a critical role in glutamate transport.

GABRR1/Gamma-aminobutyric acid type A receptor rho1 subunit encodes the rho1 subunit of the GABAA receptor, which is a ligand-gated ion channel that mediates inhibitory neurotransmission in the central nervous system.

SERPINA3N/Serine (or cysteine) peptidase inhibitor, clade A, member 3N encodes a serine protease inhibitor that belongs to the serpin superfamily.

TXN2/thioredoxin 2 is a small redox-active protein that plays a role in maintaining the reducing environment within the mitochondrial matrix.

GFAP/Glial fibrillary acidic protein is an intermediate filament protein that is primarily expressed in astrocytes in the central nervous system.

IBA-1/lonized calcium-binding adapter molecule is a calcium-binding protein that is primarily expressed in activated macrophages and microglia in the central nervous system.

SYT5/Synaptotagmin-5 is a member of the synaptotagmin family of proteins, which are involved in the regulation of synaptic vesicle exocytosis and neurotransmitter release.

CDK5/Cyclin dependent kinase 5 is involved in various neuronal processes, such as neuronal migration, synaptic function, and neurodegeneration.

HINT1/Histidine triad nucleotide binding protein 1 is a small, highly conserved protein belonging to the histidine triad (HIT) superfamily that plays important roles in cellular signaling, transcriptional regulation, and nucleotide metabolism.

MARCKSL1/Myristoylated alanine-rich C-kinase substrate Like 1 is a myristoylated protein involved in various cellular processes, including signal transduction, cytoskeleton organization, and protein-protein interactions.

NIPSNAP2/Nipsnap Homolog 2 is a protein that belongs to the NIPSNAP family of proteins, which are involved in various cellular processes, including protein-protein interactions and membrane trafficking.

PACS2/Phosphofurin acidic cluster sorting protein 2 is involved in the sorting of cargo proteins containing acidic cluster sorting motifs.

TRPV2/Transient receptor potential cation channel subfamily V member 2) is a calcium-permeable cation channel that belongs to the TRP superfamily.

For diagnosing a hypoxic-ischemic encephalopathy or monitoring an evolution of a hypoxic-ischemic encephalopathy in an individual in need thereof, a biomarker or a combination of biomarkers may be a protein or a gene transcript from a gene or from a combination of genes selected from a group of genes comprising or consisting of AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, SERPINA3N, TXN2, GFAP, and IBA-1.

In some embodiments, the combination of genes may comprise at least or consist of two genes.

In some embodiments, the combination of genes may comprise at least or consist of AGRN and ZYX.

In some embodiments, the gene or combination of genes may comprise at least or consist of:
- AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N, or
- TXN2, GFAP and IBA-1, or
- AGRN, ZYX, VWF, VEGFA, ANGT1, ANGT2, APCDD1, LETM1, PACS1, TXN2, GFAP, and IBA-1, or
- AGRN, ZYX, VWF, VEGFA, ANGT1, ANGT2, and APCDD1, or
- AGRN and ZYX.

For monitoring a therapeutic efficacy of a therapeutic treatment proposed for preventing and/or treating a hypoxic-ischemic encephalopathy in an individual in need thereof or for selecting a candidate therapeutic agent for preventing and/or treating a hypoxic-ischemic encephalopathy in an individual in need thereof, a biomarker or a combination of biomarkers may be a protein or a gene transcript from a gene or from a combination of genes selected from a group of genes comprising or consisting of AGRN, ZYX, VWF, VEGFA, ANGT1, ANGT2, APCDD1, ADGRL1, ATP2A2, ERC2, LETM1, NCALD, NCS1 , SESTD1, SYT2, SEC14, PACS1, RAB1A, MAP2, NEUN, SLC17A7, GABRR1, SERPINA3N, TXN2, GFAP, IBA-1, SYT5, CDK5, HINT1, MARCKSL1, NIPSNAP2, PACS2, and TRPV2.

In some embodiments, the combination of genes may comprise at least or consist of two genes.

In some embodiments, the combination of genes may comprise at least or consist of AGRN and ZYX, and optionally SYT5.

In some embodiments, the gene or combination of genes may comprise at least or consist of:
- AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, SERPINA3N, SYT5, CDK5, HINT1, MARCKSL1, NIPSNAP2, PACS2, and TRPV2,
   or
- AGRN, ZYX, VWF, VEGFA, ANGT1, ANGT2, APCDD1, SEC14, PACS1, RAB1A, MAP2, NEUN, SLC17A7, GABRR1, SERPINA3N, SYT5, CDK5, HINT1, MARCKSL1, NIPSNAP2, PACS2, and TRPV2,
   or
- AGRN, ZYX, VWF, VEGFA, ANGT1, ANGT2, APCDD1, SEC14, PACS1, RAB1A, MAP2, NEUN, SLC17A7, GABRR1, SERPINA3N, and SYT5,
   or
- AGRN, ZYX, VWF, VEGFA, ANGT1, ANGT2, APCDD1, SEC14, PACS1, RAB1A, and SYT5,
   or
- AGRN, ZYX, VWF, VEGFA, ANGT1, ANGT2, APCDD1, and SYT5,
   or
- AGRN, ZYX and SYT5
   or
- AGRN and ZYX.

The amounts of protein or gene transcript disclosed herein were observed as being significantly increased or decreased, depending on the protein or gene transcript, in cerebral tissue from individuals suffering from a hypoxic-ischemic encephalopathy, compared with healthy individuals.

The amounts of protein or gene transcript disclosed herein were observed as being significantly increased or decreased, depending on the protein or gene transcript, in cerebral tissue from individuals suffering from a hypoxic-ischemic encephalopathy and treated with a tri-substituted purine derivative, such as BRT_001 or BRT_002, compared with undertreated individuals suffering from a hypoxic-ischemic encephalopathy.

An **increased** amount of a protein or of a gene transcript compared with a reference value may be an increase from about 1.1 to about 10.0 fold, or from about 1.2 to about 8.0 fold, or from about 1.3 to about 6.0 fold or from about 1.4 to about 5.0 fold, or from about 1.5 to about 2.5 fold.

An increase of x fold compared with a reference value (or an increase of x-fold the reference value) means that the reference value is multiplied by x.

An **increased** amount of a protein or of a gene transcript compared with a reference value may be an increase of about 1.3, or of about 1.4, or of about 1.5, or of about 1.6, or of about 1.7, or of about 1.8, or of about 1.9, or of about 2.0, or of about 2.5, or of about 3.0, or of about 4.0, or of about 5.0, or of about 6.0, or of about 7.0, or of about 8.0, or of about 9.0, or of about 10.0-fold.

In some embodiments, an increased amount of a protein or of a gene transcript compared with a reference value may be an increase of at least about 1.5-fold, or of about 1.5 to about 5.0 fold.

A **decreased** amount of a protein or of a gene transcript compared with a reference value may be an increase from about 1.1 to about 10.0 fold, or from about 1.2 to about 8.0 fold, or from about 1.3 to about 6.0 fold or from about 1.4 to about 5.0 fold, or from about 1.5 to about 2.5 fold, or about 2.0 fold.

A decrease of x fold compared with a reference value (or a decrease of x-fold the reference value) means that the reference value is divided by x.

A **decreased** amount of a protein or of a gene transcript compared with a reference value may be a decrease of about 1.1-fold, or of about 1.2, or of about 1.3, or of about 1.4, or of about 1.5, or of about 1.6, or of about 1.7, or of about 1.8, or of about 1.9, or of about 2.0, or of about 2.5, or of about 3.0, or of about 4.0, or of about 5.0, or of about 6.0, or of about 7.0, or of about 8.0, or of about 9.0, or of about 10.0-fold.

In some embodiments, a decrease amount of a protein or of a gene transcript compared with a reference value may be a decrease of at least about 1.5-fold, or of about 1.5 to about 2.5 fold.

A decrease of x fold compared with a reference value (or a decrease of x-fold the reference value) means that the reference value is divided by x.

A reference value of a protein or of a gene transcript measured herein may be obtained from a healthy individual or a group of healthy individuals. A reference value may represent an average of measures obtained from a group of healthy individuals. To be comparable, the amounts of proteins or gene transcripts measured from a biological sample and the reference values are obtained with same measurement methods. The methods are the same for the same protein or gene transcript but may differ for different proteins.

Alternatively, a reference value of a protein or of a gene transcript measured herein may be obtained from an individual suffering from a hypoxic-ischemic encephalopathy or a group of individuals suffering from a hypoxic-ischemic encephalopathy. A reference value may represent an average of measures obtained from a group of individuals suffering from a hypoxic-ischemic encephalopathy. To be comparable, the amounts of proteins or gene transcripts measured from a biological sample and the reference values are obtained with same measurement methods. The methods are the same for the same protein or gene transcript but may differ for different proteins.

In some approaches, amounts of biomarkers, e.g., proteins or gene transcripts, may be processed into more valuable forms of information, e.g., by using either common mathematical transformations such as logarithmic or logistic functions. Other data processing approaches, such as normalization of biomarker results in reference to a population's mean values, etc. are also well known to those skilled in the art and can be used.

The amount of protein or gene transcript of the gene AGRN in a biological sample obtained from an individual suffering from a hypoxic-ischemic encephalopathy is significantly decreased compared with the amount of protein or gene transcript of the gene AGRN in a biological sample, e.g., blood, plasma or serum sample, obtained from a healthy individual or a reference value. For example, the decrease of the measured amount of protein or gene transcript of the gene AGRN in a biological sample obtained from an individual suffering from a hypoxic-ischemic encephalopathy may be from about 1.8 to about 2.5 fold, or about 2.0 fold the reference value, the reference value being from healthy individuals. Administration of therapeutic agent may increase or restore an amount of protein or gene transcript of the gene AGRN within the reference value or range for the protein or gene transcript of the gene AGRN.

The amount of protein or gene transcript of the gene ZXN in a biological sample obtained from an individual suffering from a hypoxic-ischemic encephalopathy is significantly decreased compared with the amount of protein or gene transcript of the gene ZXN in a biological sample obtained from a healthy individual or a reference value. For example, the decrease of the measured amount of protein or gene transcript of the gene ZXN in a biological sample obtained from an individual suffering from a hypoxic-ischemic encephalopathy may be from about 1.8 to about 2.5 fold, or about 2.0 fold the reference value, the reference value being from healthy individuals. Administration of therapeutic agent may increase or restore an amount of protein or gene transcript of the gene ZXN within the reference value or range for the protein or gene transcript of the gene ZXN.

The amount of protein or gene transcript of the gene SYT5 in a biological sample obtained from an individual suffering from a hypoxic-ischemic encephalopathy is significantly increased compared with the amount of protein or gene transcript of the gene SYT5 in a biological sample obtained from a healthy individual or a reference value. For example, the increase of the measured amount of protein or gene transcript of the gene SYT5 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase an amount of protein or gene transcript of the gene SYT5 above the reference value or range for the protein or gene transcript of the gene SYT5.

The amount of protein or gene transcript of the gene VWF in a biological sample obtained from an individual suffering from a hypoxic-ischemic encephalopathy is significantly decreased compared with the amount of protein or gene transcript of the gene VWF in a biological sample obtained from a healthy individual or a reference value. For example, the decrease of the measured amount of protein or gene transcript of the gene VWF in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene VWF within the reference value or range for the protein or gene transcript of the gene VWF.

The amount of protein or gene transcript of the gene VEGFA in a biological sample obtained from an individual suffering from a hypoxic-ischemic encephalopathy is significantly decreased compared with the amount of protein or gene transcript of the gene VEGFA in a biological sample obtained from a healthy individual or a reference value. For example, the decrease of the measured amount of protein or gene transcript of the gene VEGFA in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene VEGFA within the reference value or range for the protein or gene transcript of the gene VEGFA.

The amount of protein or gene transcript of the gene ANGT1 in a biological sample obtained from an individual suffering from a hypoxic-ischemic encephalopathy is significantly decreased compared with the amount of protein or gene transcript of the gene ANGT1 in a biological sample obtained from a healthy individual or a reference value. For example, the decrease of the measured amount of protein or gene transcript of the gene ANGT1 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene ANGT1 within the reference value or range for the protein or gene transcript of the gene ANGT1.

The amount of protein or gene transcript of the gene ANGT2 in a biological sample obtained from an individual suffering from a hypoxic-ischemic encephalopathy is significantly decreased compared with the amount of protein or gene transcript of the gene ANGT2 in a biological sample obtained from a healthy individual or a reference value. For example, the decrease of the measured amount of protein or gene transcript of the gene ANGT2 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene ANGT2 within the reference value or range for the protein or gene transcript of the gene ANGT2.

The amount of protein or gene transcript of the gene APCDD1 in a biological sample obtained from an individual suffering from a hypoxic-ischemic encephalopathy is significantly decreased compared with the amount of protein or gene transcript of the gene APCDD1 in a biological sample obtained from a healthy individual or a reference value. For example, the decrease of the measured amount of protein or gene transcript of the gene APCDD1 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene APCDD1 within the reference value or range for the protein or gene transcript of the gene APCDD1.

The amount of protein or gene transcript of the gene ADGRL1 in a biological sample obtained from an individual suffering from a hypoxic-ischemic encephalopathy is significantly decreased compared with the amount of protein or gene transcript of the gene ADGRL1 in a biological sample obtained from a healthy individual or a reference value. For example, the decrease of the measured amount of protein or gene transcript of the gene ADGRL1 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene ADGRL1 within the reference value or range for the protein or gene transcript of the gene ADGRL1.

The amount of protein or gene transcript of the gene ATP2A2 in a biological sample obtained from an individual suffering from a hypoxic-ischemic encephalopathy is significantly decreased compared with the amount of protein or gene transcript of the gene ATP2A2 in a biological sample obtained from a healthy individual or a reference value. For example, the decrease of the measured amount of protein or gene transcript of the gene ATP2A2 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene ATP2A2 within the reference value or range for the protein or gene transcript of the gene ATP2A2.

The amount of protein or gene transcript of the gene ERC2 in a biological sample obtained from an individual suffering from a hypoxic-ischemic encephalopathy is significantly decreased compared with the amount of protein or gene transcript of the gene ERC2 in a biological sample obtained from a healthy individual or a reference value. For example, the decrease of the measured amount of protein or gene transcript of the gene ERC2 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene ERC2 within the reference value or range for the protein or gene transcript of the gene ERC2.

The amount of protein or gene transcript of the gene LETM1 in a biological sample obtained from an individual suffering from a hypoxic-ischemic encephalopathy is significantly decreased compared with the amount of protein or gene transcript of the gene LETM1 in a biological sample obtained from a healthy individual or a reference value. For example, the decrease of the measured amount of protein or gene transcript of the gene LETM1 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene LETM1 within the reference value or range for the protein or gene transcript of the gene LETM1.

The amount of protein or gene transcript of the gene NCALD in a biological sample obtained from an individual suffering from a hypoxic-ischemic encephalopathy is significantly decreased compared with the amount of protein or gene transcript of the gene NCALD in a biological sample obtained from a healthy individual or a reference value. For example, the decrease of the measured amount of protein or gene transcript of the gene NCALD in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene NCALD within the reference value or range for the protein or gene transcript of the gene NCALD.

The amount of protein or gene transcript of the gene NCS1 in a biological sample obtained from an individual suffering from a hypoxic-ischemic encephalopathy is significantly decreased compared with the amount of protein or gene transcript of the gene NCS1 in a biological sample obtained from a healthy individual or a reference value. For example, the decrease of the measured amount of protein or gene transcript of the gene NCS1 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene NCS1 within the reference value or range for the protein or gene transcript of the gene NCS1.

The amount of protein or gene transcript of the gene SESTD1 in a biological sample obtained from an individual suffering from a hypoxic-ischemic encephalopathy is significantly decreased compared with the amount of protein or gene transcript of the gene SESTD1 in a biological sample obtained from a healthy individual or a reference value. For example, the decrease of the measured amount of protein or gene transcript of the gene SESTD1 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene SESTD1 within the reference value or range for the protein or gene transcript of the gene SESTD1.

The amount of protein or gene transcript of the gene SYT2 in a biological sample obtained from an individual suffering from a hypoxic-ischemic encephalopathy is significantly decreased compared with the amount of protein or gene transcript of the gene SYT2 in a biological sample obtained from a healthy individual or a reference value. For example, the decrease of the measured amount of protein or gene transcript of the gene SYT2 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene SYT2 within the reference value or range for the protein or gene transcript of the gene SYT2.

The amount of protein or gene transcript of the gene SEC14 in a biological sample obtained from an individual suffering from a hypoxic-ischemic encephalopathy is significantly decreased compared with the amount of protein or gene transcript of the gene SEC14 in a biological sample obtained from a healthy individual or a reference value. For example, the decrease of the measured amount of protein or gene transcript of the gene SEC14 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene SEC14 within the reference value or range for the protein or gene transcript of the gene SEC14.

The amount of protein or gene transcript of the gene PACS1 in a biological sample obtained from an individual suffering from a hypoxic-ischemic encephalopathy is significantly decreased compared with the amount of protein or gene transcript of the gene PACS1 in a biological sample obtained from a healthy individual or a reference value. For example, the decrease of the measured amount of protein or gene transcript of the gene PACS1 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene PACS1 within the reference value or range for the protein or gene transcript of the gene PACS1.

The amount of protein or gene transcript of the gene RAB1A in a biological sample obtained from an individual suffering from a hypoxic-ischemic encephalopathy is significantly decreased compared with the amount of protein or gene transcript of the gene RAB1A in a biological sample obtained from a healthy individual or a reference value. For example, the decrease of the measured amount of protein or gene transcript of the gene RAB1A in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene RAB1A within the reference value or range for the protein or gene transcript of the gene RAB1A.

The amount of protein or gene transcript of the gene MAP2 in a biological sample obtained from an individual suffering from a hypoxic-ischemic encephalopathy is significantly decreased compared with the amount of protein or gene transcript of the gene MAP2 in a biological sample obtained from a healthy individual or a reference value. For example, the decrease of the measured amount of protein or gene transcript of the gene MAP2 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene MAP2 within the reference value or range for the protein or gene transcript of the gene MAP2.

The amount of protein or gene transcript of the gene NEUN in a biological sample obtained from an individual suffering from a hypoxic-ischemic encephalopathy is significantly decreased compared with the amount of protein or gene transcript of the gene NEUN in a biological sample obtained from a healthy individual or a reference value. For example, the decrease of the measured amount of protein or gene transcript of the gene NEUN in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene NEUN within the reference value or range for the protein or gene transcript of the gene NEUN.

The amount of protein or gene transcript of the gene SLC17A7 in a biological sample obtained from an individual suffering from a hypoxic-ischemic encephalopathy is significantly decreased compared with the amount of protein or gene transcript of the gene SLC17A7 in a biological sample obtained from a healthy individual or a reference value. For example, the decrease of the measured amount of protein or gene transcript of the gene SLC17A7 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene SLC17A7 within the reference value or range for the protein or gene transcript of the gene SLC17A7.

The amount of protein or gene transcript of the gene GABRR1 in a biological sample obtained from an individual suffering from a hypoxic-ischemic encephalopathy is significantly decreased compared with the amount of protein or gene transcript of the gene GABRR1 in a biological sample obtained from a healthy individual or a reference value. For example, the decrease of the measured amount of protein or gene transcript of the gene GABRR1 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene GABRR1 within the reference value or range for the protein or gene transcript of the gene GABRR1.

The amount of protein or gene transcript of the gene SERPINA3N in a biological sample obtained from an individual suffering from a hypoxic-ischemic encephalopathy is significantly decreased compared with the amount of protein or gene transcript of the gene SERPINA3N in a biological sample obtained from a healthy individual or a reference value. For example, the increase of the measured amount of protein or gene transcript of the gene SERPINA3N in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene SERPINA3N within the reference value or range for the protein or gene transcript of the gene SERPINA3N.

The amount of protein or gene transcript of the gene CDK5 in a biological sample obtained from an individual suffering from a hypoxic-ischemic encephalopathy is significantly decreased compared with the amount of protein or gene transcript of the gene CDK5 in a biological sample obtained from a healthy individual or a reference value. For example, the increase of the measured amount of protein or gene transcript of the gene CDK5 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may increase or restore an amount of protein or gene transcript of the gene CDK5 within the reference value or range for the protein or gene transcript of the gene CDK5.

The amount of protein or gene transcript of the gene HINT1 in a biological sample obtained from an individual suffering from a hypoxic-ischemic encephalopathy is substantially non-affected compared with the amount of protein or gene transcript of the gene HINT1 in a biological sample obtained from a healthy individual or a reference value. Administration of therapeutic agent may increase the measured amount of protein or gene transcript of the gene HINT1 in a biological sample obtained from an individual suffering from a hypoxic-ischemic encephalopathy from about 1.1 to about 10.0 fold, or from about 1.2 to about 8.0 fold, or from about 1.3 to about 6.0 fold or from about 1.4 to about 5.0 fold the reference value or value of untreated individuals suffering from a hypoxic-ischemic encephalopathy. Administration of therapeutic agent may increase an amount of protein or gene transcript of the gene HINT1 above the reference value or range for the protein or gene transcript of the gene HINT1.

The amount of protein or gene transcript of the gene MARCKSL1 in a biological sample obtained from an individual suffering from a hypoxic-ischemic encephalopathy is substantially non-affected compared with the amount of protein or gene transcript of the gene MARCKSL1 in a biological sample obtained from a healthy individual or a reference value. Administration of therapeutic agent may increase the measured amount of protein or gene transcript of the gene MARCKSL1 in a biological sample obtained from an individual suffering from a hypoxic-ischemic encephalopathy from about 1.1 to about 10.0 fold, or from about 1.2 to about 8.0 fold, or from about 1.3 to about 6.0 fold or from about 1.4 to about 5.0 fold a reference value or value of untreated individuals suffering from a hypoxic-ischemic encephalopathy. Administration of therapeutic agent may increase an amount of protein or gene transcript of the gene MARCKSL1 above the reference value or range for the protein or gene transcript of the gene MARCKSL1.

The amount of protein or gene transcript of the gene NIPSNAP2 in a biological sample obtained from an individual suffering from a hypoxic-ischemic encephalopathy is substantially non-affected compared with the amount of protein or gene transcript of the gene NIPSNAP2 in a biological sample obtained from a healthy individual or a reference value. Administration of therapeutic agent may increase the measured amount of protein or gene transcript of the gene NIPSNAP2 in a biological sample obtained from an individual suffering from a hypoxic-ischemic encephalopathy from about 1.1 to about 10.0 fold, or from about 1.2 to about 8.0 fold, or from about 1.3 to about 6.0 fold or from about 1.4 to about 5.0 fold a reference value or value of untreated individuals suffering from a hypoxic-ischemic encephalopathy. Administration of therapeutic agent may increase an amount of protein or gene transcript of the gene NIPSNAP2 above the reference value or range for the protein or gene transcript of the gene NIPSNAP2.

The amount of protein or gene transcript of the gene PACS2 in a biological sample obtained from an individual suffering from a hypoxic-ischemic encephalopathy is substantially non-affected compared with the amount of protein or gene transcript of the gene PACS2 in a biological sample obtained from a healthy individual or a reference value. Administration of therapeutic agent may increase the measured amount of protein or gene transcript of the gene PACS2 in a biological sample obtained from an individual suffering from a hypoxic-ischemic encephalopathy from about 1.1 to about 10.0 fold, or from about 1.2 to about 8.0 fold, or from about 1.3 to about 6.0 fold or from about 1.4 to about 5.0 fold a reference value or value of untreated individuals suffering from a hypoxic-ischemic encephalopathy. Administration of therapeutic agent may increase an amount of protein or gene transcript of the gene PACS2 above the reference value or range for the protein or gene transcript of the gene PACS2.

The amount of protein or gene transcript of the gene TRPV2 in a biological sample obtained from an individual suffering from a hypoxic-ischemic encephalopathy is substantially non-affected compared with the amount of protein or gene transcript of the gene TRPV2 in a biological sample obtained from a healthy individual or a reference value. Administration of therapeutic agent may increase the measured amount of protein or gene transcript of the gene TRPV2 in a biological sample obtained from an individual suffering from a hypoxic-ischemic encephalopathy from about 1.1 to about 10.0 fold, or from about 1.2 to about 8.0 fold, or from about 1.3 to about 6.0 fold or from about 1.4 to about 5.0 fold a reference value or value of untreated individuals suffering from a hypoxic-ischemic encephalopathy. Administration of therapeutic agent may increase an amount of protein or gene transcript of the gene TRPV2 above the reference value or range for the protein or gene transcript of the gene TRPV2.

The amount of protein or gene transcript of the gene TXN2 in a biological sample obtained from an individual suffering from a hypoxic-ischemic encephalopathy is significantly increased compared with the amount of protein or gene transcript of the gene TXN2 in a biological sample obtained from a healthy individual or a reference value. For example, the increase of the measured amount of protein or gene transcript of the gene TXN2 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may restore an amount of protein or gene transcript of the gene TXN2 within the reference value or range for the protein or gene transcript of the gene TXN2.

The amount of protein or gene transcript of the gene GFAP in a biological sample obtained from an individual suffering from a hypoxic-ischemic encephalopathy is significantly increased compared with the amount of protein or gene transcript of the gene GFAP in a biological sample obtained from a healthy individual or a reference value. For example, the increase of the measured amount of protein or gene transcript of the gene GFAP in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may restore an amount of protein or gene transcript of the gene GFAP within the reference value or range for the protein or gene transcript of the gene GFAP.

The amount of protein or gene transcript of the gene IBA-1 in a biological sample obtained from an individual suffering from a hypoxic-ischemic encephalopathy is significantly increased compared with the amount of protein or gene transcript of the gene IBA-1 in a biological sample obtained from a healthy individual or a reference value. For example, the increase of the measured amount of protein or gene transcript of the gene IBA-1 in a biological sample obtained from an individual suffering from a hypoxic-ischemic encephalopathy may be from about 1.1 to about 10.0 fold, or from about 1.2 to about 8.0 fold, or from about 1.3 to about 6.0 fold or from about 1.4 to about 5.0 fold the reference value, the reference value being from healthy individuals. Administration of therapeutic agent may restore an amount of protein or gene transcript of the gene IBA-1 within the reference value or range for the protein or gene transcript of the gene IBA-1.

### Dosage of the biomarkers

In some embodiments, the step of measuring an amount of a protein may be performed by using one or more techniques selected from the group consisting of gas chromatography, liquid chromatography, mass spectrometry, immunoassay, ELISA, enzymatic or biochemical reactions and NMR, or combinations of two or more of these methods.

In some embodiments, a mass-spectrometry may be used to determine the presence or absence of a measured protein in a sample.

A measure by mass-spectrometry has the advantages that determining the amount of a protein can be realized with high precision and sensitivity. Further, such embodiment has the advantages that this technique is able to measure a huge number of proteins in a sample; in other words, with this technique a user might be able to measure the amount from lots of different proteins in just one run. Furthermore, mass spectrometry has the advantage that the detection of proteins can be realized from micro liter quantities of the biological sample.

As example of mass-spectrometry technique which may be used, one may refer to MS/MS technique. In such embodiment, peptide tolerance, MS/MS fragment tolerance, and a maximum of missed cleavages may be set at 5 ppm, 0.02 Da and 2, respectively. Carbamidomethylation of cysteine may be considered as fixed modification. Oxidation of methionine may be taken into account as variable modification. Peptides identified at a p-value ≤ 0.05 in homology threshold mode and proteins identified with at least two distinct peptides may be selected (false discovery rate below 1%).

In some embodiments, an immunoassay of a sample may be used to obtain an absolute or relative amount or concentration of a measured protein in a sample. A measure by immunoassay has the advantage that this technique is quite cheap in building, but highly sensitive in measuring the amount of at least one protein.

Various well-known immunological methods may be used to specifically identify and/or quantify the disclosed proteins. These methods include, but are not limited to, immunologic- or antibody-based techniques include enzyme-linked immunosorbent assay (ELISA), radioimmunoassay (RIA), western blotting, immunofluorescence, microarrays, some chromatographic techniques (i.e., immunoaffinity chromatography), flow cytometry, immunoprecipitation. These methods are based on the specificity of an antibody or antibodies for a particular epitope or combination of epitopes associated with the protein of interest.

Methods of producing antibodies for use in protein or antibody arrays, or other immunology based assays for detection of the proteins disclosed herein are known in the art. For preparation of monoclonal antibodies directed towards a protein, any technique that provides for the production of antibody molecules by continuous cell lines in culture may be used. Such techniques include, but are not limited to, the hybridoma technique originally developed by Kohler and Milstein Nature (1975) 256:495-497, the trioma technique (Gustafsson et al., Hum. Antibodies Hybridomas (1991) 2:26-32), the human B-cell hybridoma technique (Kozbor et al., Immunology Today (1983) 4:72), and the EBV hybridoma technique to produce human monoclonal antibodies (Cole et al., In: Monoclonal Antibodies and Cancer Therapy, Alan R. Liss, Inc., (1985) 77-96). In addition, techniques described for the production of single chain antibodies (U.S. Pat. No. 4,946,778) can be adapted to produce a protein-specific antibodies. An additional embodiment of may utilize the techniques described for the construction of Fab expression libraries (Huse et al., Science (1989) 246:1275-1281) to allow rapid and easy identification of monoclonal Fab fragments with the desired specificity for a protein. Non-human antibodies can be "humanized" by known methods (e.g., U.S. Pat. No. 5,225,539).

Antibody fragments that contain the idiotypes of a protein can be generated by techniques known in the art. For example, such fragments include, but are not limited to, the F(ab')2 fragment which can be produced by pepsin digestion of the antibody molecule; the Fab' fragment that can be generated by reducing the disulfide bridges of the F(ab')2 fragment; the Fab fragment that can be generated by treating the antibody molecular with papain and a reducing agent; and Fv fragments. Synthetic antibodies, e.g., antibodies produced by chemical synthesis, are useful in the present disclosure.

Antibodies or fragments thereof used in methods for measuring amounts of proteins may bear a reporter molecule. Numerous labels or reporter molecules may be used, such as:
(a) Radioisotopes, such as 35S, 14C, 125I, 3H, and 1311. Radioactivity can be measured using scintillation counting. Other radionuclides include 99Tc, 90Y, 111In, 32P, 11C, 15O, 13N, 18F, 51Cr, 57To, 226Ra, 60Co, 59Fe, 57Se, 152Eu, 67CU, 217Ci, 211At, 212Pb, 47Sc, 109Pd, 234Th, and 40K, 157Gd, 55Mn, 52Tr, and 56Fe.
(b) Colloidal gold particles.
(c) Fluorescent or chemiluminescent labels including, but not limited to, rare earth chelates (europium chelates), fluorescein and its derivatives, rhodamine and its derivatives, isothiocyanate, phycoerythrin, phycocyanin, allophycocyanin, o-phthaladehyde, fluorescamine, dansyl, umbelliferone, luciferin, luminal label, isoluminal label, an aromatic acridinium ester label, an imidazole label, an acridimium salt label, an oxalate ester label, an aequorin label, 2,3-dihydrophthalazinediones, Texas Red, dansyl, Lissamine, umbelliferone, phycocrytherin, phycocyanin, or commercially available fluorophores such SPECTRUM ORANGE^{®} and SPECTRUM GREEN^{®} and/or derivatives of any one or more of the above. The fluorescent labels can be conjugated to the antibody using the techniques disclosed in Current Protocols in Immunology, supra, for example. Fluorescence can be quantified using a fluorimeter.
(d) Enzyme catalyzing a substrate-based colorimetric reaction. Various enzyme-substrate labels are available. The enzyme generally catalyzes a chemical alteration of the chromogenic substrate that can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor.

Examples of enzymatic labels include luciferases (e.g., firefly luciferase and bacterial luciferase), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like.

Numerous enzyme-substrate combinations are available to those skilled in the art. Examples of enzyme-substrate combinations are:
(i) Horseradish peroxidase (HRP) with hydrogen peroxidase as a substrate, wherein the hydrogen peroxidase oxidizes a dye precursor, such as, e.g., 3,3' diamino benzidine (DAB), which produces a brown end product; 3-amino-9-ethylcarbazole (AEC), which upon oxidation forms a rose-red end product; 4-chloro-1-napthol (CN), which precipitates as a blue end product; and p-Phenylenediamine dihydrochloride/pyrocatecol, which generates a blue-black product; orthophenylene diamine (OPD) and 3,3',5,5'-tetramethyl benzidine hydrochloride (TMB);
(ii) alkaline phosphatase (AP) and para-Nitrophenyl phosphate, naphthol AS-MX phosphate, Fast Red TR and Fast Blue BB, napthol AS-BI phosphate, napthol AS-TR phosphate, 5-bromo-4-chloro-3-indoxyl phosphate (BCIP), Fast Red LB, Fast Garnet GBC, Nitro Blue Tetrazolium (NBT), and iodonitrotetrazolium violet (INT); and
(iii) β-D-galactosidase (β-D-Gal) with a chromogenic substrate (e.g., p-nitrophenyl-β-D-galactosidase) or fluorogenic substrate (e.g., 4-methylumbelliferyl-β-D-galactosidase).

In one embodiment, the label or reporter molecule may be selected in the group consisting of a fluorescent molecule, a radioisotope, an enzyme, a biotin, a streptavidin. For example, a label may be a fluorescent molecule.

In some embodiments, a protein may be measured by a chemiluminescent immunoassay. For example, a sample is added to a reaction vessel coated with a monoclonal anti-protein, blocking reagent. After incubation in a reaction vessel, unbound materials are washed away. Then, an enzyme able to catalyze a colorimetric reaction, such as an alkaline phosphatase, conjugated to an anti-protein antibody is added to the vessel with a chemiluminescent substrate and light generated by the reaction is measured with a luminometer. The light production is directly proportional to the level of protein in the sample. The amount of protein in the sample is determined from a stored, multi-point calibration curve.

In some embodiments, a protein may be measured by an immunofluorescent assay. For example, a sample is added to a reaction vessel along coated with a monoclonal anti-protein and blocking reagent. After incubation in a reaction vessel, unbound materials are washed away. Then, an anti-protein antibody conjugated to a fluorescence label is added to the vessel and a fluorescent signal is measured with a fluorometer. The fluorescent signal is directly proportional to the amount of protein in the sample. The amount of protein in the sample is determined from a stored, multi-point calibration curve.

Numerous variations of those chemiluminescent and immunofluorescent assays are known in the art.

Further, non-immunological methods, based on the physical or chemical properties of the proteins, can also be used to measure the disclosed proteins. Numerous methods are well known in the art and can be used to analyze/detect products of various reactions involving a protein disclosed herein. The reaction products can be detected by means of fluorescence, luminescence, mass measurement, or electrophoresis, etc. Furthermore, reactions can occur in solution or on a solid support such as a glass slide, a chip, a bead, or the like.

In some embodiments, the step of measuring an amount of a gene transcript may be performed by using one or more techniques selected from the group consisting of Quantitative polymerase chain reaction (qPCR), Northern blotting, DNA microarray analysis, SAGE (Serial Analysis of Gene Expression), RNA-seq (RNA sequencing), Nuclear run-on (NRO), or combinations of two or more of these methods

In some embodiments, an amount of gene transcript is measured by qPCR or a high-throughput sequencing process.

qPCR and or high-throughput sequencing process are well-known in the art.

qPCR involves reverse transcribing the gene transcript into DNA using reverse transcriptase (RT) with primers designed target the desired gene transcript sequence. The obtained DNA sequence is amplified using a thermocycler and Taq polymerase. The amplification is monitored in real time using fluorescent probes or dyes. The relative abundance of the target sequence is determined based on the fluorescence signal using fluorescent probes such as SYBR Green I (SYBR) or TaqMan probes.

High-throughput sequencing, also known as next-generation sequencing (NGS), allows for the rapid and parallel analysis of multiple nucleic acid sequences, enabling efficient large-scale genomic and transcriptomic studies. Illumina sequencing, 454 sequencing, Ion Torrent sequencing, and PacBio sequencing are examples of commercially available high-throughput sequencing platforms.

High-throughput sequencing may use reversible dye-terminators and a sequencing-by-synthesis approach. DNA fragments are immobilized on a solid support, and sequencing-by-synthesis occurs through the addition of fluorescently labeled nucleotides. The resulting fluorescence signals are captured, and base calls are determined, allowing the reconstruction of the original nucleic acid sequence.

High-throughput sequencing may use pyrosequencing technology. Adenosine triphosphate (ATP) is released as a byproduct of nucleotide incorporation during sequencing. The released ATP is then detected, and the sequence is determined by the order of nucleotide additions.

High-throughput sequencing may use the detection of hydrogen ions released during nucleotide incorporation. The pH changes resulting from hydrogen ion release are measured, and the nucleotide sequence is inferred.

High-throughput sequencing may use single-molecule, real-time (SMRT) sequencing. DNA polymerase catalyzes the incorporation of nucleotides into a single-stranded template, and the resulting fluorescence signals are directly detected in real-time.

### Biological samples

In some embodiments, a dosage of a biomarker may be carried in a biological sample. A biological sample may be selected from blood sample, plasma sample, serum sample, brain tissue sample, and cerebrospinal fluid sample.

A biological sample may be obtained from a biological model. A biological model may be a Rice-Vanucci model rat, obtained as disclosed in the **Examples section.**

A biological sample is an isolated biological sample.

### Kits-of parts

An object of the present disclosure relates to a kit-of-parts for use in a method as disclosed herein.

A kit-of-parts of the disclosure may comprise one or more other containers, containing for example, wash reagents or buffers.

A kit-of-parts may comprise means for acquiring a quantity of a biological sample, such as a blood or serum sample; wash reagents and buffers and means to detect and quantify the proteins as disclosed herein.

The kit-of-parts may be arranged in a way that the biological sample just has to be inserted into the kit, while a method disclosed herein may be carried out fully automatically. Therefore, an unskilled person may be able to use the kit.

The kit-of-parts may also include reagents which might be necessary for performing the methods disclosed herein. The reagents may be provided in any suitable form. The reagents might be stabilization reagents, buffer reagents, solvents etc.

The kit-of-parts may include means which are necessary for determining the amounts of a biomarker as disclosed herein.

This might be chromatographic means, spectrometric means, means for enzymatic or biochemical reactions, means for immunoassay, etc., as well as means for stabilizing the biological sample. The kit-of-parts might not be limited in just one technical means for determining the amount of a biomarker. For example, the kit-of-parts might have means for performing a HPLC (high performance liquid chromatography) or UHPLC (ultra-high performance liquid chromatography) coupled to a mass spectrometry or coupled to an immunoassay, or qPCR or High-throughput sequencing.

In some embodiments, the kit-of-parts may comprise a) means for measuring an amount of at least one protein or of at least one gene transcript from a gene or a combination of genes selected from the group of genes as defined and b) a set of instructions for carrying out said measure.

In some embodiments, a-1) the means for determining the amount of a protein may be configured for performing an immunoassay and/or a mass-spectrometric-based assay.

In some embodiments, a kit-of-parts disclosed herein may comprise immunoassay means for determining an amount of a biomarker. In this embodiment, an immunoassay might be an ELISA. Therefore, a kit-of-parts may comprise separate compartments, tubes, valves and the like. A kit-of-parts may also include reagents which are necessary for performing an immunoassay, like antibodies, buffers, blocking agents, detection reagents and the like. These reagents are well known in the art. For example, a kit-of-parts may comprise antibodies or fragments thereof, specific for the proteins markers (primary antibodies), along with one or more secondary antibodies that may incorporate a detectable label; such antibodies may be used in an assay such as an ELISA. Alternately, the antibodies or fragments thereof may be fixed to a solid surface, e.g., an antibody array. The kit-of-parts may contain a detectable label such as fluorescein, green fluorescent protein, rhodamine, cyanine dyes, Alexa dyes, luciferase, radiolabels, among others.

In some embodiments, a kit-of-parts disclosed herein may comprise a mass-spectrometer as means for determining an amount of a protein.

In some embodiments, a-2) the means for determining the amount of a gene transcript may be configured for performing a quantitative polymerase chain reaction (qPCR) or a high-throughput sequencing process.

According to another of its objects, the present disclosure relates to a kit-of-parts according to the disclosure for use in a method as disclosed herein.

The kit-of-parts as disclosed herein may further comprise a set of instructions to compare the measured amounts of the proteins with reference values or to compare the measured amounts of gene transcripts with reference values.

### Uses

The uses of the disclosure may be *in vivo* or *in vitro.* In some embodiments the uses may be *in vitro.*

In one of its objects, the disclosure relates to a use of protein or gene transcript from a gene or from a combination of genes selected from a group of genes comprising or consisting of AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, SERPINA3N, TXN2, GFAP, and IBA-1, as a biomarker of a hypoxic-ischemic encephalopathy.

In some embodiments, the disclosure relates to a use of protein or gene transcript from a gene or from a combination of genes selected from a group of genes comprising or consisting of AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N as a biomarker of a hypoxic-ischemic encephalopathy.

In some embodiments, the disclosure relates to a use of protein or gene transcript from a gene or from a combination of genes selected from a group of genes comprising or consisting of TXN2, GFAP and IBA-1 as a biomarker of a hypoxic-ischemic encephalopathy.

In some embodiments, the disclosure relates to a use of protein or gene transcript from a gene or from a combination of genes selected from a group of genes comprising or consisting of AGRN, ZYX, VWF, VEGFA, ANGT1, ANGT2, APCDD1, LETM1, PACS1, TXN2, GFAP, and IBA-1 as a biomarker of a hypoxic-ischemic encephalopathy.

In some embodiments, the disclosure relates to a use of protein or gene transcript from a gene or from a combination of genes selected from a group of genes comprising or consisting of AGRN, ZYX, VWF, VEGFA, ANGT1, ANGT2, and APCDD1 as a biomarker of a hypoxic-ischemic encephalopathy.

In some embodiments, the disclosure relates to a use of protein or gene transcript from a gene or from a combination of genes selected from a group of genes comprising or consisting of AGRN and ZYX, as a biomarker of a hypoxic-ischemic encephalopathy.

The biomarkers disclosed herein may be for use in methods for diagnosing or aiding in diagnosing a hypoxic-ischemic encephalopathy.

The biomarkers disclosed herein may be for use in methods for monitoring an evolution of a hypoxic-ischemic encephalopathy.

In one of its objects, the disclosure relates to a use of protein or gene transcript from a gene or from a combination of genes selected from a group of genes comprising or consisting of AGRN, ZYX, VWF, VEGFA, ANGT1, ANGT2, APCDD1, ADGRL1, ATP2A2, ERC2, LETM1, NCALD, NCS1 , SESTD1, SYT2, SEC14, PACS1, RAB1A, MAP2, NEUN, SLC17A7, GABRR1, SERPINA3N, TXN2, GFAP, IBA-1, SYT5, CDK5, HINT1, MARCKSL1, NIPSNAP2, PACS2, and TRPV2, as a biomarker for monitoring a therapeutic efficacy of a therapeutic treatment proposed for preventing and/or treating a hypoxic-ischemic encephalopathy in an individual in need thereof or for selecting a candidate therapeutic agent for preventing and/or treating a hypoxic-ischemic encephalopathy in an individual in need thereof.

The biomarkers disclosed herein may be for use in methods for monitoring a therapeutic efficacy of a therapeutic treatment proposed for preventing and/or treating a hypoxic-ischemic encephalopathy.

The biomarkers disclosed herein may be for use in methods for selecting a candidate therapeutic agent for preventing and/or treating a hypoxic-ischemic encephalopathy.

The biomarkers disclosed herein may be for use in methods of diagnosing and treating an individual susceptible to suffer from a hypoxic-ischemic encephalopathy.

In some embodiments, the biomarker may be protein or gene transcript form a gene or a combination of genes comprising at least or consisting of:
- AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, SERPINA3N, SYT5, CDK5, HINT1, MARCKSL1, NIPSNAP2, PACS2, and TRPV2,
   or
- AGRN, ZYX, VWF, VEGFA, ANGT1, ANGT2, APCDD1, SEC14, PACS1, RAB1A, MAP2, NEUN, SLC17A7, GABRR1, SERPINA3N, SYT5, CDK5, HINT1, MARCKSL1, NIPSNAP2, PACS2, and TRPV2,
   or
- AGRN, ZYX, VWF, VEGFA, ANGT1, ANGT2, APCDD1, SEC14, PACS1, RAB1A, MAP2, NEUN, SLC17A7, GABRR1, SERPINA3N, and SYT5,
   or
- AGRN, ZYX, VWF, VEGFA, ANGT1, ANGT2, APCDD1, SEC14, PACS1, RAB1A, and SYT5,
   or
- AGRN, ZYX, VWF, VEGFA, ANGT1, ANGT2, APCDD1, and SYT5,
   or
- AGRN, ZYX and SYT5
   or
- AGRN and ZYX.

According to another of its objects, the disclosure relates to an antibody for use in a method as disclosed herein. An antibody may be as disclosed in the Example section.

According to another of its objects, the disclosure relates to a set of primers for use in a method as disclosed herein. A set of primers may be as disclosed in the Example section.

### Methods

The methods of the disclosure may be *in vivo* or *in vitro.* In some embodiments, the methods of the disclosure may be *in vitro* methods.

The methods of the disclosure may be for diagnosing or for diagnosing and treating a hypoxic-ischemic encephalopathy, or for monitoring a therapeutic efficacy of a candidate therapeutic agent proposed for preventing and/or treating a hypoxic-ischemic encephalopathy, or for selecting a candidate therapeutic agent for preventing and/or treating a hypoxic-ischemic encephalopathy, or for monitoring an evolution of a hypoxic-ischemic encephalopathy.

One objects of the disclosure relates to a method for diagnosing a hypoxic-ischemic encephalopathy in an individual in need thereof, said method comprising at least the steps of:
a) measuring an amount of at least one protein or of gene transcript from a gene or from a combination of genes, the gene or the combination of genes being selected from a group of genes comprising or consisting of AGRN (Agrin), ZYX (Zyxin), VWF (Von Willebrand factor), VEGFA (Vascular endothelial growth factor A), ANGT1 (Angiopoietin 1), ANGT2 (Angiopoietin 2), APCDD1 (APC down-regulated 1), ADGRL1 (Adhesion G Protein-Coupled Receptor L1), ATP2A2 (ATPase sarcoplasmic/endoplasmic reticulum Ca2+ transporting 2), ERC2 (ELKS/RAB6-Interacting/CAST family member2), LETM1 (Leucine Zipper and EF-hand containing transmembrane protein 1), NCALD (Neurocalcin delta), NCS1 (Neuronal calcium sensor 1), SESTD1 (SEC14 and Spectrin domain containing 1), SYT2 (Synaptotagmin-2), SEC14 (SEC14-like protein), PACS1 (Phosphofurin acidic cluster sorting protein 1), RAB1A (RAB1A, member RAS oncogene family), MAP2 (Microtubule associated protein 2), NEUN (Neuronal nuclei), SLC17A7 (Solute carrier family 17 member 7), GABRR1 (Gamma-aminobutyric acid type A receptor rho1 subunit), SERPINA3N (Serine (or cysteine) peptidase inhibitor, clade A, member 3N), TXN2 (thioredoxin 2), GFAP (Glial fibrillary acidic protein), and IBA-1 (Ionized calcium-binding adapter molecule), in an isolated biological sample obtained from said individual, and
b) comparing the amount measured at step a) with a reference value,
wherein a difference deviation observed between the measured amount at step a) and the reference value is indicative of a hypoxic-ischemic encephalopathy in said individual.

An observed decrease of the amount of protein or gene transcript of a gene or a combination of genes selected from AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N compared to a reference value may be indicative of a hypoxic-ischemic encephalopathy.

An observed increase of the amount of protein or gene transcript of a gene or a combination of genes selected from TXN2, GFAP, and IBA-1 compared to a reference value may be indicative of a hypoxic-ischemic encephalopathy.

An observed decrease of the amount of protein or gene transcript of a gene or a combination of genes selected from AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N compared to a reference value and an observed increase of the amount of protein or gene transcript of a gene or a combination of genes selected from TXN2, GFAP, and IBA-1 compared to a reference value may be indicative of a hypoxic-ischemic encephalopathy.

In some embodiments, the measures may be varied on protein or gene transcript from a combination of genes comprising or consisting of:
- AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N, or
- TXN2, GFAP and IBA-1, or
- AGRN, ZYX, VWF, VEGFA, ANGT1, ANGT2, APCDD1, LETM1, PACS1, TXN2, GFAP, and IBA-1, or
- AGRN, ZYX, VWF, VEGFA, ANGT1, ANGT2, and APCDD1, or
- AGRN and ZYX.

In some embodiments, an observed decrease of the amount of protein or gene transcript of a gene or a combination of genes selected from AGRN and ZYX compared to a reference value may be indicative of a hypoxic-ischemic encephalopathy.

In the method, a reference value may be obtained from a healthy individual or from a group of healthy individuals.

A method as disclosed herein may be for monitoring an evolution of a hypoxic-ischemic encephalopathy in an individual in need thereof, said method comprising the steps of:
a) measuring an amount of protein or of gene transcript from a gene or from a combination of genes selected from the group of genes comprising or consisting of AGRN (Agrin), ZYX (Zyxin), VWF (Von Willebrand factor), VEGFA (Vascular endothelial growth factor A), ANGT1 (Angiopoietin 1), ANGT2 (Angiopoietin 2), APCDD1 (APC down-regulated 1), ADGRL1 (Adhesion G Protein-Coupled Receptor L1), ATP2A2 (ATPase sarcoplasmic/endoplasmic reticulum Ca2+ transporting 2), ERC2 (ELKS/RAB6-Interacting/CAST family member 2), LETM1 (Leucine Zipper and EF-hand containing transmembrane protein 1), NCALD (Neurocalcin delta), NCS1 (Neuronal calcium sensor 1), SESTD1 (SEC14 and Spectrin domain containing 1), SYT2 (Synaptotagmin-2), SEC14 (SEC14-like protein), PACS1 (Phosphofurin acidic cluster sorting protein 1), RAB1A (RAB1A, member RAS oncogene family), MAP2 (Microtubule associated protein 2), NEUN (Neuronal nuclei), SLC17A7 (Solute carrier family 17 member 7), GABRR1 (Gamma-aminobutyric acid type A receptor rho1 subunit), SERPINA3N (Serine (or cysteine) peptidase inhibitor, clade A, member 3N), TXN2 (thioredoxin 2), GFAP (Glial fibrillary acidic protein), and IBA-1 (Ionized calcium-binding adapter molecule), in an isolated biological sample obtained from said individual at a first point of time,
b) measuring an amount of protein or of gene transcript from a gene or from a combination of genes selected from the group of genes as defined in step a), in an isolated biological sample obtained from said individual at a second point of time, the gene or combination of genes of step a) and b) being the same, and
c) comparing the amount measured at step a) with the amount measured at step b),
wherein a deviation observed between the amounts measured at step a) and at step b) is indicative of an improvement or an aggravation of the hypoxic-ischemic encephalopathy in said individual.

An observed decrease of the amount of protein or gene transcript of a gene or a combination of genes selected from AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N compared to a reference value may be indicative of an aggravation of a hypoxic-ischemic encephalopathy.

An observed increase of the amount of protein or gene transcript of a gene or a combination of genes selected from TXN2, GFAP, and IBA-1 compared to a reference value may be indicative of an aggravation of a hypoxic-ischemic encephalopathy.

An observed decrease of the amount of protein or gene transcript of a gene or a combination of genes selected from AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N compared to a reference value and an observed increase of the amount of protein or gene transcript of a gene or a combination of genes selected from TXN2, GFAP, and IBA-1 compared to a reference value may be indicative of an aggravation of a hypoxic-ischemic encephalopathy.

In some embodiments, the measures may be varied on protein or gene transcript from a combination of genes comprising or consisting of:
- AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N, or
- TXN2, GFAP and IBA-1, or
- AGRN, ZYX, VWF, VEGFA, ANGT1, ANGT2, APCDD1, LETM1, PACS1, TXN2, GFAP, and IBA-1, or
- AGRN, ZYX, VWF, VEGFA, ANGT1, ANGT2, and APCDD1, or
- AGRN and ZYX.

In some embodiments, an observed decrease of the amount of protein or gene transcript of a gene or a combination of genes selected from AGRN and ZYX compared to a reference value may be indicative of an aggravation of a hypoxic-ischemic encephalopathy.

In the method, a reference value may be obtained from an individual or from a group of individuals suffering from hypoxic-ischemic encephalopathy.

An observed increase of the amount of protein or gene transcript of a gene or a combination of genes selected from AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N compared to a reference value may be indicative of an improvement of a hypoxic-ischemic encephalopathy.

An observed decrease of the amount of protein or gene transcript of a gene or a combination of genes selected from TXN2, GFAP, and IBA-1 compared to a reference value may be indicative of an improvement of a hypoxic-ischemic encephalopathy.

An observed increase of the amount of protein or gene transcript of a gene or a combination of genes selected from AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N compared to a reference value and an observed decrease of the amount of protein or gene transcript of a gene or a combination of genes selected from TXN2, GFAP, and IBA-1 compared to a reference value may be indicative of an improvement of a hypoxic-ischemic encephalopathy.

In some embodiments, an observed increase of the amount of protein or gene transcript of a gene or a combination of genes selected from AGRN and ZYX compared to a reference value may be indicative of an improvement of a hypoxic-ischemic encephalopathy.

In the method, a reference value may be obtained from a healthy individual or from a group of healthy individuals.

A method for monitoring an evolution of a hypoxic-ischemic encephalopathy may comprise in addition to the 1^{st} and 2^{nd} measures further subsequent measures taken at subsequent times. For example, a method may comprise a 3^{rd}, a 4^{th}, a 5^{th}, a 6^{th}, a 7^{th}, an 8^{th}, a 9^{th}, a 10^{th} or more measure.

In a method disclosed herein the measures may be repeated at regular or irregular intervals. For example, the measures may be carried every day, once a week every week, or every 2, 3, o r4 weeks, once a month every month, or every 2, 3, 4, 5, 6, 7, 8, 9, 10, 11 months, or once year every year, or every 2, 3, or 4 years. The measures may be carried several times a week, a month or a year, for example, twice, or three, four, five or six time a week, or more in month or in a year. The measures may be carried out over a period of time ranging from one week to one year, or more.

One objects of the disclosure relates to a method for monitoring a therapeutic efficacy of a therapeutic treatment proposed for preventing and/or treating a hypoxic-ischemic encephalopathy in an individual in need thereof, said method comprising the steps of:
a) measuring an amount of protein or of gene transcript from a gene or from a combination of genes selected from a group of genes comprising or consisting of AGRN (Agrin), ZYX (Zyxin), VWF (Von Willebrand factor), VEGFA (Vascular endothelial growth factor A), ANGT1 (Angiopoietin 1), ANGT2 (Angiopoietin 2), APCDD1 (APC down-regulated 1), ADGRL1 (Adhesion G Protein-Coupled Receptor L1), ATP2A2 (ATPase sarcoplasmic/endoplasmic reticulum Ca2+ transporting 2), ERC2 (ELKS/RAB6-Interacting/CAST family member 2), LETM1 (Leucine Zipper and EF-hand containing transmembrane protein 1), NCALD (Neurocalcin delta), NCS1 (Neuronal calcium sensor 1), SESTD1 (SEC14 and Spectrin domain containing 1), SYT2 (Synaptotagmin-2), SEC14 (SEC14-like protein), PACS1 (Phosphofurin acidic cluster sorting protein 1), RAB1A (RAB1A, member RAS oncogene family), MAP2 (Microtubule associated protein 2), NEUN (Neuronal nuclei), SLC17A7 (Solute carrier family 17 member 7), GABRR1 (Gamma-aminobutyric acid type A receptor rho1 subunit), SERPINA3N (Serine (or cysteine) peptidase inhibitor, clade A, member 3N), TXN2 (thioredoxin 2), GFAP (Glial fibrillary acidic protein), IBA-1 (Ionized calcium-binding adapter molecule), SYT5 (Synaptotagmin-5), CDK5 (Cyclin dependent kinase 5), HINT1 (Histidine triad nucleotide binding protein 1), MARCKSL1 (Myristoylated alanine-rich C-kinase substrate Like 1), NIPSNAP2 (Nipsnap Homolog 2), PACS2 (Phosphofurin acidic cluster sorting protein 2), and TRPV2 (Transient receptor potential cation channel subfamily V member 2), in an isolated biological sample obtained from said individual before administration of said therapeutic treatment,
b) measuring an amount of protein or of gene transcript from a gene or a combination of genes selected from a group the genes as defined in step a), in an isolated biological sample obtained from said individual after administration of said therapeutic treatment, the gene or combination of genes of step a) and b) being the same, and
c) comparing the amount measured at step a) with the amount measured at step b),
wherein a deviation observed between the amounts measured at step a) and at step b) is indicative of a therapeutic efficacy of said therapeutic treatment on said hypoxic-ischemic encephalopathy.

A therapeutic treatment may be a therapeutic treatment agent such as (2R)-2-[[6-[Benzyl(ethyl)amino]-9-isopropyl-purin-2-yl]amino]butan-1-ol (BRT_001) or L-valyl ester of (2R)-2-[[6-[benzyl(ethyl)amino]-9-isopropyl-purin-2-yl] amino] butan-1-ol hydrochloride (BRT_002).

A therapeutic treatment may be subjecting said individual to hypothermia.

Another object of the disclosure is a method for selecting a candidate therapeutic agent for preventing and/or treating a hypoxic-ischemic encephalopathy in an individual in need thereof, said method comprising the steps of:
a) measuring an amount of protein or of gene transcript from a gene or from a combination of genes selected from a group of genes comprising or consisting of AGRN (Agrin), ZYX (Zyxin), VWF (Von Willebrand factor), VEGFA (Vascular endothelial growth factor A), ANGT1 (Angiopoietin 1), ANGT2 (Angiopoietin 2), APCDD1 (APC down-regulated 1), ADGRL1 (Adhesion G Protein-Coupled Receptor L1), ATP2A2 (ATPase sarcoplasmic/endoplasmic reticulum Ca²⁺ transporting 2), ERC2 (ELKS/RAB6-Interacting/CAST family member 2), LETM1 (Leucine Zipper and EF-hand containing transmembrane protein 1), NCALD (Neurocalcin delta), NCS1 (Neuronal calcium sensor 1), SESTD1 (SEC14 and Spectrin domain containing 1), SYT2 (Synaptotagmin-2), SEC14 (SEC14-like protein), PACS1 (Phosphofurin acidic cluster sorting protein 1), RAB1A (RAB1A, member RAS oncogene family), MAP2 (Microtubule associated protein 2), NEUN (Neuronal nuclei), SLC17A7 (Solute carrier family 17 member 7), GABRR1 (Gamma-aminobutyric acid type A receptor rho1 subunit), SERPINA3N (Serine (or cysteine) peptidase inhibitor, clade A, member 3N), TXN2 (thioredoxin 2), GFAP (Glial fibrillary acidic protein), IBA-1 (Ionized calcium-binding adapter molecule), SYT5 (Synaptotagmin-5), CDK5 (Cyclin dependent kinase 5), HINT1 (Histidine triad nucleotide binding protein 1), MARCKSL1 (Myristoylated alanine-rich C-kinase substrate Like 1), NIPSNAP2 (Nipsnap Homolog 2), PACS2 (Phosphofurin acidic cluster sorting protein 2), and TRPV2 (Transient receptor potential cation channel subfamily V member 2), in an isolated biological sample obtained from a biological model of a hypoxic-ischemic encephalopathy before contacting said biological model with said candidate therapeutic agent,
b) measuring an amount of protein or of gene transcript from a gene or a combination of genes selected from the group of genes as defined in step a), in an isolated biological sample obtained from said biological model of step a) after contacting said biological model with said candidate therapeutic agent, the gene or combination of genes of step a) and b) being the same,
c) comparing the amount measured at step a) with the amount measured at step b), and
d) selecting a candidate therapeutic agent for which a deviation observed between the amounts measured at step a) and at step b) is indicative of a therapeutic efficacy of said candidate therapeutic agent on said hypoxic-ischemic encephalopathy.

In embodiments, an observed increase of the amount of protein or gene transcript of a gene or a combination of genes selected from AGRN, ZYX, VWF, VEGFA, ANGT1, ANGT2, APCDD1, ADGRL1, ATP2A2, ERC2, LETM1, NCALD, NCS1 , SESTD1, SYT2, SEC14, PACS1, RAB1A, MAP2, NEUN, SLC17A7, GABRR1, SERPINA3N, SYT5, CDK5, HINT1, MARCKSL1, NIPSNAP2, PACS2, and TRPV2 at step b) compared to step a) may be indicative of a therapeutic efficacy of a therapeutic treatment or of a candidate therapeutic agent on the hypoxic-ischemic encephalopathy.

In embodiments, an observed decrease of the amount of protein or gene transcript of a gene or a combination of genes selected from TXN2, GFAP, IBA-1 at step b) compared to step a) may be indicative of a therapeutic efficacy of a therapeutic treatment or of a candidate therapeutic agent on the hypoxic-ischemic encephalopathy.

An observed increase of the amount of protein or gene transcript of a gene or a combination of genes selected from AGRN, ZYX, VWF, VEGFA, ANGT1, ANGT2, APCDD1, ADGRL1, ATP2A2, ERC2, LETM1, NCALD, NCS1 , SESTD1, SYT2, SEC14, PACS1, RAB1A, MAP2, NEUN, SLC17A7, GABRR1, SERPINA3N, SYT5, CDK5, HINT1, MARCKSL1, NIPSNAP2, PACS2, and TRPV2 and an observed decrease of the amount of protein or gene transcript of a gene or a combination of genes selected from TXN2, GFAP, and IBA-1 at step b) compared to step a) may be indicative of a therapeutic efficacy of a therapeutic treatment or of a candidate therapeutic agent on the hypoxic-ischemic encephalopathy.

In some embodiments, the combination of genes may comprise at least or consist of two genes.

In some embodiments, the combination of genes may comprise at least or consist of AGRN and ZYX, and optionally SYT5.

In some embodiments, the gene or combination of genes may comprise at least or consist of:
- AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, SERPINA3N, SYT5, CDK5, HINT1, MARCKSL1, NIPSNAP2, PACS2, and TRPV2,
   or
- AGRN, ZYX, VWF, VEGFA, ANGT1, ANGT2, APCDD1, SEC14, PACS1, RAB1A, MAP2, NEUN, SLC17A7, GABRR1, SERPINA3N, SYT5, CDK5, HINT1, MARCKSL1, NIPSNAP2, PACS2, and TRPV2,
   or
- AGRN, ZYX, VWF, VEGFA, ANGT1, ANGT2, APCDD1, SEC14, PACS1, RAB1A, MAP2, NEUN, SLC17A7, GABRR1, SERPINA3N, and SYT5,
   or
- AGRN, ZYX, VWF, VEGFA, ANGT1, ANGT2, APCDD1, SEC14, PACS1, RAB1A, and SYT5,
   or
- AGRN, ZYX, VWF, VEGFA, ANGT1, ANGT2, APCDD1, and SYT5,
   or
- AGRN, ZYX and SYT5
   or
- AGRN and ZYX.

According to another of its objects, the present disclosure relates to a method of diagnosing and treating an individual susceptible to suffer from a hypoxic-ischemic encephalopathy. The step of treating comprises administering to said individual diagnosed with a hypoxic-ischemic encephalopathy a therapeutic treatment proposed for preventing and/or treating a hypoxic-ischemic encephalopathy.

According to another of its objects, the present disclosure relates to a method of diagnosing and treating an individual susceptible to suffer from a hypoxic-ischemic encephalopathy, the method comprising at least the step of diagnosing a hypoxic-ischemic encephalopathy in said individual and a step of administering to said individual diagnosed a hypoxic-ischemic encephalopathy a therapeutic treatment proposed for preventing and/or treating a hypoxic-ischemic encephalopathy.

The method or the step of diagnosing comprises at the least the steps of:
a) measuring an amount of at least one protein or of gene transcript from a gene or from a combination of genes, the gene or the combination of genes being selected from a group of genes comprising or consisting of AGRN (Agrin), ZYX (Zyxin), VWF (Von Willebrand factor), VEGFA (Vascular endothelial growth factor A), ANGT1 (Angiopoietin 1), ANGT2 (Angiopoietin 2), APCDD1 (APC down-regulated 1), ADGRL1 (Adhesion G Protein-Coupled Receptor L1), ATP2A2 (ATPase sarcoplasmic/endoplasmic reticulum Ca2+ transporting 2), ERC2 (ELKS/RAB6-Interacting/CAST family member2), LETM1 (Leucine Zipper and EF-hand containing transmembrane protein 1), NCALD (Neurocalcin delta), NCS1 (Neuronal calcium sensor 1), SESTD1 (SEC14 and Spectrin domain containing 1), SYT2 (Synaptotagmin-2), SEC14 (SEC14-like protein), PACS1 (Phosphofurin acidic cluster sorting protein 1), RAB1A (RAB1A, member RAS oncogene family), MAP2 (Microtubule associated protein 2), NEUN (Neuronal nuclei), SLC17A7 (Solute carrier family 17 member 7), GABRR1 (Gamma-aminobutyric acid type A receptor rho1 subunit), SERPINA3N (Serine (or cysteine) peptidase inhibitor, clade A, member 3N), TXN2 (thioredoxin 2), GFAP (Glial fibrillary acidic protein), and IBA-1 (Ionized calcium-binding adapter molecule), in an isolated biological sample obtained from said individual, and
b) comparing the amount measured at step a) with a reference value,
wherein a difference deviation observed between the measured amount at step a) and the reference value is indicative of a hypoxic-ischemic encephalopathy in said individual.

The method or the step of treating comprises at least a step c) of administering to said individual diagnosed a hypoxic-ischemic encephalopathy a therapeutic treatment proposed for preventing and/or treating a hypoxic-ischemic encephalopathy.

In some embodiments, the present disclosure relates to a method of treating an individual susceptible to suffer from a hypoxic-ischemic encephalopathy, the method comprises at least the steps of:
a) measuring an amount of at least one protein or of gene transcript from a gene or from a combination of genes, the gene or the combination of genes being selected from a group of genes comprising or consisting of AGRN (Agrin), ZYX (Zyxin), VWF (Von Willebrand factor), VEGFA (Vascular endothelial growth factor A), ANGT1 (Angiopoietin 1), ANGT2 (Angiopoietin 2), APCDD1 (APC down-regulated 1), ADGRL1 (Adhesion G Protein-Coupled Receptor L1), ATP2A2 (ATPase sarcoplasmic/endoplasmic reticulum Ca2+ transporting 2), ERC2 (ELKS/RAB6-lnteracting/CAST family member2), LETM1 (Leucine Zipper and EF-hand containing transmembrane protein 1), NCALD (Neurocalcin delta), NCS1 (Neuronal calcium sensor 1), SESTD1 (SEC14 and Spectrin domain containing 1), SYT2 (Synaptotagmin-2), SEC14 (SEC14-like protein), PACS1 (Phosphofurin acidic cluster sorting protein 1), RAB1A (RAB1A, member RAS oncogene family), MAP2 (Microtubule associated protein 2), NEUN (Neuronal nuclei), SLC17A7 (Solute carrier family 17 member 7), GABRR1 (Gamma-aminobutyric acid type A receptor rho1 subunit), SERPINA3N (Serine (or cysteine) peptidase inhibitor, clade A, member 3N), TXN2 (thioredoxin 2), GFAP (Glial fibrillary acidic protein), and IBA-1 (Ionized calcium-binding adapter molecule), in an isolated biological sample obtained from said individual, and
b) comparing the amount measured at step a) with a reference value,
wherein a difference deviation observed between the measured amount at step a) and the reference value is indicative of a hypoxic-ischemic encephalopathy in said individual, and
c) administering to said individual diagnosed with a hypoxic-ischemic encephalopathy a therapeutic treatment proposed for preventing and/or treating a hypoxic-ischemic encephalopathy.

An observed decrease of the amount of protein or gene transcript of a gene or a combination of genes selected from AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N compared to a reference value may be indicative of a hypoxic-ischemic encephalopathy.

An observed increase of the amount of protein or gene transcript of a gene or a combination of genes selected from TXN2, GFAP, and IBA-1 compared to a reference value may be indicative of a hypoxic-ischemic encephalopathy.

An observed decrease of the amount of protein or gene transcript of a gene or a combination of genes selected from AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N compared to a reference value and an observed increase of the amount of protein or gene transcript of a gene or a combination of genes selected from TXN2, GFAP, and IBA-1 compared to a reference value may be indicative of a hypoxic-ischemic encephalopathy.

In some embodiments, the measures may be varied on protein or gene transcript from a combination of genes comprising or consisting of:
- AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N, or
- TXN2, GFAP and IBA-1, or
- AGRN, ZYX, VWF, VEGFA, ANGT1, ANGT2, APCDD1, LETM1, PACS1, TXN2, GFAP, and IBA-1, or
- AGRN, ZYX, VWF, VEGFA, ANGT1, ANGT2, and APCDD1, or
- AGRN and ZYX.

In some embodiments, an observed decrease of the amount of protein or gene transcript of a gene or a combination of genes selected from AGRN and ZYX compared to a reference value may be indicative of a hypoxic-ischemic encephalopathy.

In the method, a reference value may be obtained from a healthy individual or from a group of healthy individuals.

Suitable treatment for an individual diagnosed with a hypoxic-ischemic encephalopathy may include 2R)-2-[[6-[Benzyl(ethyl)amino]-9-isopropyl-purin-2-yl]amino]butan-1-ol (BRT_001), L-valyl ester of (2R)-2-[[6-[benzyl(ethyl)amino]-9-isopropyl-purin-2-yl] amino] butan-1-ol hydrochloride (BRT_002) or subjecting said individual to hypothermia.

It shall be understood that the features mentioned above and those to be mentioned in the following cannot only be used in the combinations indicated in each case but also in other combinations or in isolated positions without departing from the scope of the present disclosure.

The disclosure is now explained in more details by means of embodiments which result in further characteristics, features and advantages. The embodiments are purely illustrative and do not restrict the scope of the disclosure. Features which are described in connection with a specific embodiment are isolated features of the disclosure in its general form and are not only features in the specific technical context.

### [EXAMPLES]

The following examples illustrate the embodiments of the invention that are presently best known. However, it is to be understood that the following are only exemplary or illustrative of the application of the principles of the present invention. Numerous modifications and alternative compositions, methods, and systems may be devised by those skilled in the art without departing from the spirit and scope of the present invention. Thus, while the present invention has been described above with particularity, the following examples provide further detail in connection with what are presently deemed to be the most practical and preferred embodiments of the invention.

### Example 1: Materials & Methods

### Design and synthesis of BRT_002

A new trisubstituted purine BRT_001 was first identified. However, its low solubility limited its use in vivo. It was converted to its valyl ester BRT_002, as detailed in the Example 2, which could be used in animal studies.

### Animal preparation, study groups, and experimental design

### Study approval

The study to assess brain lesion-mediated HI in the Vannucci model was conducted after approval from the Institutional Animal Care and Use Committees of the Alpert Medical School of Brown University and Women & Infants Hospital of Rhode Island (study approval number 20-05-0006). All experimental procedures were carried out following the National Institutes of Health Guidelines for the Use of Experimental Animals. For PK studies, approval was obtained from the French Ministry of Health (authorization number: APAFIS#30306-2021031009268181 v1). Animal experiments were conducted in compliance with the European Communities Council Directive of 22 September 2010 and approved by the French Ministry of Health.

### Animal use for toxicity assessment

A toxicology study was carried out following the general requirements of GLP (Good Laboratory Practice). This study was conducted following Directive 2010/63/UE of the European Parliament and of the Council of 22 September 2010 for the protection of animals used for scientific purposes (approval for the site of experimentation: No. E 18-023-01). The aims of this phase of the study were to determine the MTD following a single oral dose of BRT_002 in Wistar rats and to establish a suitable dose range for repeated-dose toxicity studies. The toxicity assessment had two stages: an increasing dose level phase (MTD - stage 1) and a 7-day fixed dose phase (DRF - stage 2).

In step1, 2 × 4 groups of 3 males each were treated with 4 increasing doses of BRT_002. Each dose was followed by at least a one-day observation period, after which the next dose was given. Dosing was stopped if signs of intolerance occurred, or a dose of 100 mg/kg was reached. The animals used in step 1 were observed for an additional 2-3 days after BRT_002 administration before sacrifice. The choice of the doses is based on previous studies on the intended therapeutic dose in humans.

### Clinical signs

### General observations:

Animals were observed in the cage before the first dosing and then daily between 30 min and 2 h post-dose. The general disposition, behavior and activity were observed according to the following signs: body posture, central excitation, mood / awareness, motor activity and autonomic profile/miscellaneous.

### Functional and neurobehavioral tests.

Before the first dosing and, on the day of each treatment for phase 1 or on the last day of the treatment (day7) for phase 2, animals were observed according to a standardized observation battery for neurobehavioral, neurovegetative or psychotropic signs or neurotoxic effects and body temperature measured. The method is based on an Irwin screen modified by suppressing the graduation of intensity of clinical signs. Animals were observed individually in a cage without sawdust in a quiet room. Clinical signs were observed according to the following: behavior (awareness, mood, motor activity, motor coordination), neurologic profile (muscle tone, body posture, autonome profile, central excitations, reflexes). The time of observation was between at approximately 2 h post dose or at the peak of occurrence of any clinical signs if different.

### Clinical laboratory determination (phase 2)

Blood samples were taken from the retro-orbital sinus (or from another vessel) from animals following anesthesia by isoflurane inhalation. Sample volumes and anticoagulants were as follows: about 0.3 mL into EDTA tubes (hematology), about 1 mL into lithium heparin tubes (for analysis blood chemistry) and about 1.3 mL into citrated tubes (for coagulation parameters analysis). Tubes were kept at room temperature, until the analysis, for a maximum of 8 h. Labels on the tubes were marked in waterproof ink with at least Testing Facility, Animal Number, Study Number, Sampling Day, Test item, Dose or Group, Sex and Kind of Sampling. Hematology, Blood chemistry and coagulation parameters. They were determined using the general standard methods.

### Toxicokinetics - (Phase 2)

Blood samples for drug analysis were taken from satellite animals. Blood samples (0.3 mL) were drawn from the jugular vein or other vessels under isoflurane gas anesthesia and placed in lithium heparin. For treated animals, blood samples were taken at the following times: (i) on day 1: 1 h, 3 h, 8 h and 24 h and (ii) on day 7: predose, 1 h, 2 h, 3 h, 4 h and 8 h. For control animals, blood samples were taken at the following times: (i) on day 1: 1 h and 3 h and (ii) day 7: predose, 3 h and 8 h. The blood samples were cooled on ice, and plasma was prepared within 60 min of sampling by centrifugation for 10 min at 1500 g under refrigeration (+4 ± 2°C). Plasma was divided into polypropylene tubes, with 100 µl in one tube that was frozen within 90 min after blood sampling and stored frozen (≤-18°C) for drug analysis.

### Exposure to hypoxia ischemia (HI)

Pregnant Wistar rats were obtained from Charles River Laboratories (Wilmington, MA, USA) on embryonic day 15 or 16 and housed in a temperature-controlled facility in the Care Facility at Brown University on a 12-h light/dark cycle with *ad libitum* access to food and water. The dates upon which the rat pups were delivered were confirmed and designated P0. Litters were culled to a maximum of 10 pups on P1 with equal numbers of male and female pups to the greatest extent possible. On P7, the litters were randomly assigned to one of three groups: the sham-operated control (sham), HI+Vehicle-treated (Osredkar, D., et al., Resuscitation 85, 567-572 (2014)), or HI+BRT_002-treated group. The sex of each animal was recorded.

The Rice Vannucci model was used to induce HI-related brain injury (Rice, J.E., Vannucci, R.C. & Brierley, J.B., Annals of Neurology 9, 131-141 (1981)). Briefly, rat was anesthetized using 3-4% isoflurane, and the total absence of leg withdrawal reflexes was verified before proceeding with surgery. A midline ventral incision was made on the neck. The right common carotid artery (RCCA) was located and double ligated with a 5-0 silk suture. Sham animals were exposed to the same procedure except the RCCA was not ligated. The neck incision was closed with 5-0 silk suture. Body temperature was maintained at 36°C during surgery using an isothermal heating pad (Mishima, K., etal., Behavioural Brain Research 151, 209-217 (2004); Reinboth, B.S., et al., Experimental Neurology 283, 264-275 (2016); Silveira, R.C. & Procianoy, R.S., Jornal De Pediatria 91, S78-S83 (2015); Marks, K., Shany, E., Shelef, I., Golan, A. & Zmora, E., Israel Medical Association Journal 12, 494-500 (2010)). The pups were returned to their dams after the completion of surgery for 1.5-3 h of recovery before exposure to hypoxic conditions. The animals in the HI groups were placed in a hypoxia chamber with 8% humidified oxygen (balanced with nitrogen) at 36°C for 90 min. Sham animals were exposed to room air at 36°C for 90 min. This procedure has been found to result in reproducible levels of brain injury in P10 rats. Immediately after HI, the pups received intraperitoneal (IP) injections of 30 mg/kg BRT_002 or an equivalent volume of vehicle (normal saline/PEG400). The pups were then returned to their dams.

For pharmacokinetic (PK) studies, pups were sacrificed at 2, 4, 6 and 24 h after BRT_002 administration. Blood was collected by cardiac puncture, cerebral spinal fluid (CSF) was obtained with a micropipette, and the brain were perfused with PBS by cardiac puncture. Thereafter, the brains were removed, weighed and frozen.

### Infarct volume determinations

To measure the infarct volume, the pups received second and third doses of 30 mg/kg IP BRT_002 or vehicle at 24 h and 48 h after exposure to HI. Seventy-two hours after exposure to HI (P10), the pups were euthanized. The brain and blood were collected for further analyses. Some of the pups were transcardially perfused with normal saline followed by 10% PFA. Fixed tissue was then sectioned, mounted on glass slides, and processed for infarct volume and immunohistochemistry analysis. Each brain was divided into four or five 2-mm coronal sections using a brain slicer matrix (Zivic instruments, Pittsburgh, PA, USA), immersed in optimal cutting temperature (OCT) embedding medium, and frozen in a metal beaker filled with isopentane surrounded by crushed dry ice. Five cryosections (20 µm) were obtained from each 2-mm section and mounted on gelatin-coated slides. We randomly selected one of every fourth cryosection for cresyl violet staining to evaluate Hl-related brain injury. Images of the hemispheric and damaged areas were obtained using a Micropublisher 6 CCD camera (Qimaging, Surrey, British Columbia, Canada) and analyzed with imaged (NIH) by an experimenter blinded to group assignment. The respective volume of each measured area was calculated by multiplying the distance between sections. The infarct volume was calculated as a percentage of the ratio of the damaged volume to the total volume of the contralateral hemisphere with correction for hemispheric edema, according to the following formula: infarct (%) = [1 - (volume of total ipsilateral hemisphere - volume of infarct)/volume of total contralateral hemisphere)] x 100%. After the cryosections were obtained for analysis, the residual frozen tissue sections were paraffin-embedded for further immunohistological analysis.

### Drug quantification in rat serum and brain tissue

Brain and serum samples from P7 rats (sham and HI+BRT_002 groups; PK studies) as well as brain and plasma samples from adult rats (toxicokinetic studies) were used for drug quantification and PK parameter determination. A compound (BRT_001) with a structure close to that of BRT_001 was used as an internal standard. All solvents (VWR France) were LC/MS grade.

Brain samples were homogenized with two volumes of water using a Berlin Technologies Precellys homogenizer. Five microliters of the internal standard solution was added to 50 µL of rat plasma or brain homogenate, and then 200 µL of acetonitrile was added for protein precipitation. The whole sample was vortexed for 15 s and centrifuged for 10 min at 20,000 g and 5°C. The supernatant was diluted in a one-fifth volume of water and transferred into a new vial for analysis.

Five microliters of serum extract maintained at +5°C was injected into an LC-MS/MS system consisting of a Waters ACQUITY UPLC^{®} System with an Acquity UPLC BEH C18 2.1*50 mm column and run on a reversed-phase gradient for a 5-minute run time. A mix of mobile phase A (water and 0.1% formic acid) and mobile phase B (acetonitrile and 0.1% formic acid) was used at a flow rate of 0.5 mL/min and a column temperature of 50°C. The gradient was increased from 5% B to 100% B between 0.5 and 3 min, maintained between 3 and 3.5 min, decreased to 5% B at 3.51 min and maintained for up to 5.0 min for reequilibration. The mass spectrometry (MS) analysis was performed on a Waters XEVO^{™} TQ-S mass spectrometer operating in positive ion electrospray MRM mode. The multiple transitions monitored were m/z 482.3 > 364.9, 383.3 > 261.1 and 395.0 > 273.1 for BRT_002 and BRT_001, respectively. Under these conditions, the mean retention times were approximately 2.15 and 2.70 for BRT_002 and BRT_001, respectively.

Quantification was performed using linear regression with 1/X weighting and calibration ranges from 1.87 to 3215 ng/mL and 0.373 to 641 ng/mL for BRT_002 and BRT_001, respectively; a standard calibration curve and quality control samples prepared with a blank matrix spiked with compounds were used.

### Shotgun proteomics

Total proteins (15 µg) were extracted from the residual brain tissue sections, mixed with lithium dodecyl sulphate lysis buffer (Thermo, Fisher Scientific Inc.), and incubated at 99°C for 5 min. Proteins were then subjected to electrophoresis for 5 min at 200 V on a NuPAGE 4-12% Bis-Tris gel in 1X MES/SDS (Thermo) running buffer. Gels were stained with SimplyBlue SafeStain (Thermo) for 5 min, followed by an overnight wash in water with gentle agitation. The band containing the whole proteome from each sample was excised from the polyacrylamide gel and treated as previously described⁶⁴. The proteins were in-gel proteolyzed with trypsin gold (Promega Corporation, USA )in the presence of 0.01% Protease Max surfactant (Promega) at 50°C for 60 min. One µL of the resulting peptide fraction (of the 50 µL total volume), corresponding to approximately 300 ng of peptide, was analysed by liquid chromatography-tandem mass spectrometry (LC-MS/MS) with an Orbitrap Exploris 480 tandem mass spectrometer (Thermo Scientific) coupled to a Vanquish Neo UHPLC system, as previously described ^{65,64}. The peptides were loaded on a reverse-phase Acclaim PepMap 100 C18 precolumn (5 µm, 100 Å, 300 µm i.d. × 5 mm, Thermo Fisher) and then resolved on a Acclaim PepMap 100 C18 column (3 µm, 100 Å, 75 µm i.d. × 50 cm, Thermo Fisher) at a flow rate of 0.2 µL.min⁻¹ using a 90-min gradient (4-25% B in 75 min, and 25-40% B in 15 min), with 0.1% HCOOH/100% H₂O as mobile phase A and 0.1% HCOOH/100% CH3CN as mobile phase B. The mass spectrometer was operated in data dependent acquisition mode with a Top20 strategy, a scan range of 350 to 1800 m/z, and selection and fragmentation were performed using a 10 sec dynamic exclusion time. Only ion precursors with a 2+ or 3+ charge were selected for HCD fragmentation. Peptides and proteins were identified from the dataset using the MaxQuant software version 1.6.6.0 (Cox, J. & Mann. Nat Biotechnol 26, 1367-1372 (2008)). against the Rattus norvegicus (Norway rat) genome assembly mRatBN7.2. Carbamidomethylation of cysteine was set as a fixed modification, whereas variable modifications included oxidation of methionine, deamidation of asparagine and glutamine, and acetylation of protein N-terminal residue. Peptide tolerance, MS/MS fragment tolerance, minimum peptide length, and maximum number of missed cleavages were set to 20 ppm, 20 ppm, 7 residues, and 2, respectively. Peptide-to-spectrum matches and proteins were established at a false discovery rate (FDR) below 1% with the option match between runs activated. Collapse of protein isoform replicates was based on the isoform with maximum abundance.

### Proteomics data interpretation

Differentially expressed proteins (DEPs) were identified using a modified version of the R/Bioconductor package for reproducibility-optimized statistical testing (ROTS) (Suomi, T., Seyednasrollah, F., Jaakkola, M.K., Faux, T. & Elo, L.L., Plos Computational Biology 13 (2017)). Specifically, the Maxquant proteomics LFQ data were Log₂ transformed and normalized using variance stabilizing normalization (VSN) (Huber, W., von Heydebreck, A., Sültmann, H., Poustka, A. & Vingron, M., Bioinformatics 18 Suppl 1, S96-104 (2002)). Pairwise comparisons were performed, and a modified T-statistic test was applied to rank the proteins according to the statistical evidence for significant differential expression between the three groups (sham, HI+Veh and BRT_002 treatment) in the ipsilateral and contralateral brain hemispheres of the male and female rats. Proteins were considered to be differentially expressed at p < 0.05. Reproducibility plots and principal component analysis (PCA) were used to assess the quality of data separation between the various groups that were being compared. Heatmaps were generated using unsupervised hierarchical clustering based on Ward linkage and Euclidean distance to assess the degree of proteomic profile separation across the compared groups. Stepwise regression statistical modeling was used to further reduce the marker set and identify the proteins whose abundance was significantly modified by BRT_002 treatment. Protein-protein interaction (PPI) network was constructed using GeneMANIA software with default parameters (Franz, M., et al., Nucleic Acids Res 46, W60-w64 (2018); Szklarczyk, D., et al., Nucleic Acids Res 51, D638-d646 (2023)). Proteins were annotated using Gene Ontology (GO) (Ashburner, M., et al., Nat Genet 25, 25-29 (2000)), the Kyoto Encyclopedia of Genes and Genomes (KEGG) database ((Ashburner, M., et al., Nat Genet 25, 25-29 (2000)), canonical pathways ((Ashburner, M., et al., Nat Genet 25, 25-29 (2000)) from MSigDB, Reactome gene sets (Fabregat, A., et al., Nucleic Acids Res 46, D649-d655 (2018)), and CORUM structural complexes (Ruepp, A., et al., Nucleic Acids Res 38, D497-501 (2010)) at a significance cut-off of p ≤ 0.01. Relevant enriched terms were selected based on the following criteria: enrichment factor ≥ 1.5, at least 3 overlapped proteins, and p ≤ 0.01. Additionally, the enrichment clustering for each comparison was further explored to select only the most abundant proteins and identify their potential functions in HI neonatal rats after BRT_002 treatment.

### Immunohistochemistry

Fixed brain sectioned and mounted on glass slides were heated in an oven at 60°C for 1 h on the first day. Then, the slides were deparaffinized and rehydrated using the following steps: 2 washes each with xylene, 100% EtOH, and 95% EtOH for 5 min and 1 wash with 70% EtOH for 5 min. The slides were then rinsed twice with distilled water for 5 min each. Antigen retrieval was performed using both heat-induced and proteolytic-induced techniques. First, slides were submerged in citrate-based buffer (Vector Laboratories, Burlingame, CA, USA) in a steamer at 95°C for 20 min. The slides were then cooled for 30 min and washed 3 times with Tris-buffered saline (TBS, 10 mM) with Tween-20 (TBS-T) for 5 min each. Proteinase K (#S302080-2, Agilent Dako, Santa Clara, CA, USA) was then applied to the slides for 7 min. The slides were subsequently washed 3 times with TBS-T for 5 min each. The slides were then placed in a humidified chamber and blocked with Superblock T20 blocking buffer (Thermo Fisher Scientific, Waltham, MA, USA) at RT for 2 h. Primary antibody was applied to the appropriate slides and incubated overnight at 4°C. Primary antibodies against the following proteins were used: Agrin (1:200, #NB110-11069, Novus Biologicals, Centennial, CO, USA), Zyxin (1:50, #sc-293448, Santa Cruz, Dallas, TX, USA), Collagen IV (1:200, #R1041B, Origene, Rockville, MD, USA), MAP2 (1:200, #ab32454, Abcam, Cambridge, UK), and GFAP (1:500, #Z0334, Agilent Dako, Santa Clara, CA, USA). The next day, after being washed 3 times with TBS-T for 5 min each, the slides were incubated for 1 h with the following secondary antibodies at RT as appropriate: Alexa Fluor 594 goat anti-mouse antibody (1:1000, #A32742, Thermo Fisher Scientific, Waltham, MA, USA) and Alexa Fluor 488 goat anti-rabbit antibody (1:1000, #A11008, Thermo Fisher Scientific, Waltham, MA, USA). After three 5-minute washes with TBS-T, the slides were incubated with 0.3% Sudan Black B for 20 min to quench autofluorescence. The slides were washed again with TBS-T, incubated with DAPI (H-1200, Vector Laboratories, Burlingame, CA, USA), and mounted.

### mRNA extraction and RT-qPCR

Total RNA was extracted with QIAzol^{®} reagent and purified on RNeasy^{®} Plus Universal Tissue Mini Kit columns (Qiagen, Courtaboeuf, France). Briefly, brain tissue samples were homogenized with 1 mL of QlAzol reagent using a Precellys 24 tissue homogenizer (Bertin technologies), and gDNA was eliminated. After the addition of 180 µL of chloroform, the mixtures were centrifuged at 12,000 × g for 15 min at 4°C. The resulting aqueous phases were mixed with 600 µL of 70% ethanol and loaded onto RNeasy columns. Total RNA was washed and eluted with RNase-free water according to the manufacturer's protocol and stored at -80°C. RNA concentrations were measured spectrophotometrically in a NanoDrop 2000c (Labtech France). cDNA was synthesized from 0.5 µg of total RNA with the RT² HT First Strand kit (Qiagen, Courtaboeuf, France). For qPCR, 2 µL of cDNA was mixed with 6.25 µL of iTaq^{™} Universal SYBR^{®} Green Supermix (Bio-Rad, Marnes-la-Coquette, France) and 0.375 µL of a 10 µM primer mixture (The table below is table 7) and brought to 10 µL with ultrapure DNase-RNase-free distilled water.

**Table 1: qPCR primers**

| | **for** | **rev** |
|---|---|---|
| ***Gfap*** | GCTAATGACTATCGCCGCCAAC (SEQ ID NO: 1) | GCATTTGCCTCTCCAAGGACTC (SEQ ID NO: 2) |
| ***Iba1*** | ATCGTCATCTCCCCACCTAAGG (SEQ ID NO: 3) | TCCCATCCAACCTCTCTTCCTG (SEQ ID NO: 4) |
| ***Map2*** | CGGAAAACCACAGCAACAAGT (SEQ ID NO: 5) | GGTCTTGGGAGGGAAGAACG (SEQ ID NO: 6) |
| ***NeuN*** | AGCACAGACAGATAGCCAGC (SEQ ID NO: 7) | TTTCCCGAATTGCCCGAACA (SEQ ID NO: 8) |
| ***Vegfa*** | CCACTTCTGAGGAGCCTAG (SEQ ID NO: 9) | GGAGGAGGAGCCATTACC (SEQ ID NO: 10) |
| ***Angt1*** | TGCTAACAGGAGGTTGGTGG (SEQ ID NO: 11) | TTTTCCATGGTTTTGCCCCG (SEQ ID NO: 12) |
| ***Angt2*** | GACGGCTGTGATGATCGAGA (SEQ ID NO: 13) | CGTCTGGTTTAGTACTTGGGCT (SEQ ID NO: 14) |
| ***Apcdd1*** | CTGCAGAACGCCAAGAATCAC (SEQ ID NO: 15) | CATTCCCCATGGAGGCCAAT (SEQ ID NO: 16) |
| ***Pacs1*** | CCTGCAGGCATAAGTTCCCT (SEQ ID NO: 17) | GGAGGGCGAGTCTTCAACAA (SEQ ID NO: 18) |
| ***Letm1*** | GGTTGACGAGCCATGAGAGT (SEQ ID NO: 19) | CAGGCCTCTGAGTCCAACAG (SEQ ID NO: 20) |
| ***Vwf*** | CCAGCCACATTCCATACAATC (SEQ ID NO: 21) | CCAATCAACACAGACTCCATTAG (SEQ ID NO: 22) |
| ***Agrin*** | TGCCGTAAGGGATGACTGTG (SEQ ID NO: 23) | CAGGTGTCATGGGATCTGCT (SEQ ID NO: 24) |
| ***SIc17a7*** | ACAGCCTTTTGCGGTTCCTA (SEQ ID NO: 25) | CCCGAAGCTGCCATAGACAT (SEQ ID NO: 26) |
| **Gabbr1** | CTACAATGTCGCGGTCCTGT (SEQ ID NO: 27) | GGCACAAAGAGCACAACCAG (SEQ ID NO: 28) |
| **Txn2** | ATTTGCCTCTGGTGTATTTC (SEQ ID NO: 29) | TTATCCATTACTCTCACTTGTTC (SEQ ID NO: 30) |

qPCR was performed in a CFX96 Real-Time Detection System (Bio-Rad, Marnes-la-Coquette, France) with the following cycling conditions: denaturation at 95°C for 10 min, 40 cycles of 15 sec at 95°C followed by 1 min at 60°C, and a final step of 30 sec at 72°C. Cycle threshold (Ct) values of the target gene and housekeeping gene (hypoxanthine guanine phosphoribosyl transferase, HPRT) were recorded, and gene expression was calculated as 2-ΔCt (where ΔCt= Ct target - Ct HPRT).

### Mitochondrial respiration assessment

### Preparation of Primary Neurons

Animal use was in accordance with local rules and with the regulations and ethical guidelines. Primary cortical neurons were prepared from embryonic day 14-16 pregnant C57BL/6 mice described previously [Thornton, C., Bright, N.J., Sastre, M., Muckett, P.J. & Carling, D. Biochem J 434, 503-512 (2011). Briefly, cortices from embryos in a single litter were dissected, meninges were removed, and tissue was pooled. Cortices were roughly chopped before incubation in 0.25% trypsin/EDTA followed by trituration. Cells were pelleted by centrifugation, resuspended, and plated in neurobasal medium supplemented with B27, 100 units/mL penicillin, 100 µg/mL streptomycin, 0.25 µg/mL amphotericin B, 300 µM glutamine and 25 µM 2-mercaptoethanol. Neurons were prepared and plated at a density of 30-50-70 × 10³ cells/well. Treatments were carried out on neurons grown for a minimum of 7 days *in vitro,* and all media were obtained from Invitrogen.

### Exposure to oxygen-glucose deprivation (OGD)

Primary neurons were cultured for a minimum of 7 days (DIV7) from C57BL/6 mice. Growth medium was replaced with de-gassed, glucose-free, neurobasal-A medium (Invitrogen, Life Technologies Glasgow, UK) and culture plates were maintained at 37°C in a 95% N₂/5% CO₂ environment for the duration of the OGD. Following OGD, the medium was replaced with a standard neurobasal medium (containing additions described above) and cultures returned to 5% CO₂/95% air incubation. For control plates, medium was replaced with standard Neurobasal media at the start of the OGD plates and replaced again with media after 90 min. BRT_002 was added.

### Measurement of Oxygen Consumption Rate (OCR)

Real-time measurements of oxygen consumption rates were performed on an XFe96 Seahorse extracellular flux analyzer (Seahorse Biosciences, North Billerica, MA, USA). The optimal seeding density and test compound concentrations were empirically determined before commencement of experiments. According to the methods described in the XFe96 extracellular flux analyzer user manual (Seahorse Bioscience), preliminary studies were run with Carbonyl cyanide-4-(trifluoromethoxy) phenylhydrazone (FCCP) to identify the optimal number of cells required to observe a sufficient shift in OCR. Once the cell number was decided, we determined the optimal working concentrations for each of the stimulating compounds used in the mitochondrial function analysis (oligomycin, FCCP, and rotenone). Cells were then plated into XFe96 cell culture plates (Seahorse Biosciences, North Billerica, MA, USA) at a density of 50,000/well in 80 µL of DMEM (Sigma-Aldrich, St. Louis, MO, USA). Cells were allowed to adhere overnight in a 37_{°}C incubator with 5% CO₂. The day prior to the experiment, 200 µL of XF calibration media was added to the XF sensor cartridges and kept in a non-CO₂ incubator for 24 h. XF sensor cartridges were loaded with test compounds and OCR measured. OCR was measured by sequential injections of oligomycin (2 µM final concentration, blocks ATP synthase to assess respiration required for ATP turnover), FCCP (carbonyl cyanide 4-trifluoromethoxy-phenylhydrazone, 2 µM final concentration, a proton ionophore uncoupler inducing maximal respiration), and rotenone plus antimycin A (0.5 µM final concentration of each, which completely inhibits electron transport to measure non-mitochondrial respiration). Each step had three cycles; each cycle consisted of 3 min mixing, 2 min incubation and 3 min measurement. All experiments were run in three replicates with 3-4 samples per replicate. Cell counts were used to normalize OCR.

### Duolink proximity ligation assay

The proximity ligation assay was used to detect PP!s using the following key components: the PLA probe anti-rabbit PLUS (DUO92002), the PLA probe anti-mouse MINUS (DUO92004) and the detection reagent ORANGE (DUO92007).

Paraffin-embedded slides were heated in a 60°C oven for 1 h. Then, the slides were deparaffinized and rehydrated using the following steps: xylene twice for 5 min each, 100% EtOH twice for 5 min each, 95% EtOH twice for 3 min each, 70% EtOH once for 3 min and distilled water twice for 3 min each. Antigen retrieval was performed in sodium citrate buffer at 95°C for 20 min. After cooling, the slides were washed 3 times in 10 mM TBS-T for 5 min each. Then, blocking solution (1% bovine serum albumin; 5% normal goat serum) was applied at RT for 2 h. Samples were incubated overnight at 4°C with the following primary antibodies: rabbit anti-synaptotagmin 5 (Biotechne, NBP1-69104) with mouse anti-Thioredoxin-2 (Thermo Scientific PA5-87276), rabbit polyclonal anti-AGRIN Agrin (Thermo Scientific PA5-103585), mouse anti-VEGFA (Protein Tech, 66828-1), rabbit polyclonal anti-Zyxin (Thermo Scientific PA5-68635), mouse monoclonal anti-Zyxin (Santa Cruz Biotechnology sc-293448), mouse monoclonal anti-VWF (Invitrogen MA541692) or rabbit polyclonal anti-Angiopoietin 1 (Thermo Scientific PA1-32150).

On the second day, after three washes with the blocking solution, the slides were incubated at 37°C for 1 h in a preheated humidified chamber with a PLA mixture (the PLA probes PLUS and MINUS diluted 1/5 in the blocking solution and incubated for 20 min prior to application). After two 5-minute washes with wash buffer A [0.01 M Tris, 0.15 M NaCl, and 0.05% Tween 20], the samples were incubated for 30 min at 37°C with a ligation solution consisting of ligation stock and ligase diluted in high-purity water at ratios of 1/5 and 1/40, respectively. Cells were washed again with wash buffer A and incubated for 100 min with a mixture of amplification stock (1/5) and polymerase (1/80) diluted in high-purity water. Samples were then washed twice with 1× wash buffer B [0.2 M Tris and 0.1 M NaCl] for 10 min and once in 0.01× wash buffer B for 1 minute. Finally, DAPI (0.1 µg/ml) was applied for 10 min, and the slides were washed 3 times in TBS-T for 5 min each and mounted in mounting medium (Sigma Aldrich, FluoromountTM aqueous mounting medium).

### Western blotting

Samples containing 20 µg of protein were loaded in each well of a 4-15 % Criterion^{™} TGX Stain-free^{™} 18-well gel (Bio-Rad, #5678084). After 55 min of migration at 500 mA, 50W, and 200V, proteins were transferred to a PVDF membrane using the Trans-Blot Turbo system from Bio-Rad. Then, after one h of blocking in 5% milk, the primary antibodies anti-Agrin (Invitrogen; #PA5-103585), anti-Zyxin (Invitrogen; PA5-68635), , anti-GAPDH (Sigma-Aldrich; G8795) or anti-Tubulin (Sigma Aldrich; T6199) were incubated overnight at 4°C. On the second day, the membranes were incubated with HRP-conjugated secondary antibody (Invitrogen #31466 or #31431) for 1 h at RT, and the protein bands were visualized using Immobilon Crescendo or Forte Western HRP substrate (Millipore, #WBLUR0100 or #WBLUF0100) on the ChemiDoc system from Bio-Rad. Quantification was performed with ImageLab Studio software (Bio-Rad, version 6.1.0).

### Statistical analysis

Statistical analyses were conducted with GraphPad Prism (GraphPad Prism version 9.4.0). First, the distributions of all experimental data were tested for normality test. If the data were normally distributed, the sham, HI+Veh and BRT_002-treated groups were compared by one-way analysis of variance (ANOVA) or two-way ANOVA with sex and treatment as factors, followed by Bonferroni correction as a post hoc test (p < 0.05) to identify intergroup differences. If the data were not normally distributed, Kruskal-Wallis ANOVA and the median test were applied. Protein abundance was compared between two groups using the Wilcoxon test. A p value < 0.05 was considered to indicate statistical significance in all statistical tests.

### Example 2: Results

### Synthesis of valyl ester BRT_002

The compounds used in the experiments are tri-substituted purine derivatives. Several tri-substituted purines and some structurally related heterocycles are kinase inhibitors. These compounds bind to the kinase catalytic site by an hydrogen bond. Compounds BRT_001 and BRT_002 that lack hydrogen on the N6 are unable to bind the kinases and therefore devoid of inhibition (Delehouzé, C., et al., Oncogene 33, 5675-5687 (2014)). In the present study, the valyl ester, BRT_002 was preferred to its precursor BRT_001.

BRT_001 (compound 4) and its valyl ester, BRT_002, were obtained as follows.

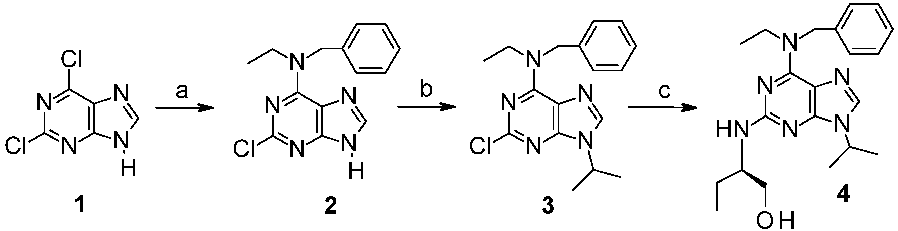

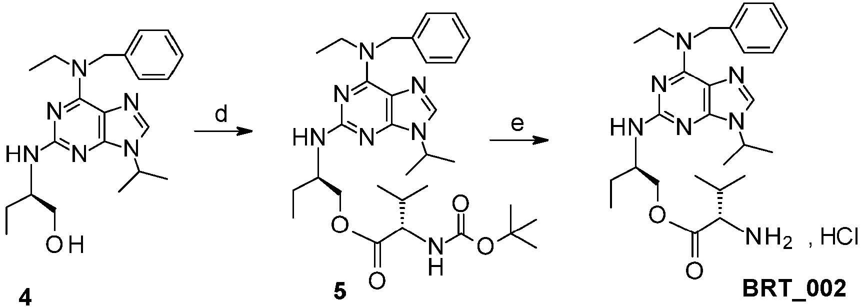

. Trisubstituted purine derivative 4 was prepared from 2,6-dichloropurine in three steps. N-Ethylbenzylamine was heated with 2,6-dichloropurine and triethylamine in isopropanol to obtain 2. In the second step, selective alkylation of 2 occurred at position 9 of the purine to yield derivative 3, which was heated with R-aminobutanol without added solvent to obtain 4. Boc-valine was activated by a mixture of 1-hydroxybenzotriazole (HOBt) and dicyclohexylcarbodiimide (DCC) in the next step. The active ester was reacted with 4 to yield 5. Deprotection of 5 with HCl in Et2O afforded BRT_002, which was isolated as a hydrochloride.

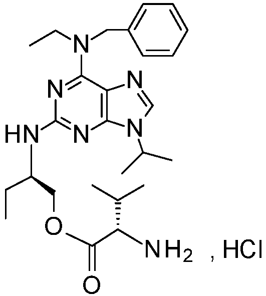

Reagents and conditions. (a) N-Ethylbenzylamine, NEt₃, isoPrOH, 3 h, 85 °C; (b) K₂CO₃, 2-bromopropane, DMSO, 24 h, 20 °C; (c) (R)-2-aminobutan-1-ol, 160 °C, 6 h; (d) Boc-valine, DCC, HOBt, AcOEt, THF, 3 h; (e) HCl in Et₂O, 20 °C.

### N-Benzyl-2-chloro-N-ethyl-9H-purin-6-amine (2)

2,6-Dichloropurine, 1 (9.6 g, 41.5 mmol), was added to a solution of N-ethylbenzylamine (8.4 mL, 62.2 mmol) and NEt3 (8.4 mL, 62.2 mmol) in i-PrOH (150 mL) while stirring. The mixture was heated for 3 h at 85 °C. After cooling, the crystallized solid was filtered and washed twice (10 mL, 10 °C). After drying, compound 2 was found to be pure in a vacuum by TLC (cyclohexane-CH2Cl2, 6:4; Rf 0.8 compared to 1 Rf 0.25). Yield 66%. 1H-NMR (400 MHz, DMSO-d6, δ ppm): 1.17 (brs, 3H), 3.58 and 4.16 (2 brs, 2H), 4.93 and 4.58 (2 brs, 2H), 7.29 (brs, 5H), 8.15 (s, 1H).

### N-Benzyl-2-chloro-N-ethyl-9-isopropyl-purin-6-amine (3)

A solution of 2 (4.98, 49.7 mmol) in DMSO (75 mL) was cooled at 15 °C. K2CO3 (27.5 g, 19.9 mmol) was added under stirring. After 5 min at 15 °C, 2-bromopropane (23.2 mL, 24.8 mmol) was introduced slowly. The mixture was stirred and maintained at 20 °C overnight. The solution was cooled to 10 °C, and 200 mL of cold water was added. The mixture was extracted with AcOEt (3x20 mL). The organic layer was washed with brine (2x50 mL) and H2O (25 mL). The solution was dried over Na2SO4 and concentrated in vacuo to yield 3 as a white solid, which was triturated with 3 mL of AcOEt and isolated by filtration on a Büchner funnel. Yield 82%. 1H-NMR (400 MHz, DMSO-d6, δ ppm): 1.18 (brs, 3H), 1.51 (d, J =6.5 Hz, 6H), 3.58 and 4.15 (2 brs, 2H), 4.70 (hept, J =6.5 Hz, 1H), 4.91 and 5.55 (2 brs, 2H), 7.32 (m, 5H), 8.30 (s, 1H).

### (2R)-2-[[6-[Benzyl(ethyl)amino]-9-isopropyl-purin-2-yl]amino]butan-1-ol (4)

A mixture of 3 (5.72, 17.3 mmol) and (2R)-aminobutanol (16.3 mL, 17.3 mmol) was heated at 160 °C for 6 h. The reaction was monitored by TLC. After completion, the mixture was cooled and partitioned with a mixture of H2O (20 mL) and AcOEt (3x30 mL). The organic layer was washed with brine (2x20 mL) and H2O (20 mL). After drying over Na2SO4, the solution was evaporated under a vacuum. The solid was triturated with AcOEt (3 mL). The solid was filtered with a Büchner filter and dried under a vacuum. Yield 75%. 1H-NMR (400 MHz, DMSO-d6, δ ppm): 0.78 (t, 3H, J = 6.5 Hz), 0.78 (brs, 3H), 1.06 (t, 3H, J = 6.5 Hz), 1.39 (d, 6H, J = 6 Hz), 1.53 (m, 2H), 3.41 (m, 1H), 3.71 (m, 1H), 5.15 (brs, 2H), 5.78 (brd 1H), 7.22 (brs, 5H), 7.74 (s, 1H).

### [(2R)-2-[[6-[Benzyl(ethyl)amino]-9-isopropyl-purin-2-yl]amino]butyl] (2S)-2-(tert-butoxycarbonylamino)-3-methyl-butanoate (5)

Dicyclohexylcarbodiimide (2.44 g, 12 mmol) was added at 5 °C to a solution of tert-butyloxycarbonylvaline, Boc-valine (2.60 g, 12 mmol), and 1-hydroxybenzotriazole (HOBt) (1.62 g, 12 mmol) in 60 mL of AcOEt. The cooling bath was removed after 5 min, and stirring at 20 °C continued for 3 h. The suspension was filtered through a Büchner filter, and the precipitate (DCU) was washed with AcOEt (2x5 mL). The combined filtrates were added to a mixture of compound 4 (3.8 g, 10 mmol) and NEt3 (6 mL, 20 mmol) in THF (50 mL). Stirring was continued at 20 °C for 2 days. The mixture was transferred to a separatory funnel and washed with 25 mL of 1 M Na2CO3, followed by 50 mL of brine and 50 mL of H2O. The organic layer was dried over Na₂SO₄ and concentrated under a vacuum below 40 °C. The ester crystallized and then was triturated with 5 mL of AcOEt and isolated by filtration on a Büchner filter. Yield: 65%. 1H-NMR (400 MHz, DMSO-d6, δ ppm): 0.75 (3H, t, J =6.5 Hz), 1.12 (m, 3H), 1.38 (d, 6H), 1.54 (m, 17H), 1.95 (m, 1H), 3.65 (m, 1H), 4.05 (m, 4H), 4.55 (1H, Hept, J = 6.5 Hz), 5.05 and 5.45 (2 brs, 2H), 6.25 (brs, 1H), 7.25 (m, 5H), 7.80 (s, 1H).

### [(2R)-2-[[6-[Benzyl(ethyl)amino]-9-isopropyl-purin-2-yl]amino]butyl](2S)-2-amino-3-methyl-butanoate hydrochloride or the L-valyl ester of (2R)-2-[[6-[benzyl(ethyl)amino]-9-isopropyl-purin-2-yl]amino]butan-1-ol hydrochloride (BRT 002)

Ester 5 (1.45 g, 2.5 mmol) was dissolved in 50 mL of anhydrous EtzO. A 2 M HCl solution in Et2O (20 mL) was added and stirred at 20 °C for 24 h. The solvent was decanted, and the solid was triturated with Et₂O (3x20 mL). The precipitate was then dried under a vacuum (P₂O₅) for 48 h to yield BRT_002. Yield: 43%. 1H-NMR (400 MHz, DMSO-d6, δ ppm): 0.75 (m, 3H), 1.15 (m, 3H), 1.45 (m, 6H), 1.64 (m, 8H), 2.35 (m, 1H), 3.75 (m, 1H), 4.12 (m, 4H), 4.55 (m, 1H), 4.95 and 5.45 (2 brs, 2H), 6.25 (brs, 1H), 7.25 (m, 5H), 8.20 (brs, 1H), 8.45 (m, 3H).

### A repeated high dose of a novel non-substituted purine derivative, BRT_002, is nontoxic and well tolerated in WT rats

Firstly, dose range for subsequent repeated BRT_002 dose toxicity was analyzed in a non GLP-study. Adult male Wistar rats were administered BRT_002 orally at either 0.3, 3, 30 or 100 mg/kg (3 animals per dose). Even at 100 mg/kg, no unexpected mortality occurred, and no clinical signs were observed. At necropsy, no remarkable macroscopic findings related to the organs were observed. Therefore, a dose of 100 mg/kg was selected for the 7-day fixed-dose study.

Seven days fixed dose study showed no obvious change in body weight related to the administration of BRT_002. Compared to the controls, BRT_002-treated animals showed no significant changes in hematology and coagulation parameters (p > 0.05). Among the other clinical parameters, cholesterol showed a non-significant decrease in BRT_002-treated animals compared to controls (p > 0.05). After sacrifice, the organs were recovered and weighed. The administration of 100 mg/kg BRT_002 every day for 7 days did not modify the weight of the brain, spleen, liver, kidneys or heart. Subsequently, the amount of BRT_001 in each organ was quantified by LC-MS/MS to study its bioaccumulation. No accumulation of BRT_001 was observed in any organ of animals in the treated group. Taken together, these results indicate that the administration of BRT_002 up to 100 mg/kg was not associated with any mortality or changes in clinical parameters or food consumption and did not accumulate, demonstrating its safety.

### Ability of BRT_002 to penetrate the brain and serum

PK studies were carried out to evaluate effective delivery of BRT_002 in vivo when administered via IP injection into sham male and female neonatal rats in which moderate HI was induced. Blood, and brain tissue (ipsilateral and contralateral hemispheres) samples were collected at 1, 2, 6 and 24 h after the administration of BRT_002 (30 mg/kg). BRT_002 and BRT_001, which is formed rapidly from the cleavage of BRT_002, were quantified by LC-MS/MS.

In both hemispheres of the male and female animals in the sham group, the concentration of BRT_001 peaked at 1 h post-injection and then slowly decreased to 0 at 24 h post-injection. BRT_001 concentration was comparable in sham group males [(ipsilateral brain hemisphere (667.6 ng/ml); (contralateral hemisphere: 609.2 ng/ml,)] and sham group females (ipsilateral hemisphere:591.3 ng/ml; contralateral hemisphere: 613 ng/ml).

In the HI group, the BRT_001 concentration peaked 1 h after injection was the same in male about 553 ng/ml in ipsilateral hemisphere and 926.8 ng/ml in contralateral hemisphere. In the female neonatal rats BRT_001 concentration was about 463.9 ng/ml in the ipsilateral hemisphere and 637.2 ng/ml in contralateral hemisphere. The same pattern was observed in the serum with specifically, BRT_001 concentration in female HI group peaked at 2 h. The partition coefficient (the ratio between drug concentration in the brain and in the blood; Kp) was about 1 in males and females in HI groups and in both brain hemispheres while in sham groups was between 0.6. and 0.7 in males and females respectively.

These data reflect the ability of BRT_001 to penetrate the brain in both sham and HI groups. Since BRT_002 is cleaved in BRT_001 in plasma, overall, these results indicated the penetration of BRT_001 into the brain, with maximum exposure in the brain and serum occurring 1 h after IP BRT_002 administration.

### BRT_002 achieves therapeutic efficacy in both male and female neonatal rats after moderate HI

The pharmacodynamic effect of BRT_002 was determined on both male and female neonatal rats after moderate HI. Pups were distributed into 3 groups: 1) sham control, 2) moderate HI + placebo (HI+Veh), and 3) moderate HI+BRT_002 (30 mg/kg).BRT_002 was administered immediately after carotid ligation and 90 min of hypoxia and administered two additional doses at 24 h and 48 h after HI. The animals were sacrificed 72 h after HI at P10, and the brains were collected for analysis. The brains were stained with cresyl violet, which binds Nissl bodies in the rough endoplasmic reticulum of neurons ³⁴ and thus can be used to visualize neurons; a decrease in the intensity of Nissl body staining indicates neuronal loss or degeneration. Administration of BRT_002 significantly reduced brain infarct volumes in both male and female neonatal rats. Visual examination of the coronal images revealed noticeable differences between the groups. Specifically, among both male and female neonatal rats, the HI+ Veh group exhibited decreased staining in the ipsilateral brain hemisphere compared to that in the sham group. These observations suggest that treatment with BRT_002 mitigates the reduction in staining associated with HI brain injury in male and female neonatal rats.Conversely, for both male and female neonatal rats, the HI+BRT_002-treated group showed increased staining compared to that in the HI+Veh group. Collectively, we prove that BRT_002 has a protective effect, preserving the integrity of brain tissue and reducing neuronal damage following moderate HI insult.

Quantitative analysis of the percentage infarct volume was conducted to validate the visual observations of cresyl violet-stained brain sections from neonatal rats exposed to moderate HI injury. Among the female rats, the ipsilateral infarct volume was 50.26 ± 4.91% in the HI+Veh group) and treatment with BRT_002 reduced the infarct volume to 32.55% **(****Fig. 1A****,** p < 0.01). In males, the infarct volume was significantly greater in the HI+Veh group than in the treatment with BRT_002 treated group (51.67 ± 4.95%vs 32.55 ± 3.64 (Fig. **1B****,** p=0.1322). The ipsilateral hemispheric infarct volumes in both male and female neonatal rats were significantly larger in the HI+Veh group (50.96 ± 3.42%) than in treated BRT_002 group (35.3 ± 3.08%; **Fig. 1C****,**p < 0.001). Sex had no major effect, and no treatment-related sex interactions were observed **(****Fig. 1A-C,** Tukey's HSD, all p > 0.05).

These results in both male and female neonatal rats suggest that treatment with BRT_002 immediately following moderate HI exposure has the potential to alleviate neuronal damage and preserve the integrity of brain tissue.

### Proteomics revealed that BRT_002 treatment results in unique protein profile in the ipsilateral and contralateral regions of the brain in male and female neonatal rats under moderate HI

Next, we explored the proteomic profiles of the different groups to decipher the molecular mechanisms underlying the preservation of brain tissue integrity and the reduction in neuronal damage by BRT_002 following moderate HI insult.

To conduct an in-depth exploration of the protein profiles in the ipsilateral and contralateral brain regions in BRT_002-treated male and female neonatal rats after moderate HI, we analyzed by label-free shotgun proteomics 60 biological samples with at least 3-6 replicates each in the sham, HI+Veh and HI+BRT_002 groups. The distribution of biological replicates among these three groups is presented in **Table 2.**

**Table 2: Distribution of the numbers of biological replicates in the sham, HI+Veh and HI+BRT_002 groups**

| **#** | **Group** | **Label-status** | **Gender** | **Brain Hemisphere** | **Number of replicates** |
|---|---|---|---|---|---|
| **1** | Control (Not Treated) | Sham | Female | Contralateral | 6 |
| | | | | Ipsilateral | 6 |
| | | | Male | Contralateral | 3 |
| | | | | Ispsilateral | 3 |
| **2** | Control (Placebo Treated) | Hypoxia ischemia + Veh | Female | Contralateral | 6 |
| | | | | Ipsilateral | 6 |
| | | | Male | Contralateral | 6 |
| | | | | ipsiliaterl | 6 |
| **3** | BRT_002- Treated | Hypoxia ischemia + BRT_002 | Female | Contralateral | 5 |
| | | | | Ipsilateral | 5 |
| | | | Male | Contralateral | 4 |
| | | | | Ipsilateral | 4 |

A total of 3724 proteins were identified and their abundance monitored among the 60 biological samples. Given a possible difference in the pharmacodynamic and therapeutic effects of BRT_002 between males and females, differential protein expression analysis was done for each comparison of ipsilateral and contralateral brain hemispheres from neonatal rats in the HI+BRT_002 and HI+Veh groups 72 h after Hl,with and without separation by sex. Significantly up- and downregulated proteins (p < 0.05) between the studied groups are shown in **Table 3** for the ten resulting comparisons.

**Table 3: List of dysregulated proteins common to the ipsilateral and contralateral brain hemispheres of BRT_002-treated female neonatal rats with moderate HI**

| **#** | **Protein ID** | **Protein name** |
|---|---|---|
| 1 | Ap2b1 | Adaptor related protein complex 2 subunit beta 1 |
| 2 | Ap2m1 | Adaptor related protein complex 2 subunit mu 1 |
| 3 | Bzw1 | Basic leucine zipper and W2 domains 1 |
| 4 | Copb1 | COPI coat complex subunit beta 1 |
| 5 | Copb2 | COPI coat complex subunit beta 2 |
| 6 | Copg1 | COPI coat complex subunit gamma 1 |
| 7 | Coro7 | Coronin 7 |
| 8 | Dpp8 | Dipeptidylpeptidase 8 |
| 9 | Dynll2 | Dynein light chain LC8-type 2 |
| 10 | Eef2 | Eukaryotic translation elongation factor 2 |
| 11 | Eif3b | Eukaryotic translation initiation factor 3, subunit B |
| 12 | Ide | Insulin degrading enzyme |
| 13 | Ipo5 | Importin 5 |
| 14 | Ipo7 | Importin 7 |
| 15 | Lonp1 | Ion peptidase 1, mitochondrial |
| 16 | Macf1 | Microtubule-actin crosslinking factor 1 |
| 17 | Mms19 | MMS19 homolog, cytosolic iron-sulfur assembly component |
| 18 | Pfdn2 | Prefoldin subunit 2 |
| 19 | Psmd1 | Proteasome 26S subunit, non-ATPase 1 |
| 20 | Psmd2 | Proteasome 26S subunit ubiquitin receptor, non-ATPase 2 |
| 21 | Rab12 | RAB12, member RAS oncogene family |
| 22 | Rpl26 | Ribosomal protein L26 |
| 23 | Sephs1 | Selenophosphate synthetase 1 |
| 24 | Sf3b3 | Splicing factor 3b, subunit 3 |
| 25 | Snd1 | Staphylococcal nuclease and tudor domain containing 1 |
| 26 | Snrpa | Small nuclear ribonucleoprotein polypeptide A |
| 27 | Strip1 | Striatin interacting protein 1 |
| 28 | Thop1 | Thimet oligopeptidase 1 |
| 29 | Uba1 | Ubiquitin-like modifier activating enzyme 1 |
| 30 | Ube2i | Ubiquitin-conjugating enzyme E2I |
| 31 | Ube4a | Ubiquitination factor E4A |
| 32 | Usp5 | Ubiquitin specific peptidase 5 |
| 33 | Vapa | VAMP associated protein A |
| 34 | Xpo7 | Exportin 7 |

Principal component analysis (PCA) assessed favorably the degree and quality of data separation between different comparisons of the sham, HI+Vehicle and HI+BRT_002 groups of neonatal rats. As expected, all comparisons of DEPs in the ipsilateral vs. contralateral brain hemispheres of animals in the HI-BRT_002 and HI+Veh groups with and without separation by sex identified unique abundant proteins in each specific group, further suggesting that BRT_002 has distinct effects in males vs. females and the ipsilateral vs. contralateral brain hemisphere.

### Proteomics highlighted agrin and zyxin in both the ipsilateral and contralateral brains of neonatal rats under moderate HI and BRT_002 treatment

Comparison of DEPs in the ipsilateral and contralateral brain hemispheres of HI+BRT_002 and HI+Veh males and females identified 34 proteins common to both brain hemispheres from female BRT_002-treated neonatal rats. These proteins were found to be mainly involved in the adaptative immune response, Golgi vesicle transport, nucleoplasmic transport, the PCP/CE pathway, and peptide metabolic processes **(Table 4).**

**Table 4: List of dysregulated proteins common to the ipsilateral and contralateral brain hemispheres of BRT_002-treated female neonatal rats with moderate HI.**

| **#** | **Protein ID** | **Protein name** |
|---|---|---|
| 1 | Ap2b1 | Adaptor related protein complex 2 subunit beta 1 |
| 2 | Ap2m1 | Adaptor related protein complex 2 subunit mu 1 |
| 3 | Bzw1 | Basic leucine zipper and W2 domains 1 |
| 4 | Copb1 | COPI coat complex subunit beta 1 |
| 5 | Copb2 | COPI coat complex subunit beta 2 |
| 6 | Copg1 | COPI coat complex subunit gamma 1 |
| 7 | Coro7 | Coronin 7 |
| 8 | Dpp8 | Dipeptidylpeptidase 8 |
| 9 | Dynll2 | Dynein light chain LC8-type 2 |
| 10 | Eef2 | Eukaryotic translation elongation factor 2 |
| 11 | Eif3b | Eukaryotic translation initiation factor 3, subunit B |
| 12 | Ide | Insulin degrading enzyme |
| 13 | Ipo5 | Importin 5 |
| 14 | Ipo7 | Importin 7 |
| 15 | Lonp1 | Ion peptidase 1, mitochondrial |
| 16 | Macf1 | Microtubule-actin crosslinking factor 1 |
| 17 | Mms19 | MMS19 homolog, cytosolic iron-sulfur assembly component |
| 18 | Pfdn2 | Prefoldin subunit 2 |
| 19 | Psmd1 | Proteasome 26S subunit, non-ATPase 1 |
| 20 | Psmd2 | Proteasome 26S subunit ubiquitin receptor, non-ATPase 2 |
| 21 | Rab12 | RAB12, member RAS oncogene family |
| 22 | Rp126 | Ribosomal protein L26 |
| 23 | Sephs1 | Selenophosphate synthetase 1 |
| 24 | Sf3b3 | Splicing factor 3b, subunit 3 |
| 25 | Snd1 | Staphylococcal nuclease and tudor domain containing 1 |
| 26 | Snrpa | Small nuclear ribonucleoprotein polypeptide A |
| 27 | Strip1 | Striatin interacting protein 1 |
| 28 | Thop1 | Thimet oligopeptidase 1 |
| 29 | Uba1 | Ubiquitin-like modifier activating enzyme 1 |
| 30 | Ube2i | Ubiquitin-conjugating enzyme E2I |
| 31 | Ube4a | Ubiquitination factor E4A |
| 32 | Usp5 | Ubiquitin specific peptidase 5 |
| 33 | Vapa | VAMP associated protein A |
| 34 | Xpo7 | Exportin 7 |

Twelve proteins common to both brain hemispheres were identified. They are mainly involved in the regulation of protein transport and organization of the cytoskeleton **(Table 5).**

**Table 5: List of dysregulated proteins common to the ipsilateral and contralateral brain hemispheres of BRT_002-treated male neonatal rats with moderate HI.**

| # | **Protein ID** | **Protein name** |
|---|---|---|
| 1 | Atp5ifl | ATP synthase inhibitory factor subunit 1 |
| 2 | Bsg | Basigin (Ok blood group) |
| 3 | Cadm2 | Cell adhesion molecule 2 |
| 4 | Coro1b | Coronin 1B |
| 5 | Cyp51 | Cytochrome P450, family 51 |
| 6 | Dynlt1 | Dynein light chain Tctex-type 1 |
| 7 | Eml2 | EMAP like 2 |
| 8 | Epb4112 | Erythrocyte membrane protein band 4. I-like 2 |
| 9 | Fv1 | Friend virus susceptibility 1 |
| 10 | Pals2 | Protein associated with LIN7 2, MAGUK p55 family member |
| 11 | Septin8 | Septin 8 |
| 12 | Sms | Spermine synthase |

Integration of the comparative protein profiles of both hemispheres of male and female BRT_002-treated neonatal rats with moderate HI identified only two common proteins, namely Agrin and Zyxin (Fig. 2A,B). The former protein plays major roles in several important pathways and has more functions than the latter. Interestingly, both proteins are involved in overlapping pathways and pivotal for the regulation of complex components that link the extracellular matrix and the cytoskeleton in brain injury.

Zyxin has been reported to export VWF. VEGFA is involved in VWF exocytosis for brain tissue repair. The literature has reported that when vascular brain injury occurs, VWF is secreted locally following cellular activation for brain repair. Therefore, we performed RT-qPCR for VWF transcripts on samples from the ipsilateral brain hemisphere of BRT_002-treated female and male neonatal rats after moderate HI. At 72 h after injury, BRT_002 treatment of HI rats strongly increased VWF mRNA expression VWF **(Fig. 3a)** among female rats (p<0.0001), male rats (p<0.01), and in both male and female neonatal rats (p<0.0001). qRT-PCR also revealed a significant increase in VEGFA gene expression in brain endothelial cells **(Fig. 3b)** in the HI-+BRT_002 group compared to the Hl+-Veh group for females (p<0.01), males (Fig. 5i, p<0.001), and all animals (p<0.0001). The literature has also reported other factors, such as angiopoietins, cross talk with VEGFA, modulating its effects. Angiopoietin-1 (Ang1) and Angiopoietin-2 (Ang2) compete for binding to Tie-2 and, while Ang1 promotes BBB stabilization 36. Therefore, the mRNA expression of Ang1 and Ang2 was figured out. The mRNA expression of Ang1 **(Fig. 3c)** in the ipsilateral hemisphere was significantly increased in the HI-+BRT_002 groups compared to the Hl-+Veh groups of females (p< 0.05) and males (p < 0.01) neonatal rats as well as in both female and male neonatal rats (p < 0.00001). In addition, the expression of Ang2 **(Fig. 3d)** in the ipsilateral hemisphere was also significantly increased in the HI-+BRT_002 groups compared to the Hl-+Veh groups of female (p< 0.0001) and male (p < 0.001) neonatal rats as well as in both female and male neonatal rats (p < 0.0001). The role of Ang1 in the stabilization of BBB is emphasized with the high mRNA expression of Apccd1-CNS endothelial cells in the HI-+BRT_002 groups compared to the HI-+Veh groups of females (p< 0.001) and males (p < 0.001) neonatal rats (Fig. 3e). These findings suggest that BRT_002 affect Zyxin expression and probably crosstalk with VWF, VEGF, Ang1, Ang 2 and Apcdd1 promoting vascular stabilization.

### BRT_002 interacts with mitochondrial targets, modulates the expression of neurotransmitters and calcium sensors, and prevents neuronal death

In-depth interpretation of the proteome profiles revealed distinct signatures for proteins involved in the modulation of neurotransmitters and calcium sensor activity across the HI+BRT_ 002 and HI+Veh groups of neonatal rats. The most striking finding is that 15 DEPs were significantly upregulated by BRT_002 treatment in neonatal rats with moderate HI compared to the HI+Veh group. To further understand the effect of the BRT_002 treatment in neonatal rats with moderate HI, the abundance of DEPs were further explored in the HI+Veh group compared to the sham group showing that Adgrl1, Atp2a2, Erc2, Letm1, Ncald, Ncs1, Sestd1, and Syt2 were overrepresented after the BRT_002 treatment in neonatal rats with moderate HI while Cdk5, Hint1, Marcksl1, Nipsnap2, Pacs2, Syt5, and Trpv2 were induced by the BRT_002 treatment in neonatal rats with moderate HI. These DEPs are neurotransmitters or calcium sensors that are associated with the regulation of mitochondrial membranes (e.g., Phosphofurin Acidic Cluster Sorting Protein 2, Pacs2), developmental and cellular mitochondrial respiration (e.g., Leucine Zipper And EF-Hand Containing Transmembrane Protein 1, Letm1), dendrite arborescence (e.g., SEC14 and Spectrin Domain Containing 1, Sestd1), synaptic transmission (e.g., Synaptotagmin 5, Syt5; Synaptotagmin 2, Syt2; SEC14 And Spectrin Domain Containing 1, Sestd1; and Neuronal Calcium Sensor 1, Ncs1), and the regulation of neuroinflammation (e.g., Neurocalcin Delta, Ncald). Functional enrichment analysis revealed that the major pathways related to the neurotransmitters and calcium sensors whose production was induced by BRT_002 treatment under HI are involved in the regulation of calcium ion transport, regulation of the presynaptic cytosolic calcium ion concentration and intracellular calcium ion homeostasis **(****Fig. 3****).** Notably, Syt5 was the only commonly upregulated protein in the HI+BRT_ 002 and HI+Veh groups when both male and female animals and the ipsilateral and contralateral brain hemispheres were compared, suggesting a potential role for this protein in the regulation of neuroprotection and neuronal activity mediated by BRT_002.

To identify important protein networks that mediate the ability of BRT_002 to regulate neuroprotection and neuronal activity, a PPI network was built reflecting interactions between the neurotransmitters and calcium sensors whose production was induced by BRT_ 002, including Cdk5, Hint1, Marcksl1, Nipsnap2, Pacs2, Syt5, and Trpv2. Interestingly, Syt5, Cdk5, Hint1, Marcksl1, Nipsnap2 and Pacs2 all interact with multiple markers, suggesting the possibility of interplay between these proteins through interaction with predicted markers from a similar family. The Syt5 subnetwork showed close interactions with key markers involved in mitochondrial/respiratory function, such as Pacs1, thioredoxin 2 (Txn2), and Rab1a, suggesting that Syt5 might be a protein driver of the neuroprotective regulation of mitochondrial function. Txn2 is a key mitochondrial protein involved in the regulation of oxidative stress. To further investigate the potential interaction between Txn2 and Syt5, we performed Duolink proximity ligation assay (PLA) which detect protein-protein interactions or protein modifications. The results obtained suggested the *in situ* interaction between Syt5 and Txn2.

Moreover, additional qPCR experiments revealed increased expression of 4 genes encoding proteins involved in mitochondrial functions, namely, LETM1 **(****Fig. 5a****),** PACS1 **(****Fig. 5b****),** PACS2 **(****Fig. 5c****),** and TXN2 **(****Fig. 5d****),** in BRT_002-treated female and male neonatal rats exposed to moderate HI. Collectively, these findings provide evidence that changes in key components of mitochondrial function are associated with the positive regulation of Syt5 by BRT_002, which could impact neuronal activity and neuroprotection, ultimately indicating that BRT_002 plays a role in modulating proteins involved in the regulation of mitochondrial functions.

Oxygen Consumption rate (OCR) in mice primary neurons exposed to OGD and BRT_002 showed that basal OCR and ATP-linked OCR was significantly increased in neurons following exposure to 10 µM of BRT_002 for 24 h compared with controls **(****Fig. 5e** and **f).** FCCP-induced maximal OCR and spare respiratory capacity (SRC) decreased, whereas leak-driven OCR significantly increased with exposure to 10 µM of BRT_002. These results point out the major role of BRT_002 in the regulation of mitochondrial neuronal activity and neuroprotection.

These observations are in line with the data that HI significantly reduced the number of neuronal cells, BRT_002 significantly increased the expression of MAP2 **(****Fig. 6a****),** Neun **(****Fig. 6b****),** SLC17a7 **(****Fig. 6c****)** and the GABAergic marker GABRR1 **(****Fig. 6d****),** indicating that it prevents neuronal death in the ipsilateral brain hemisphere in both female and male neonatal rats.

### Filamin Binding LIM Protein 1: a link between Syt5, Agrin and Zyxin

To further demonstrate potential interactions between the three identified key players in the effects of BRT_002, Syt5, Agrn and Zyx, we performed in situ PLA using specific antibodies against each protein. We revealed that, at the endogenous protein level, Syt5 interacts with Agrin and Zyxin (at distances <40 nm). These results are in agreement with the involvement of Agrin and Zyxin in overlapping pathways and their potential participation in brain repair tissue and/or attenuation of the brain injury in HI.

Moreover, in silico PPI network shows prominent indirect coexpression interactions of Syt5, Agrin and Zyxin involving mainly Filamin Binding LIM Protein 1 (Fblim1), which was thus identified to be a key intermediate player in these interactions, suggesting that these proteins are functionally related or controlled by the same transcriptional regulatory system.

Since Zyxin, Agrin and Syt5 are coexpressed and Zyxin has been reported to export VWF, and VEGFA is involved in the exocytosis of VWF for brain tissue repair, we assessed the possibility of an in situ interaction between Syt5 and VEGFA, as well as VWF and Ang1, using Duolink PLA. Unexpectedly, PLA demonstrated an *in situ* interaction between Syt5, and VWF, VEGFA and Ang1 (immunofluorescence signal, red PLA dots) upon immunostaining for different proteins using specific antibodies. No PLA dots were observed when cells were stained with either the VEGFA, VWF and Ang1antibodies alone or Syt5 antibody alone. Taken together, these findings suggest that through Fblim1, Syt5 interacts with major players in the brain repair tissue and/or attenuation of the brain injury processes and neuroprotection (Agrin, Zyxin, VEGFA, VWF and mitochondria targets).

### BRT_002 prevents neuroinflammation in neonatal rats after moderate HI

Furthermore, focusing on the proteins specific to the ipsilateral and contralateral brain hemispheres of BRT_002-treated neonatal rats after moderate HI showed a unique and abundant protein, Serpina3n, in the contralateral brain hemisphere in both sex types compared to the other hemisphere (Fig. 7a). This suggests the crucial role of Serpina3n in protection of the contralateral brain hemisphere from HI insult. Serpina3n is an inhibitor of a variety of serine proteases and may also exert a neuroprotective effect 37.

As SerpinA3 is a potential marker of reactive astrogliosis, which repairs initial damage after CNS injury 37, we next focused on astrocyte/microglia activation after HI and BRT_002 treatment. Gfap gene expression was quantified after HI to monitor astroglios **(****Fig. 7a****).** At 72 h after injury, the ipsilateral expression of GFAP showed a nominal decrease in HI+-BRT_002-treated females compared to Hl+-Veh females and a significant decrease in male neonatal rats (p < 0.05). Since microglia are the first cells to become activated after HI insult 38, 39 and migrate to the damaged region 40, we evaluated the effect of treatment with BRT_002 on microglial activation based on Iba-1 expression. Gene expression was quantified at 24 h **(****Fig. 7b****)** after HI. At 24 h after HI, Iba-1 expression was significantly increased in the Hl+-Veh group (p < 0.0001) and albeit to a lesser degree in the HI+BRT_002 group (p = 0.0108) compared to the sham group when both female and male neonatal rats were considered. These results suggest that BRT_002 prevents astrocyte/microglia activation in neonatal rats after moderate HI.

### Example 3: Discussion

A major limitation of previous unsuccessful therapeutic approaches for hypoxic-ischemic (H)I brain injury is their focus on either the neuronal compartment or the blood-brain barrier (BBB) without accounting for the dynamic interactions between these biological systems. Several pieces of evidence suggest that changes in cortical blood flow and activity during HI are related to fluctuations in breathing and blood oxygenation, which predominantly affect central nervous system (CNS) endothelial cells. Importantly, this suggests that dysfunction of CNS endothelial cells impairs neuronal activity. The new, driving hypothesis of our study is that deleterious crosstalk between neurons and CNS endothelial cells of the BBB in HI is associated with worse functional outcomes and could be targeted by a novel tri-substituted purine derivative, BRT_002, in neonatal HI to improve neuronal plasticity. This is the first study exploring an innovative therapeutic strategy to target two biological systems (the BBB and neuronal compartment) for the treatment of term and preterm infants with HI.

Our study provides strong *in vivo* evidence that highlights the potential of a novel substituted purine derivative for the treatment of brain ischemic disorders such as neonatal HI, for which there is currently no available treatment. We examined alterations in the expression of proteins that are potentially connected to HI pathology. Next-generation proteomics suggests that BRT_002 treatment results in specific effects in males and females and in the ipsilateral and contralateral brain hemispheres. Through differentially expressed proteins (DEPs) analysis, we identified Agrin and Zyxin as common proteins expressed in the ipsilateral and contralateral brain hemispheres of BRT_002-treated and vehicle-treated neonatal rats with moderate HI. Moreover, among neurotransmitters and calcium sensors, Syt5 links mitochondrial proteins whose accumulation appeared to be induced by treatment with BRT_002, independently of animal sex. We demonstrated that the regulation of co-expressed Agrin, Zyxin and Syt5 in the ipsilateral and contralateral brain hemispheres from male and female neonatal rats treated with BRT_002 played a significant role in the mechanisms underlying brain tissue repair, the modulation of mitochondrial function and neuroprotection in animals exposed to moderate HI.

Here we developed a tri-substituted purine derivative (BRT_002) as a potential drug for treatment of neonatal HI. BRT_002 was well tolerated in adult and neonatal rats and there were no significant adverse events. Our results showed that IP administration of BRT_002 immediately after neonatal HI resulted in good systemic and brain concentrations of BRT_002 in all groups. Exposure to HI was associated with similar pharmacokinetic parameters in male and female neonatal rats. This preclinical safety and pharmacokinetic (PK) studies are an essential first step to potentially advance the use of BRT_002 for clinical application.

BRT_002 promoted the reduction of brain injury by reducing infarct volume in male and female neonatal rats after exposure to HI, establishing the neuroprotective efficacy of BRT_002 administration. Deep proteomics pointed at Agrin and Zyxin that were not significantly dysregulated when comparing the ipsilateral/contralateral brain hemispheres from male and female Hl+-Veh versus sham neonatal rats but were upregulated in both brain hemispheres from male and female neonatal rats after BRT_002 treatment.

Agrin is a heparan sulfate proteoglycan widely expressed at intraneuronal synapses and involved in the establishment of a tight BBB, suggesting its role in the repair of brain lesions in moderate HI. The role of Agrin in brain repair tissue has been previously reported. Specifically, it was observed that in a mouse ischemia/reperfusion (I/R) injury model, intrathecal administration of Agrin led to its spatiotemporal distribution and inhibited BBB disruption by reducing loss of tight-junctional proteins; this was followed by reduction in infarct volume, decrease in apoptotic neurons and improvement in neurological function. These findings are in agreement with previous observations indicating that Agrin contributes to the barrier properties of endothelial cells and stabilizes tight connections in the BBB. The role of Agrin in promoting coordinated therapeutic processes leading to improvements in cardiac repair has also been reported in pigs.

Zyxin is involved in regulating the secretion of endothelial VWF, which participates in brain lesion repair after HI insult. PLA demonstrated an *in situ* interaction between Zyxin and Agrin, suggesting their coordinated roles in brain tissue repair. Their molecular interaction and their link with VEGFA reinforce the role of both proteins in the brain tissue repair process. The literature reports that when vascular brain injury occurs, VWF is secreted locally following cellular activation for brain repair, and VEGF is involved in this exocytosis of VWF. Here, at 72 h after injury, the ipsilateral expression of VWF in endothelial cells was highly increased in the HI+-BRT_002 groups compared to the Hl+-Veh groups of female and male neonatal rats.RT-qPCR also revealed a significant increase in the ipsilateral expression of VEGFA in endothelial cells in the HI-+BRT_002 groups compared to the HI+-Veh groups of female, male, and female+male neonatal rats. In this regard, modulation of Agrin and Zyxin expression in the brains of male and female neonatal rats under HI might have therapeutic value for restoring cognitive function and/or controlling brain injury.

On another hand, further interpretation of proteomic profiles showed distinct protein signatures that are involved in the modulation of neurotransmitters and calcium sensor activity across the HI+BRT_002 groups of neonatal rats. Particularly these proteins were reported to be associated with neurotransmitter function, calcium sensing, regulation of mitochondrial membranes, developmental and cellular mitochondria respiration, dendrite arborescence, synaptic transmission or regulation of neuroinflammation. Alteration of the expression patterns of these relevant proteins indicates that they account for the brain tissue repair and neuroprotection effects mediated by BRT_002. Identification of these particular proteins expands our knowledge of how BRT_002 mediates its therapeutic effects.

Furthermore, proteomics revealed for the first the first time that Syt5 was significantly more abundant (p<0.05) in both the ipsilateral and contralateral hemispheres of the brains of HI-+BRT_002-treated male and female neonatal rats compared to those in the Hl-+Veh group. The Duolink PLA confirmed the co-accumulation of Syt5 with Agrin, Zyxin, VEGFA and VWF and interactions between Syt5 and VEGFA, VWF, Ang1, suggesting the potential role of these proteins in mediating the BRT_002-induced reduction in infarct volume in neonatal rats exposed to moderate HI.

Due to the role of Syt5 in regulating mitochondrial membranes and developmental and cellular mitochondrial respiration, we performed a functional study of the relationship between Syt5 and mitochondrial respiration in BRT_002-treated neonatal male and female rats after exposure to moderate HI. Interactions between neurotransmitters and calcium sensors whose synthesis was induced by BRT_002 revealed potential interactions between Syt5 and abundant BRT_002-regulated proteins, such as Pacs1 and TXN2. These predictive interactions were confirmed by Duolink PLA, suggesting the likely formation of a transient complex between Syt5 and Txn2. Txn2 is a small protein essential for controlling mitochondrial reactive oxygen species homeostasis and regulating apoptosis and cell viability. Txn2 deficiency impairs mitochondrial redox homeostasis and causes early-onset neurodegeneration with severe cerebellar atrophy, epilepsy, dystonia, optic atrophy, and peripheral neuropathy. Syt5 production was upregulated by BRT_002 treatment in the ipsilateral brain hemisphere of male and female neonatal rats exposed to moderate HI, and Syt5 and Txn2 were found to interact. These findings identified a novel phenotypic target through which BRT_002 regulates mitochondrial function associated with brain repair tissue and neuroprotection.

Bioinformatic analysis revealed the interaction between Syt5 and Pacs1 as well as Pacs2. Pacs1 has been described as a regulator of the intrinsic apoptosis pathway and mitochondrial outer membrane permeabilization shown to be an important factor for development of brain injury in neonatal HI. Pacs1 is associated with a neurodevelopmental disorder characterized by mild-to-severe neurodevelopmental delays and other features, including seizures, behavioral issues, congenital heart anomalies, short stature, and microcephaly. The key features associated with Pacs2 mutation are moderate to severe intellectual disability, cerebellar dysgenesis and other CNS malformations, reduced growth, and facial dysmorphism. BRT_002 treatment upregulated Syt5, Pacs1 and Pacs2 in the ipsilateral brain hemisphere of male and female neonatal rats exposed to moderate HI.

Proteomics also showed the regulatory impact of HI on LETM1, a mitochondrial inner membrane protein needed for maintaining mitochondrial morphology and cristae structures and regulating mitochondrial ion homeostasis. Although Syt5 and LETM1 proteins have not been shown to interact, however we provide evidence of their regulation in the ipsilateral and contralateral brain hemispheres of BRT_002-treated compared to vehicle-treated neonatal rats with moderate HI. These findings suggest that BRT_002 protects integrity of mitochondria in the brain, safeguarding the brain by preventing mitochondrial permeabilization, respiratory deterioration and apoptosis in both neurons and the vascular bed. LETM1 has been associated with optic atrophy 1 (OPA1), and the dysregulation of LETM1 in HI could destabilize L-OPA1 and cause mitochondrial fragmentation, disturb the mitochondrial ultrastructure and increase apoptotic sensitivity. Moreover, our data indicate that by stimulating these protective mechanisms, BRT_002 significantly improves outcomes in HI neonates.

*In vitro* experiments confirmed that BRT_002 provides neuroprotection by increasing mitochondrial respiration and metabolic changes in the neurons of mice exposed to oxygen-glucose deprivation and treated with BRT_002. Neuroprotection mediated by BRT_002 was confirmed in the neonatal Vannucci model, where HI decreased expression of a dendrite marker (MAP2) and a mature neuronal marker (Neun), while BRT_002 treatment prevented neuronal death, as shown by a significant increase in glutamatergic (SLC17A7) and GABAergic neurons in the ipsilateral brain hemisphere of male and female neonatal rats. These findings suggest that Syt5 and the mitochondrial proteins associated with mitochondrial dynamics during the modulation of oxidative stress and neuronal death are likely linked.

Numerous studies have shown that the modulation of mitochondrial function can regulate neuroinflammation and participate in neuroprotection. The accumulation of Agrin, Zyxin and Syt5 in the ipsilateral brain hemisphere of male and neonatal rats was associated with decreased levels of astrocyte and microglial markers compared to those in the HI-Veh group. Even though relationships between markers of inflammation, BBB permeability and neurodegeneration have been reported in previous studies in the context of HI in neonatal rats, the link between markers of inflammation and the modulation of Syt5, Agrin and Zyxin must be further examined. The modulation of these biomarkers highlights the neuroprotective properties of BRT_002 in the treatment of this devastating disease characterized by intellectual disability.

Serpina3n is upregulated in astrocytes and neurons within the ischemic penumbra in the acute phase after stroke. In a previous study, the expression of serpina3n was shown to be gradually increased 6 h after reperfusion, peaked on days 2-3, and then decreased by day 7. Here, we confirmed the upregulation of serpinaA3nN after HI in the contralateral brain hemisphere from BRT_002-treated male and female neonatal rats. Based on the main known function of this protein, it is likely that its unilateral production in the contralateral brain hemisphere suppressed neuroinflammation, oxidative stress, and neuronal apoptosis, thus providing a neuroprotective effect. Further studies are needed to explain why serpina3n was expressed only in the contralateral brain hemisphere of BRT_002-treated male and female neonatal rats 37.

In summary, our study provides strong in vivo evidence that highlights the potential of a novel substituted purine derivative for the treatment of brain ischemic disorders such as neonatal HI, for which there is currently no available treatment. We anticipate that our findings will offer hope for the possibility of a new line of treatments. By taking a multidisciplinary approach, looking changes in HI at the protein and cellular levels, and studying mitochondrial function and neuroinflammation to understand HI at the cellular and subcellular levels, we expand our knowledge of how BRT_002 mediates its therapeutic effects to prevent HI or HI-mediated damage or to treat neonates once HI has been detected. This study supports the continued investigation of BRT_002 for clinical translation as a treatment for HIE.

## Claims

1. **A method for diagnosing a hypoxic-ischemic encephalopathy** in an individual in need thereof, said method comprising at least the steps of:
a) measuring an amount of protein or of gene transcript from a gene or from a combination of genes, the gene or the combination of genes being selected from a group of genes comprising or consisting of AGRN (Agrin), ZYX (Zyxin), VWF (Von Willebrand factor), VEGFA (Vascular endothelial growth factor A), ANGT1 (Angiopoietin 1), ANGT2 (Angiopoietin 2), APCDD1 (APC down-regulated 1), ADGRL1 (Adhesion G Protein-Coupled Receptor L1), ATP2A2 (ATPase sarcoplasmic/endoplasmic reticulum Ca²⁺ transporting 2), ERC2 (ELKS/RAB6-Interacting/CAST family member 2), LETM1 (Leucine Zipper and EF-hand containing transmembrane protein 1), NCALD (Neurocalcin delta), NCS1 (Neuronal calcium sensor 1), SESTD1 (SEC14 and Spectrin domain containing 1), SYT2 (Synaptotagmin-2), SEC14 (SEC14-like protein), PACS1 (Phosphofurin acidic cluster sorting protein 1), RAB1A (RAB1A, member RAS oncogene family), MAP2 (Microtubule associated protein 2), NEUN (Neuronal nuclei), SLC17A7 (Solute carrier family 17 member 7), GABRR1 (Gamma-aminobutyric acid type A receptor rho1 subunit), SERPINA3N (Serine (or cysteine) peptidase inhibitor, clade A, member 3N), TXN2 (thioredoxin 2), GFAP (Glial fibrillary acidic protein), and IBA-1 (Ionized calcium-binding adapter molecule), in an isolated biological sample obtained from said individual, and
b) comparing the amount measured at step a) with a reference value,
wherein a deviation observed between the measured amount at step a) and the reference value is indicative of a hypoxic-ischemic encephalopathy in said individual.

2. A **method for monitoring an evolution** of a hypoxic-ischemic encephalopathy in an individual in need thereof, said method comprising the steps of:
a) measuring an amount of protein or of gene transcript from a gene or from a combination of genes selected from the group of genes as defined in claim 1, in an isolated biological sample obtained from said individual at a first point of time,
b) measuring an amount of protein or of gene transcript from a gene or from a combination of genes selected from the group of genes as defined in step a), in an isolated biological sample obtained from said individual at a second point of time, the gene or combination of genes of step a) and b) being the same, and
c) comparing the amount measured at step a) with the amount measured at step b),
wherein a deviation observed between the amounts measured at step a) and at step b) is indicative of an improvement or an aggravation of the hypoxic-ischemic encephalopathy in said individual.

3. The method according to claim 1 or 2, wherein the combination of genes comprises at least two genes.

4. The method according to any one of claims 1-3, wherein a combination of genes comprises at least AGRN and ZYX.

5. The method according to any one of claims 1-4, wherein the gene or combination of genes comprises or consists of:
- AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, and SERPINA3N, or
- TXN2, GFAP and IBA-1, or
- AGRN, ZYX, VWF, VEGFA, ANGT1, ANGT2, APCDD1, LETM1, PACS1, TXN2, GFAP, and IBA-1, or
- AGRN, ZYX, VWF, VEGFA, ANGT1, ANGT2, and APCDD1, or
- AGRN and ZYX.

6. **A method for monitoring a therapeutic efficacy of a therapeutic treatment** proposed for preventing and/or treating a hypoxic-ischemic encephalopathy in an individual in need thereof, said method comprising the steps of:
a) measuring an amount of protein or of gene transcript from a gene or from a combination of genes selected from a group of genes comprising or consisting of AGRN (Agrin), ZYX (Zyxin), VWF (Von Willebrand factor), VEGFA (Vascular endothelial growth factor A), ANGT1 (Angiopoietin 1), ANGT2 (Angiopoietin 2), APCDD1 (APC down-regulated 1), ADGRL1 (Adhesion G Protein-Coupled Receptor L1), ATP2A2 (ATPase sarcoplasmic/endoplasmic reticulum Ca²⁺ transporting 2), ERC2 (ELKS/RAB6-Interacting/CAST family member 2), LETM1 (Leucine Zipper and EF-hand containing transmembrane protein 1), NCALD (Neurocalcin delta), NCS1 (Neuronal calcium sensor 1), SESTD1 (SEC14 and Spectrin domain containing 1), SYT2 (Synaptotagmin-2), SEC14 (SEC14-like protein), PACS1 (Phosphofurin acidic cluster sorting protein 1), RAB1A (RAB1A, member RAS oncogene family), MAP2 (Microtubule associated protein 2), NEUN (Neuronal nuclei), SLC17A7 (Solute carrier family 17 member 7), GABRR1 (Gamma-aminobutyric acid type A receptor rho1 subunit), SERPINA3N (Serine (or cysteine) peptidase inhibitor, clade A, member 3N), TXN2 (thioredoxin 2), GFAP (Glial fibrillary acidic protein), IBA-1 (Ionized calcium-binding adapter molecule), SYT5 (Synaptotagmin-5), CDK5 (Cyclin dependent kinase 5), HINT1 (Histidine triad nucleotide binding protein 1), MARCKSL1 (Myristoylated alanine-rich C-kinase substrate Like 1), NIPSNAP2 (Nipsnap Homolog 2), PACS2 (Phosphofurin acidic cluster sorting protein 2), and TRPV2 (Transient receptor potential cation channel subfamily V member 2), in an isolated biological sample obtained from said individual before administration of said therapeutic treatment,
b) measuring an amount of protein or of gene transcript from a gene or a combination of genes selected from a group the genes as defined in step a), in an isolated biological sample obtained from said individual after administration of said therapeutic treatment, the gene or combination of genes of step a) and b) being the same, and
c) comparing the amount measured at step a) with the amount measured at step b),
wherein a deviation observed between the amounts measured at step a) and at step b) is indicative of a therapeutic efficacy of said therapeutic treatment on said hypoxic-ischemic encephalopathy.

7. **A method for selecting a candidate therapeutic agent** for preventing and/or treating a hypoxic-ischemic encephalopathy in an individual in need thereof, said method comprising the steps of:
a) measuring an amount of protein or of gene transcript from a gene or a combination of genes selected from the group of genes as defined in claim 6, in an isolated biological sample obtained from a biological model of a hypoxic-ischemic encephalopathy before contacting said biological model with said candidate therapeutic agent,
b) measuring an amount of protein or of gene transcript from a gene or a combination of genes selected from the group of genes as defined in step a), in an isolated biological sample obtained from said biological model of step a) after contacting said biological model with said candidate therapeutic agent, the gene or combination of genes of step a) and b) being the same,
c) comparing the amount measured at step a) with the amount measured at step b), and
d) selecting a candidate therapeutic agent for which a deviation observed between the amounts measured at step a) and at step b) is indicative of a therapeutic efficacy of said candidate therapeutic agent on said hypoxic-ischemic encephalopathy.

8. The method according to claim 6 or 7, wherein the combination of genes comprises at least two genes.

9. The method according to any one of claims 6-8, wherein the combination of genes comprises at least AGRN and ZYX, and optionally SYT5.

10. The method according to any one of claims 6-9, wherein the gene or combination of genes comprises or consists of:
- AGRN, ZYX, VWF, VEGFA, ANGT1, ANGT2, APCDD1, ADGRL1, ATP2A2, ERC2, LETM1, NCALD, NCS1, SESTD1, SYT2, SEC14, PACS1, RAB1A, MAP2, NEUN, SLC17A7, GABRR1, SERPINA3N, SYT5, CDK5, HINT1, MARCKSL1, NIPSNAP2, PACS2, and TRPV2,
or
- AGRN, ZYX, VWF, VEGFA, ANGT1, ANGT2, APCDD1, SEC14, PACS1, RAB1A, MAP2, NEUN, SLC17A7, GABRR1, SERPINA3N, SYT5, CDK5, HINT1, MARCKSL1, NIPSNAP2, PACS2, and TRPV2,
or
- AGRN, ZYX, VWF, VEGFA, ANGT1, ANGT2, APCDD1, SEC14, PACS1, RAB1A, MAP2, NEUN, SLC17A7, GABRR1, SERPINA3N, and SYT5,
or
- AGRN, ZYX, VWF, VEGFA, ANGT1, ANGT2, APCDD1, SEC14, PACS1, RAB1A, and SYT5,
or
- AGRN, ZYX, VWF, VEGFA, ANGT1, ANGT2, APCDD1, and SYT5,
or
- AGRN, ZYX and SYT5
or
- AGRN and ZYX.

11. The method according to anyone of claims 1 to 10, wherein the biological sample is selected from the group consisting of blood sample, plasma sample, serum sample, brain tissue sample, and cerebrospinal fluid sample.

12. **An antibody or a set of primers for use** in a method according to any one of claims 1-11.

13. **Kit-of-parts** for use in a method according to any one of claims 1-11, the kit-of-parts comprising:
a) means for measuring an amount of at least one protein or of at least one gene transcript from a gene or a combination of genes selected from the group of genes as defined in any one of claims 1-10, and
b) a set of instructions for carrying out said measure.

14. The kit-of-parts according to claim 13, wherein
a-1) the means for determining the amount of said protein are configured for performing an immunoassay and/or a mass-spectrometric-based assay, and/or
a-2) the means for determining the amount of said gene transcript are configured for performing a quantitative polymerase chain reaction (qPCR) or a high-throughput sequencing process.
